# EUROPEAN PATENT APPLICATION

(11) **EP 4 238 571 A1**
(43) Date of publication of application: **06.09.2023**
(21) Application number: 21885340.6
(22) Date of filing: 29.10.2021
(51) Int. Cl.: A61K 31/7064, A61K 31/7068, A61K 31/7072, A61K 31/706, A61P 35/00

(54) **APPLICATION OF THYMIDINE DERIVATIVE IN PREPARATION OF DRUGS**

(30) Priority: 30.10.2020 CN 202011192954
(71) Applicant: OnQuality Pharmaceuticals China Ltd., Shanghai 201112 (CN)
(72) Inventor: ZHANG, Shiyi, Shanghai 201112 (CN); WU, Zhaoyu, Shanghai 201112 (CN); LIU, Shilan, Shanghai 201112 (CN)
(74) Representative: Weickmann & Weickmann PartmbB
(86) International application number: PCT/CN2021/127683
(87) International publication number: WO 2022/089614

(57) **Abstract**

The present application relates to a thymidine derivative, or use of a uridine derivative in combination with a thymidine derivative in preparation of a drug for preventing or treating a disease or disorder associated with administration of a chemotherapeutical drug in a subject. The present application further provides a method of preventing or treating a disease or disorder associated with administration of a chemotherapeutical drug in a subject comprising administering a prophylactically or therapeutically effective amount of a thymidine derivative or uridine and thymidine derivatives to a subject in need thereof.

## Description

### CROSS-REFERENCE TO RELATED APPLICATIONS

This application is a National Stage Application and claims priority under 35 U.S.C. § 371 to Patent Cooperation Treaty application PCT/CN2021/127683, filed October 29, 2021, which claims the benefit of Chinese patent application CN2020111929549, filed October 30, 2020. Priority is claimed to these applications and the disclosures of these prior applications are considered part of the disclosure of this application and to the extent allowed the entire contents of the aforementioned applications are incorporated herein.

### INVENTION FIELD

The present application relates to the bio-medical field, in particular to a thymidine derivative and a combination of thymidine derivative and uridine derivative for the treatment of side effects caused by chemotherapeutical drugs.

### BACKGROUND

Currently, chemotherapy is one of the main methods of cancer treatment. Chemotherapy drugs can damage normal tissues of patients while killing cancer cells. Therefore, chemotherapy often causes serious side effects, especially those in skin, facial features, hematopoietic tissues and gastrointestinal tract. Severe side effects caused by chemotherapy can damage the life quality of patients, and reduce the patient's medication tolerance, thereby adversely affecting the treatment effect, leading to disease progression, and then affecting the patient's survival.

Currently, there is not yet an effective therapeutical regimen to control the side effects associated with administration of chemotherapeutical drugs. Therefore, there is an urgent need for new solutions to fill this gap.

### SUMMARY OF THE INVENTION

The present application relates to use of a thymidine derivative and a combination of uridine derivative and thymidine derivative in preparation of a drug for preventing or treating a disease or disorder associated with administration of a chemotherapeutical drug in a subject. In particular, the present application relates to the use of a thymidine derivative and a combination of uridine derivative and thymidine derivative for preventing or treating a disease or disorder associated with administration of a chemotherapeutical drug in a subject, which can effectively control the side effects caused by the chemotherapeutical drug, such as, skin tissue disease or disorder, facial feature disease or disorder and/or gastrointestinal disease or disorder, etc. associated with administration of the chemotherapeutical drug. The uridine derivative and/or thymidine derivative of the present application can comprise a non-steroidal anti-inflammatory drug (NSAID) moiety, which can not only treat and/or prevent a disease or disorder associated with a chemotherapeutical drug, but also have an analgesic and anti-inflammatory effect to further improve the symptoms of the subject.

In an aspect, the present application provides use of a thymidine derivative in preparation of a drug for preventing or treating a disease or disorder associated with administration of a chemotherapeutical drug in a subject, wherein at least one hydroxyl hydrogen of a deoxyribose in the thymidine derivative is substituted.

In some embodiments, the thymidine derivative comprises a structure of Formula (I): 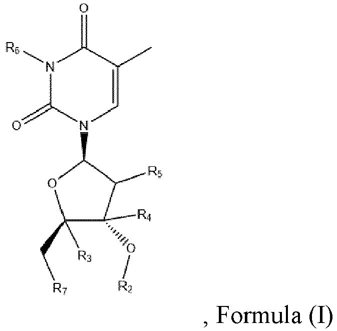 wherein R₂, R₃, R₄, R₅, R₆ and R₇ are not simultaneously hydrogen.

In some embodiments, R₇ is 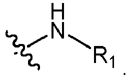 In some embodiments, R₇ is

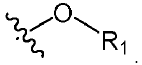

In some embodiments, R₆ is hydrogen.

In some embodiments, R₆ is 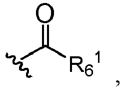 wherein the R₆¹ is C₁-C₆ alkyl. In some embodiments, R₆ is

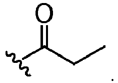

In some embodiments, R₁ is 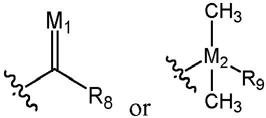 , wherein M₁ is oxygen or sulfur, and R₈ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; M₂ is silicon or carbon, and R₉ is selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; R₉ comprises one or more groups selected from the group consisting of C₁-C₅ alkyl, C₁-C₅ cycloalkyl, phenyl or benzyl.

In some embodiments, R₂ is 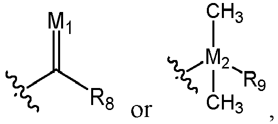 wherein M₁ is oxygen or sulfur, and R₈ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; M₂ is silicon or carbon, and R₉ is selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; R₉ comprises one or more groups selected from the group consisting of C₁-C₅ alkyl, C₁-C₅ cycloalkyl, phenyl or benzyl.

In some embodiments, R₃ is 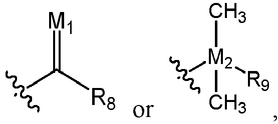 wherein M₁ is oxygen or sulfur, and R₈ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; M₂ is silicon or carbon, and R₉ is selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; R₉ comprises one or more groups selected from the group consisting of C₁-C₅ alkyl, C₁-C₅ cycloalkyl, phenyl or benzyl.

In some embodiments, R₁ is selected from the group consisting of -(C=O)-R₈¹, -(C=O)-OR₈², -(C=O)-NR₈³R₈⁴, -(C=S)-R₈⁵, -(C=S)-OR₈⁶ and -(C=S)-NR₈⁷R₈⁸, wherein any one of R₈¹, R₈², R₈³, R₈⁴, R₈⁵, R₈⁶, R₈⁷ and R₈⁸ independently comprises one or more groups selected from the group consisting of hydrogen, substituted or unsubstituted alkyl, substituted or unsubstituted alkynyl, substituted or unsubstituted cycloalkyl, substituted or unsubstituted heterocycloalkyl, substituted or unsubstituted aryl, and substituted or unsubstituted heteroaryl.

In some embodiments, R₂ is selected from the group consisting of -(C=O)-R₈¹, -(C=O)-OR₈², -(C=O)-NR₈³R₈⁴, -(C=S)-R₈⁵, -(C=S)-OR₈⁶ and -(C=S)-NR₈⁷R₈⁸, wherein any one of R₈¹, R₈², R₈³, R₈⁴, R₈⁵, R₈⁶, R₈⁷ and R₈⁸ independently comprises one or more groups selected from the group consisting of hydrogen, substituted or unsubstituted alkyl, substituted or unsubstituted alkynyl, substituted or unsubstituted cycloalkyl, substituted or unsubstituted heterocycloalkyl, substituted or unsubstituted aryl, and substituted or unsubstituted heteroaryl.

In some embodiments, R₃ is selected from the group consisting of -(C=O)-R₈¹, -(C=O)-OR₈², -(C=O)-NR₈³R₈⁴, -(C=S)-R₈⁵, -(C=S)-OR₈⁶ and -(C=S)-NR₈⁷R₈⁸, wherein any one of R₈¹, R₈², R₈³, R₈⁴, R₈⁵, R₈⁶, R₈⁷ and R₈⁸ independently comprises one or more groups selected from the group consisting of hydrogen, substituted or unsubstituted alkyl, substituted or unsubstituted alkynyl, substituted or unsubstituted cycloalkyl, substituted or unsubstituted heterocycloalkyl, substituted or unsubstituted aryl, and substituted or unsubstituted heteroaryl.

In some embodiments, any one of R₈¹, R₈², R₈³, R₈⁴ , R₈⁵, R₈⁶, R₈⁷ and R₈⁸ independently comprises one or more groups selected from the group consisting of hydrogen, C₁-C₅ alkyl, C₁-C₅ cycloalkyl, C₁-C₅ heterocycloalkyl, phenyl or benzyl.

In some embodiments, R₈¹ is selected from the group consisting of C₁-C₅ alkyl, C₁-C₅ cycloalkyl, phenyl, and benzyl. In some embodiments, R₈¹ is selected from the group consisting of methyl, ethyl, propyl, butyl, cyclopentyl, cyclopropyl, and benzyl.

In some embodiments, R₈² is selected from the group consisting of C₁-C₅ alkyl, C₁-C₅ cycloalkyl, phenyl, and benzyl. In some embodiments, R₈² is selected from the group consisting of cyclopropyl, propyl, butyl, pentyl, phenyl, and benzyl.

In some embodiments, R₈³ is hydrogen.

In some embodiments, R₈⁴ is selected from the group consisting of C₁-C₅ alkyl, C₁-C₅ cycloalkyl, phenyl, and benzyl. In some embodiments, R₈⁴ is selected from the group consisting of cyclopropyl, cyclopentyl, and phenyl.

In some embodiments, R₉ is butyl.

In some embodiments, R₃ is hydrogen. In some embodiments, R₄ is hydrogen. In some embodiments, R₅ is hydrogen.

In some embodiments, R₁ is 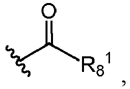 wherein R₈¹ comprises one or more groups selected from the group consisting of C₁-C₆ alkyl, C₁-C₆ silyl, C₁-C₆ alkoxy, C₁-C₆ alkylnitro, C₃-C₇ cycloalkyl, C₃-C₇ cycloalkylnitro, C₄-C₇ aryl, C₄-C₇ alkoxyaryl and/or C₄-C₇ heteroaryl. In some embodiments, R₂ is 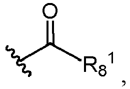 wherein R₈¹ comprises one or more groups selected from the group consisting of C₁-C₆ alkyl, C₁-C₆ silyl, C₁-C₆ alkoxy, C₁-C₆ alkylnitro, C₃-C₇ cycloalkyl, C₃-C₇ cycloalkylnitro, C₄-C₇ aryl, C₄-C₇ alkoxyaryl and/or C₄-C₇ heteroaryl.

In some embodiments, in the structure of Formula (I), R₃, R₄, R₅ and R₆ are all hydrogen, R₇ 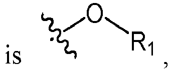 R₁ is selected from the group consisting of hydrogen, 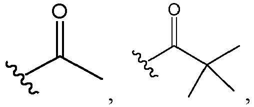 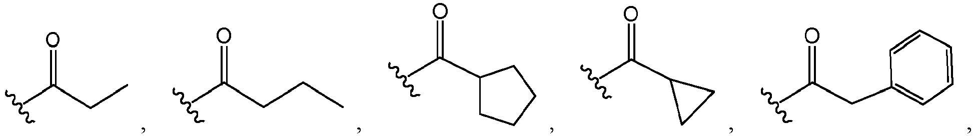 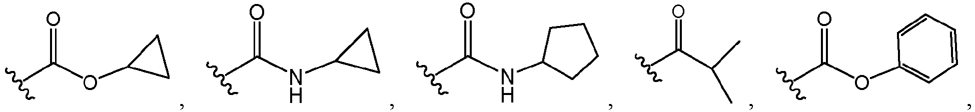 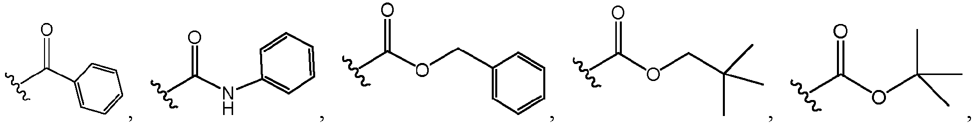 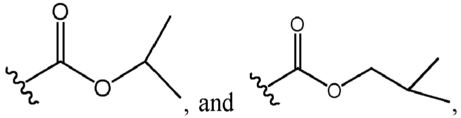 R₂ is selected from the group consisting of hydrogen, 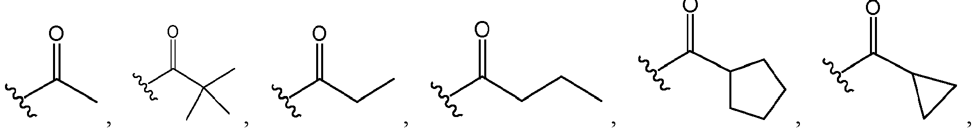 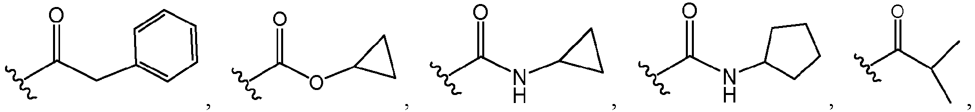 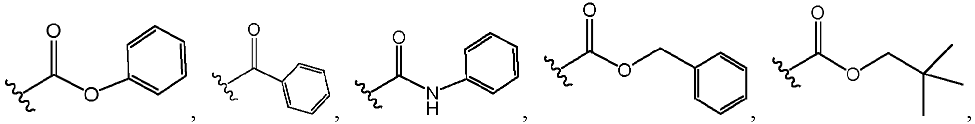 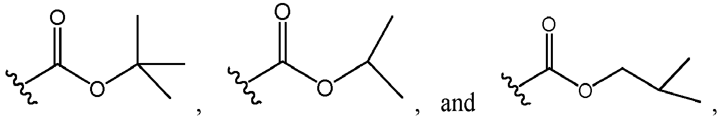 and R₂ and R₁ are not simultaneously hydrogen.

In some embodiments, the thymidine derivative is selected from one or more of compound T1 to compound T24 of the following group:

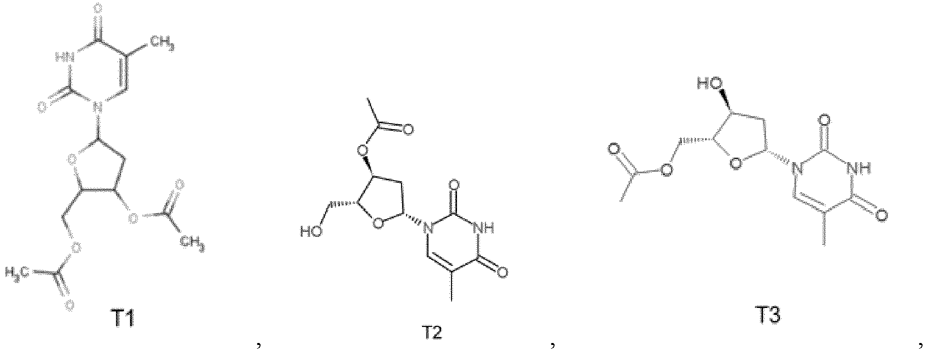

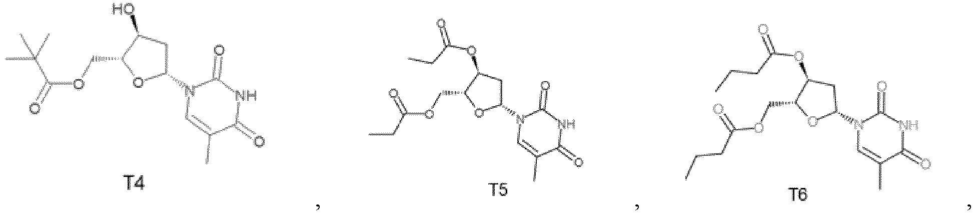

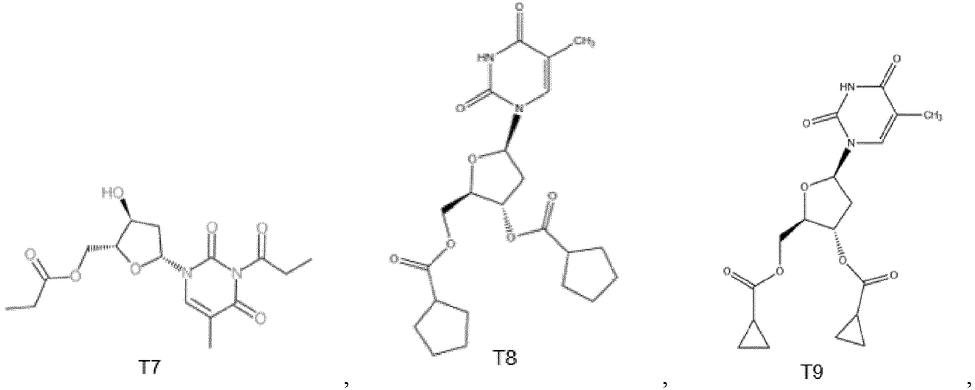

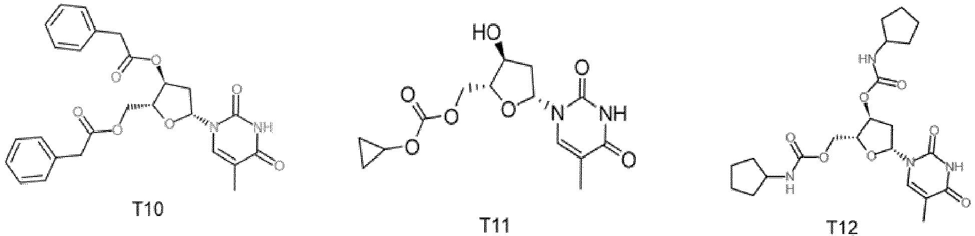

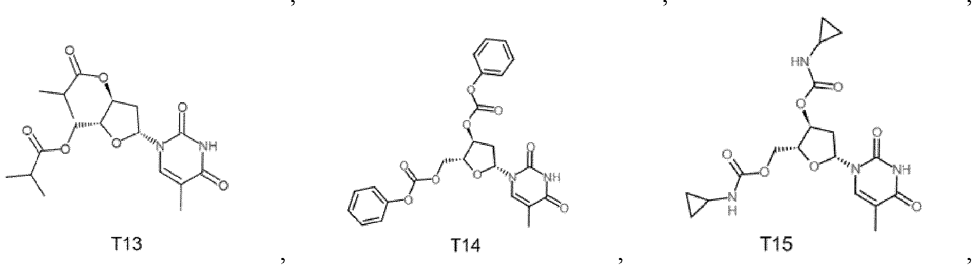

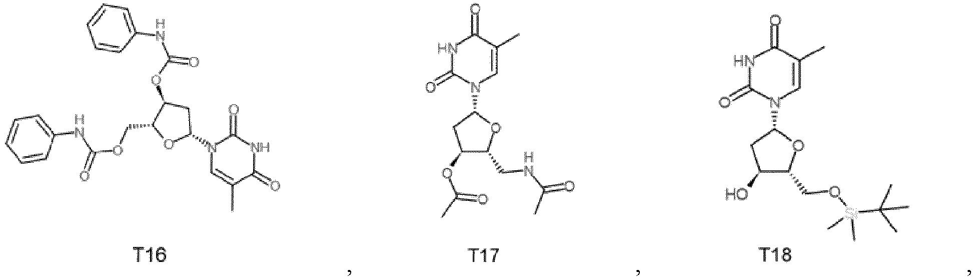

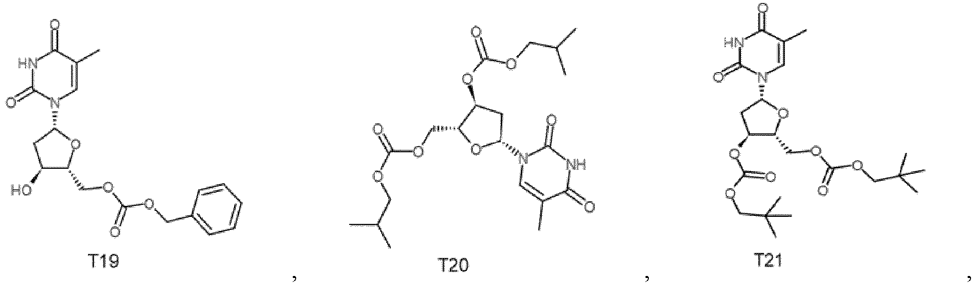

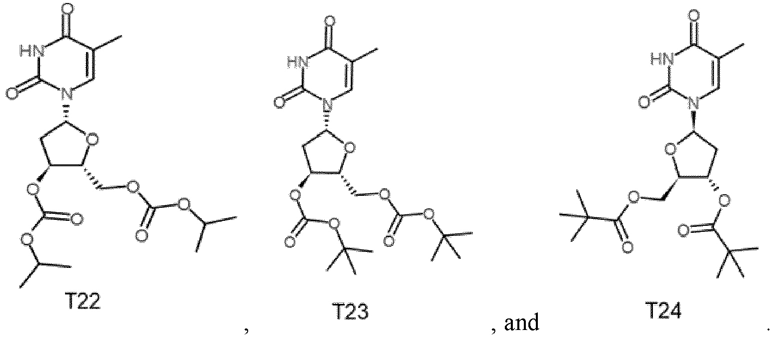

In some embodiments, the thymidine derivative comprises the structure of Formula (II): 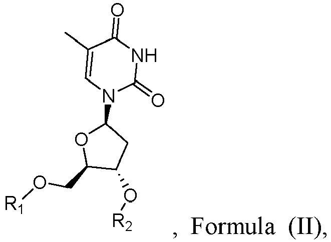 wherein R₁ and/or R₂ comprise/comprises a non-steroidal anti-inflammatory drug (NSAID) moiety. For example, R₁ comprises a NSAID moiety, and R₂ is hydrogen. For example, R₂ comprises a NSAID moiety, and R₁ is hydrogen. For example, R₁ comprises a NSAID moiety, and R₂ comprises a NSAID moiety.

In some embodiments, the NSAID moiety comprises salicylic acid or a derivative thereof, aryl acetic acid or a derivative thereof, heteroaryl acetic acid or a derivative thereof, indoleacetic acid or a derivative thereof, indene acetic acid or a derivative thereof, anthranilic acid or a derivative thereof and/or enolic acid or a derivative thereof.

In some embodiments, R₁ or R₂ is hydrogen.

In some embodiments, R₁ and R₂ are not simultaneously hydrogen.

In some embodiments, R₁ and/or R₂ are/is 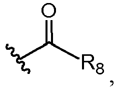 wherein R₈ is Rₛ² or 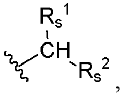 wherein R_{S}¹ is hydrogen or methyl, Rₛ² is 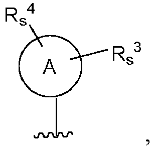 wherein the ring A is C₄-C₇ aryl, C₄-C₇ heteroaryl, indene ring, naphthalene ring, indoline ring, unsaturated polycyclic hydrocarbon and/or heterocyclic polycycle, Rs³ and/or Rs⁴ are/is independently selected from the group consisting of hydrogen, C₁-C₆ alkyl, C₁-C₆ alkyl ester, halogen, C₄-C₇ aryl, C₄-C₇ heteroaryl, and 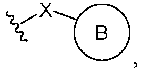 wherein ring B is C₄-C₇ aryl, C₄-C₇ heteroaryl, X is -CH₂, -NH-, -O- or 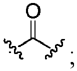 wherein, the C₄-C₇ aryl, C₄-C₇ heteroaryl are optionally substituted with one or more substituents selected from the group consisting of halogen, C₁-C₆ alkyl, C₁-C₆ alkynyl, and C₁-C₆ alkenyl.

In some embodiments, ring A is pyrrole ring, Rₛ³ is C₁-C₆ alkyl, Rₛ⁴ is 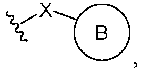 wherein Xis 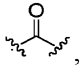 ring B is benzene ring, and the ring B is optionally substituted with one or more C₁-C₆ alkyl.

In some embodiments, Rₛ¹ and/or Rₛ² are/is halogen. 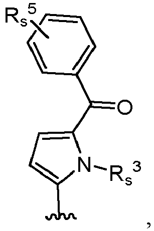 wherein the Rₛ³ is C₁-C₆ alkyl or

In some embodiments, Rₛ¹ and/or Rₛ² are/is 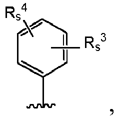 wherein Rₛ³ and/or Rₛ⁴ are/is selected from hydrogen, C₁-C₆ alkyl, C₁-C₆ alkyl ester, halogen, C₄-C₇ aryl, C₄-C₇ heteroaryl, and 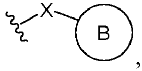 ring B is C₄-C₇ aryl, C₄-C₇ heteroaryl, X is -CH₂, -NH-, -O- or 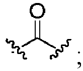 wherein, the C₄-C₇ aryl, C₄-C₇ heteroaryl are optionally substituted with one or more substituents selected from the group consisting of halogen, C₁-C₆ alkyl, C₁-C₆ alkynyl, and C₁-C₆ alkenyl.

In some embodiments, Rₛ² is 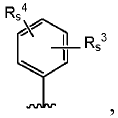 wherein Rₛ³ and/or Rₛ⁴ are/is selected from the group consisting of hydrogen, C₁-C₆ alkyl, 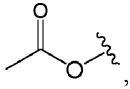 fluorine, chlorine, bromine, benzene ring, and 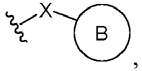 wherein ring B is benzene ring, X is -CH₂, -NH-, -O- or 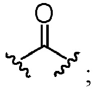 the benzene ring is optionally substituted with one or more substituents selected from the group consisting of fluorine, chlorine and bromine.

In some embodiments, R₈ is 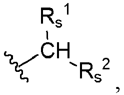 Rₛ¹ is hydrogen or methyl, Rₛ² is selected from the group consisting of

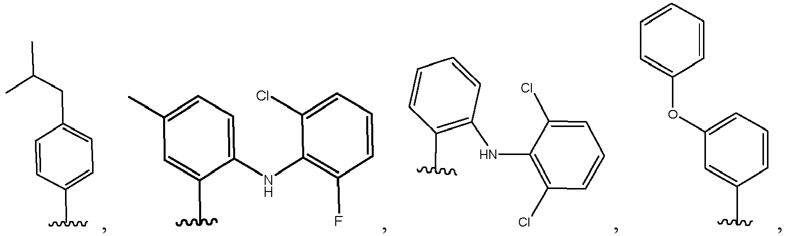

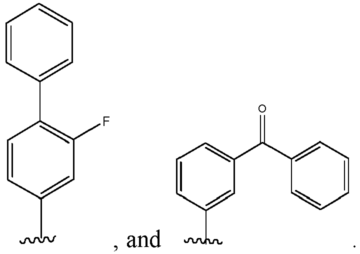

In some embodiments, R₈ is 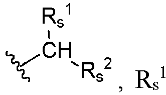 is hydrogen or methyl, Rₛ² is 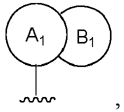 wherein ring A₁ is C₄-C₇ cycloalkyl, C₄-C₇ heterocycloalkyl, C₄-C₇ aryl and/or C₄-C₇ heteroaryl, ring B₁ is C₄-C₇ cycloalkyl, C₄-C₇ heterocycloalkyl, C₄-C₇ aryl, C₄-C₇ heteroaryl or 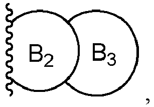 wherein ring B₂ is C₄-C₇ cycloalkyl, C₄-C₇ heterocycloalkyl, C₄-C₇ aryl and/or C₄-C₇ heteroaryl, ring B₃ is C₄-C₇ cycloalkyl, C₄-C₇ heterocycloalkyl, C₄-C₇ aryl and/or C₄-C₇ heteroaryl, wherein the C₄-C₇ cycloalkyl, C₄-C₇ heterocycloalkyl, C₄-C₇ aryl and/or C₄-C₇ heteroaryl are optionally substituted with halogen, 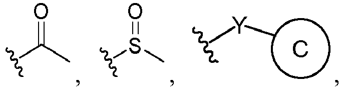 C₁-C₆ alkyl, C₁-C₆ alkyl-substituted ester groups and/or C₁-C₆ alkyl-substituted aldehyde groups, wherein ring C is benzene ring, Y is -CH₂, -NH-, - O- or 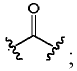 the benzene ring is optionally substituted with one or more substituents selected from the group consisting of fluorine, chlorine, bromine and a ring atom on ring B₂ or ring B₃. 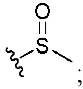 the Y may form a double bond with

In some embodiments, Rₛ² is 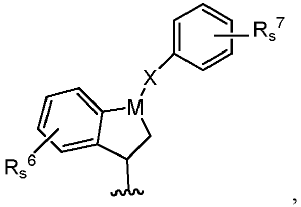 wherein the Rₛ⁶ is fluorine, chlorine or bromine, 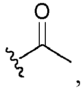 M is nitrogen or carbon, X is carbon or 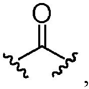 a double bond is optionally formed between X and M, Rₛ⁷ is fluorine, chlorine, bromine or

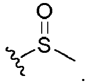

In some embodiments, R₈ is 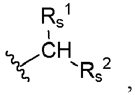 Rₛ¹ is hydrogen or methyl, Rₛ² is

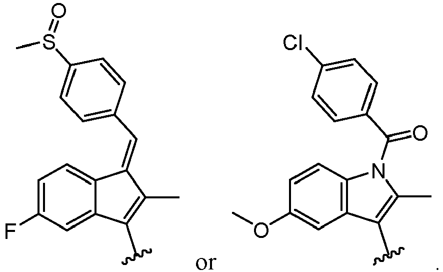

In some embodiments, R₈ is 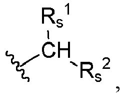 Rₛ¹ is hydrogen or methyl, Rₛ² is

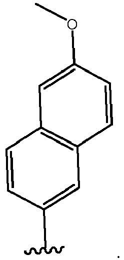

In some embodiments, Rₛ² is 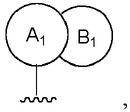 the ring A₁ is benzene ring, ring B₁ is 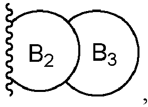 and ring B₂ is pyrrole ring, B₃ is pyran ring, and the pyran ring is optionally substituted with one or more C₁-C₆ alkyl and/or C₁-C₆ alkyl-substituted aldehyde groups.

In some embodiments, R₈ is 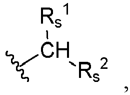 Rₛ¹ is hydrogen or methyl, Rₛ² is

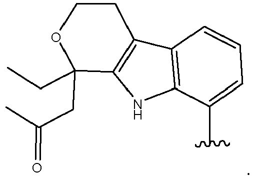

In some embodiments, R₈¹ is

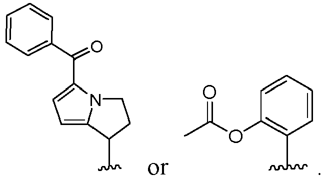

In some embodiments, R₁ and/or R₂ are/is

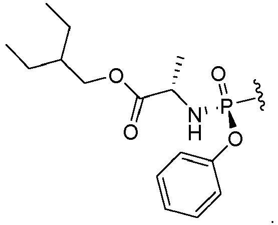

In some embodiments, the R₁ comprises a NSAID moiety, and R₂ is hydrogen.

In some embodiments, the R₁ is any one group selected from the group consisting of: 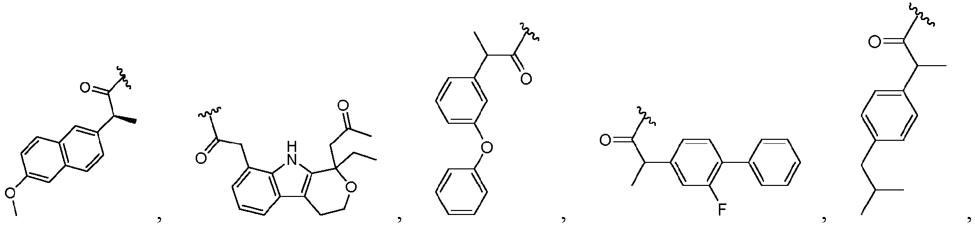 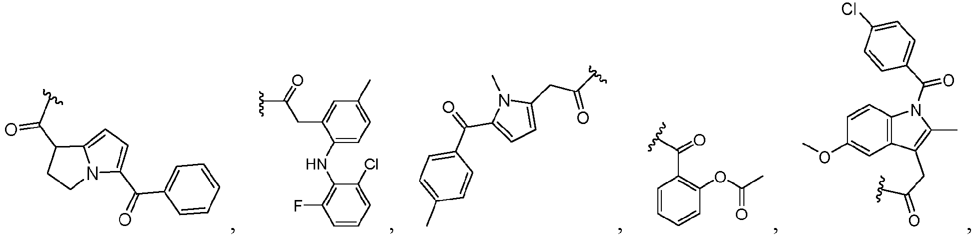 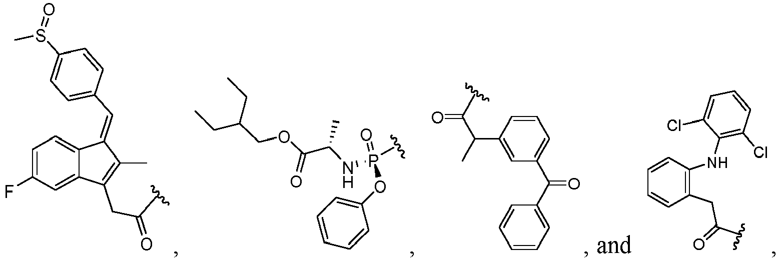 and R₂ is hydrogen.

In some embodiments, the R₂ comprises a NSAID moiety, and R₁ is hydrogen.

In some embodiments, the R₂ is any one group selected from the group consisting of: 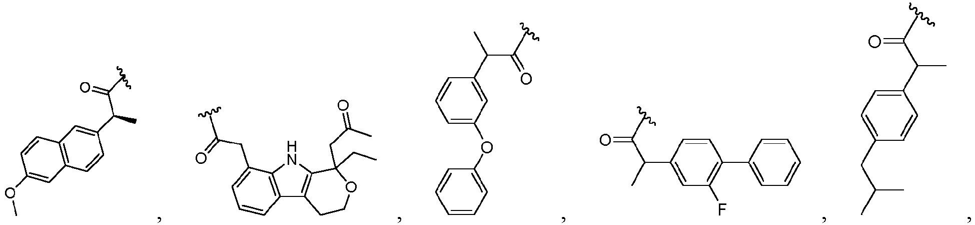 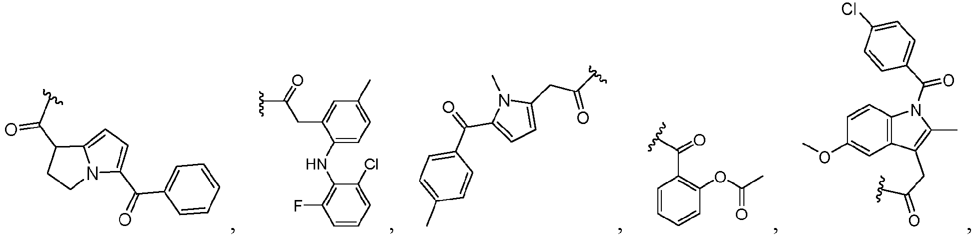 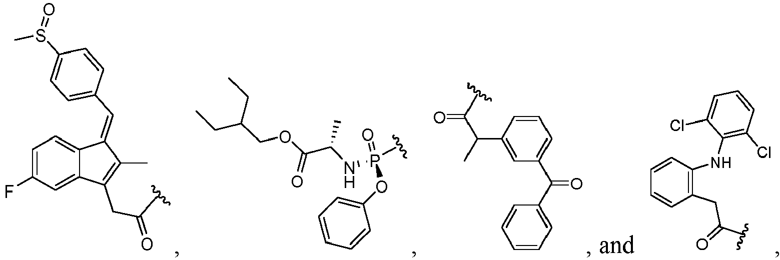 and R₁ is hydrogen.

In some embodiments, the R₁ and R₂ both comprise the NSAID moiety.

In some embodiments, the R₁ and R₂ are each independently any one group selected from the group consisting of:

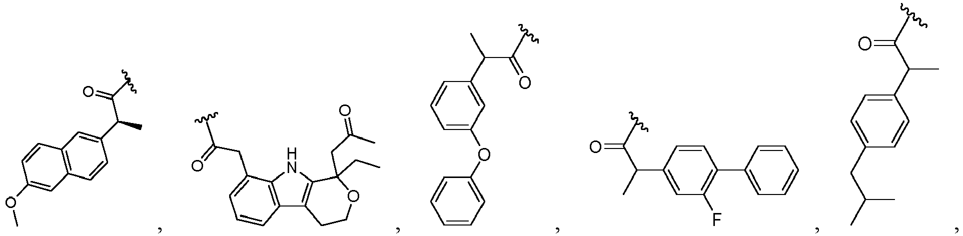

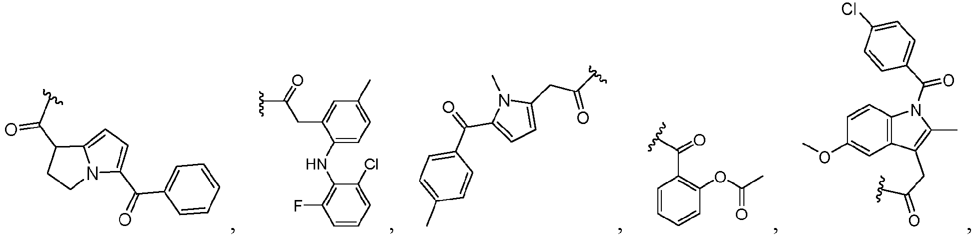

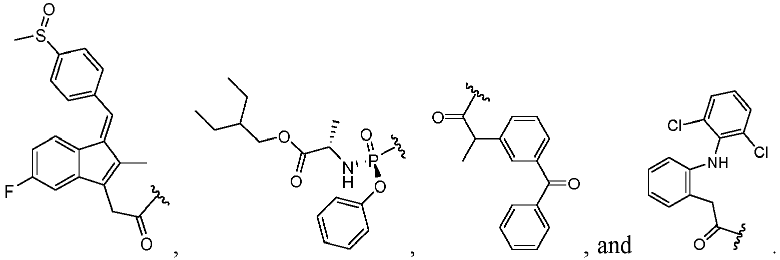

In some embodiments, the thymidine derivative is selected from one or more of compound T25 to compound T50 of the following group: 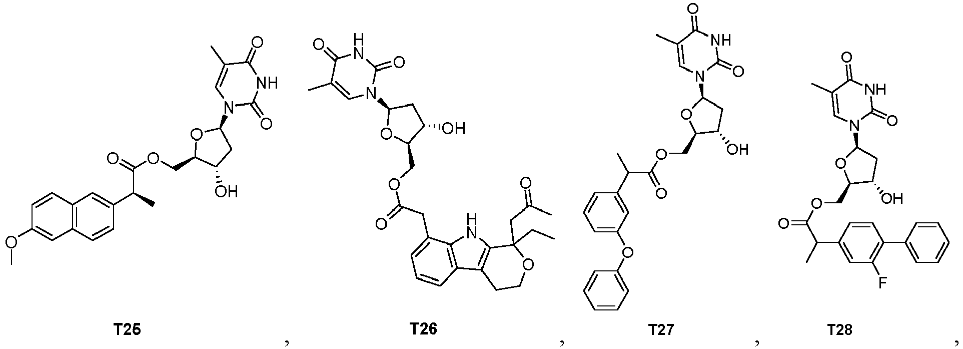 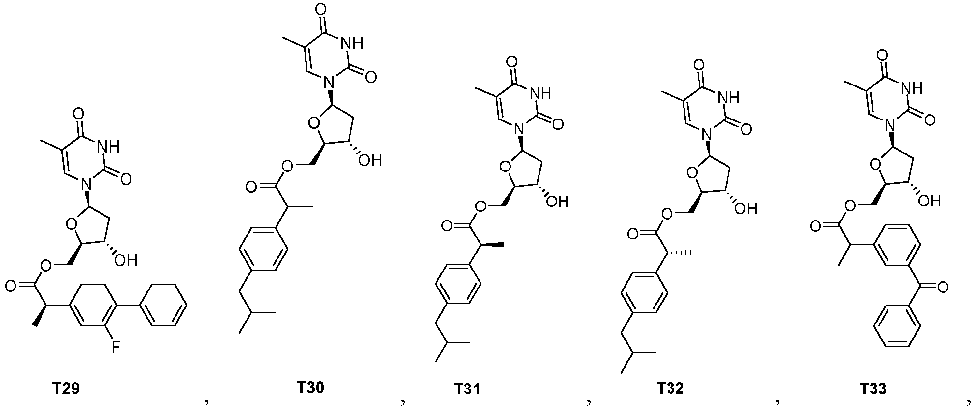 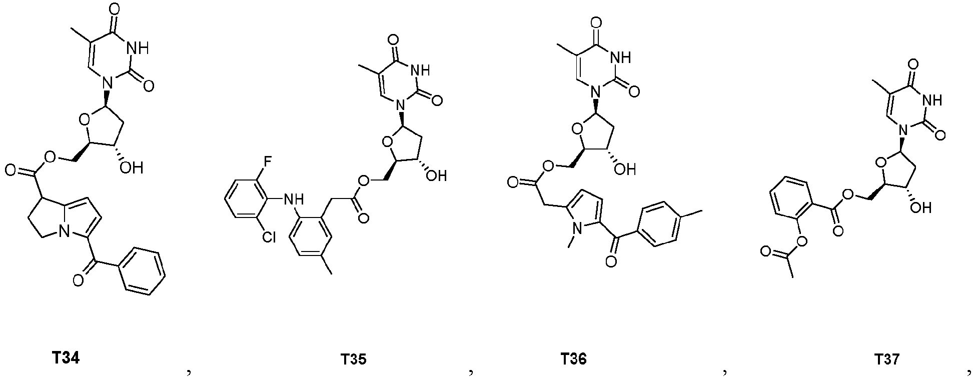 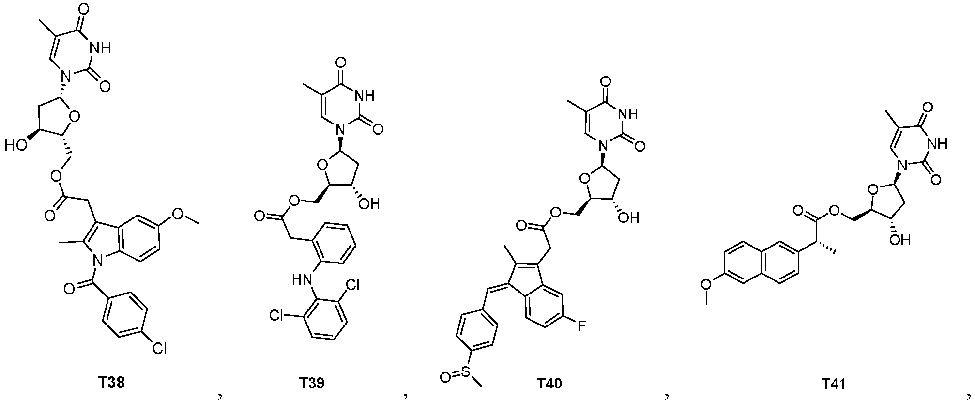 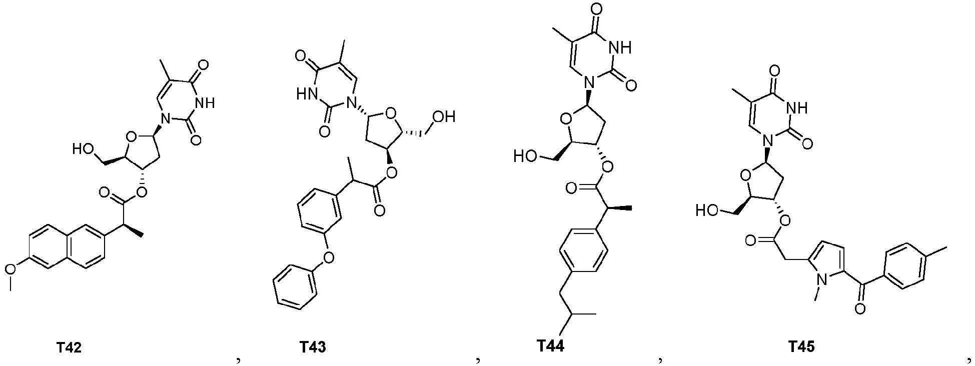 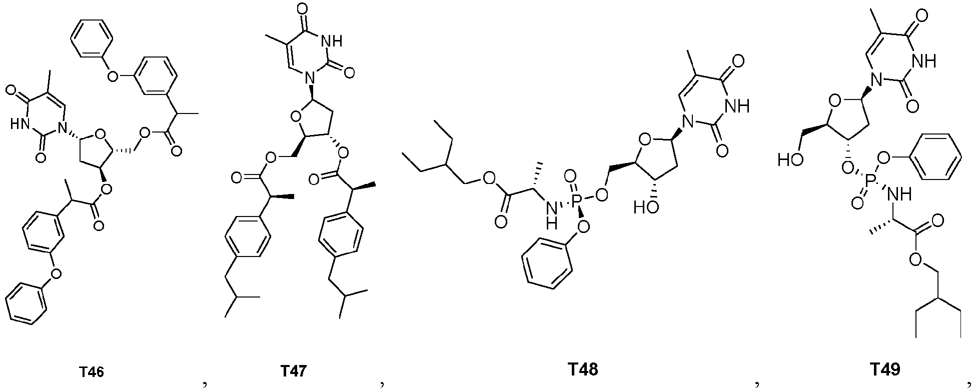 and

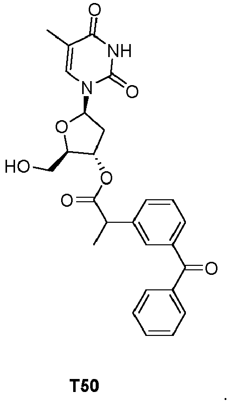

In some embodiments, the chemotherapeutical drug is used for treating cancers.

In some embodiments, the chemotherapeutical drug comprises a pyrimidine nucleoside analog or a prodrug thereof.

In some embodiments, the chemotherapeutical drug comprises one or more selected from the group consisting of capecitabine, cytarabine, docetaxel, adriamycin, fluorouracil (5-FU), floxuridine, tegafur, idarubicin, paclitaxel, epirubicin, acelarin (NUC-1031), doxorubicin, folinic acid, cis-platinum, cyclophosphamide, vincristine and 5-FU pro-drugs (e.g., tegafur and 5'-deoxyfloxuridine, floxuridine, 2'-deoxyfloxuridine, a pro-drug derivative of floxuridine or a pro-drug derivative of 2'-deoxyfloxuridine, trifluoro-methyl-2'-deoxyuridine, 6-azauridine, 3-deazauridine).

In some embodiments, the disease or disorder in the use is caused by administration of the chemotherapeutical drug.

In some embodiments, the disease or disorder associated with administration of the chemotherapeutical drug comprises skin tissue disease or disorder associated with administration of the chemotherapeutical drug, hemopoietic tissue disease or disorder associated with administration of the chemotherapeutical drug, limb disease or disorder associated with administration of the chemotherapeutical drug, cardiac disease or disorder associated with administration of the chemotherapeutical drug, nervous system disease or disorder associated with administration of the chemotherapeutical drug, facial feature disease or disorder associated with administration of the chemotherapeutical drug and/or gastrointestinal disease or disorder associated with administration of the chemotherapeutical drug.

In some embodiments, the hemopoietic tissue disease or disorder associated with administration of the chemotherapeutical drug comprises marrow disease or disorder associated with administration of the chemotherapeutical drug and blood disease or disorder associated with administration of the chemotherapeutical drug.

In some embodiments, the hemopoietic tissue disease or disorder associated with administration of the chemotherapeutical drug comprises abnormal blood cell proliferative disease or disorder associated with administration of the chemotherapeutical drug.

In some embodiments, the disease or disorder associated with administration of the chemoth erapeutical drug comprises epithelial tissue disease or disorder associated with administration of the chemotherapeutical drug in the skin, limbs, facial features and/or gastrointestinal tract.

In some embodiments, the epithelial tissue disease or disorder associated with administration of the chemotherapeutical drug in the skin, limbs, facial features and/or gastrointestinal tract comprises disease or disorder associated with endothelial cell lesion, and/or disease or disorder associated with epithelial cell lesion, and wherein the endothelial cell lesion and/or epithelial cell lesion are/is associated with administration of the chemotherapeutic drug.

In some embodiments, the endothelial cell comprises the vascular endothelial cell.

In some embodiments, the epithelial cell comprises skin epithelial cell, oral epithelial cell, nasal epithelial cell, gastric epithelial cell and/or intestinal epithelial cell.

In some embodiments, the disease or disorder associated with administration of the chemotherapeutical drug in the use comprises rash associated with administration of the chemotherapeutical drug, hand-foot syndrome associated with administration of the chemotherapeutical drug, pruritus associated with administration of the chemotherapeutical drug, erythema associated with administration of the chemotherapeutical drug, dry skin associated with administration of the chemotherapeutical drug, alopecia associated with administration of the chemotherapeutical drug, paronychia associated with administration of the chemotherapeutical drug, pigmentation disorder associated with administration of the chemotherapeutical drug, oral ulcer associated with administration of the chemotherapeutical drug, xerostomia associated with administration of the chemotherapeutical drug, epistaxis associated with administration of the chemotherapeutical drug, nasopharyngitis associated with administration of the chemotherapeutical drug, cheilitis associated with administration of the chemotherapeutical drug, esophagus mucositis associated with administration of the chemotherapeutical drug, gastric mucositis associated with administration of the chemotherapeutical drug, gastric ulcer associated with administration of the chemotherapeutical drug, diarrhea associated with administration of the chemotherapeutical drug, vomiting associated with administration of the chemotherapeutical drug, nausea associated with administration of the chemotherapeutical drug, anorexia associated with administration of the chemotherapeutical drug, constipation associated with administration of the chemotherapeutical drug, abdominal pain associated with administration of the chemotherapeutical drug, nonspecific chest pain associated with administration of the chemotherapeutical drug, angina pectoris associated with administration of the chemotherapeutical drug, palpitation associated with administration of the chemotherapeutical drug, dyspnea associated with administration of the chemotherapeutical drug, diffuse pleuritic chest pain associated with administration of the chemotherapeutical drug, supraventricular arrhythmia associated with administration of the chemotherapeutical drug, hypotension associated with administration of the chemotherapeutical drug, myocardial infarction associated with administration of the chemotherapeutical drug, bradycardia associated with administration of the chemotherapeutical drug, arrhythmia associated with administration of the chemotherapeutical drug, ventricular fibrillation associated with administration of the chemotherapeutical drug, ventricular tachycardia associated with administration of the chemotherapeutical drug, myocarditis associated with administration of the chemotherapeutical drug, heart failure associated with administration of the chemotherapeutical drug, acute pneumonedema associated with administration of the chemotherapeutical drug, cardiac arrest associated with administration of the chemotherapeutical drug, pericarditis associated with administration of the chemotherapeutical drug, leukemia associated with administration of the chemotherapeutical drug, marrow proliferative disease associated with administration of the chemotherapeutical drug, marrow suppression associated with administration of the chemotherapeutical drug, anemia associated with administration of the chemotherapeutical drug, leukopenia associated with administration of the chemotherapeutical drug, thrombocytopenia associated with administration of the chemotherapeutical drug, distal paresthesia associated with administration of the chemotherapeutical drug, muscle contraction associated with administration of the chemotherapeutical drug, and/or limb stiffness associated with administration of the chemotherapeutical drug.

In some embodiments, the disease or disorder associated with administration of the chemotherapeutical drug comprises hand-foot syndrome associated with administration of the chemotherapeutical drug. In some embodiments, the disease or disorder associated with administration of the chemotherapeutical drug comprises paronychia associated with administration of the chemotherapeutical drug. In some embodiments, the disease or disorder associated with administration of the chemotherapeutical drug comprises gastrointestinal disease associated with administration of the chemotherapeutical drug. In some embodiments, the disease or disorder associated with administration of the chemotherapeutical drug comprises diarrhea associated with administration of the chemotherapeutical drug.

In some embodiments, the severity of the disease or disorder associated with administration of the chemotherapeutical drug is grade 1 or above, grade 2 or above, grade 3 or above, grade 4 or above, and/or grade 5 in accordance with NCI-CTCAE V5.0.

In some embodiments, the drug is prepared to be applicable for topical administration. In some embodiments, the drug is prepared to be applicable for topical administration with limited area. In some embodiments, the drug is prepared to be applicable for oral administration.

In some embodiments, the drug is prepared to be applicable for oral administration. Oral formulations can comprise, but are not limited to, capsules, sachets, pills, tablets, lozenges, powders, granules, aqueous or nonaqueous solutions or suspensions, water-in-oil or oil-in-water emulsions, elixirs or syrups, troches and/or mouthwashes or the like.

In some embodiments, the drug is prepared as cream, emulsion, gel, oil, ointment, spray, foam, liposome formulation, liniment, lotion, aerosol and/or transdermal agent absorbed via skin.

In some embodiments, the concentration of the thymidine derivative in the drug is about 0.0001% (w/w) to about 50% (w/w). In some embodiments, the concentration of the thymidine derivative in the drug is about 0.1% (w/w) to about 5% (w/w). In some embodiments, the concentration of the thymidine derivative in the drug is about 0.5% (w/w) to about 3% (w/w).

In some embodiments, the administration dose of the thymidine derivative is about 0.5 µM to about 1000 µM. In some embodiments, the administration dose of the thymidine derivative is about 1 µM to about 500 µM.

In some embodiments, the administration dose of the thymidine derivative is about 15 mpk to about 300 mpk. In some embodiments, the administration dose of the thymidine derivative is about 10 mpk to about 500 mpk.

In some embodiments, the drug further comprises one or more active ingredients.

In some embodiments, the drug does not substantially affect the therapeutical effect of the chemotherapeutical drug.

In some embodiments, the subject in the use comprises a cancer patient.

In some embodiments, the subject has been, is being and/or will be administered with the chemotherapeutical drug.

In some embodiments, the subject has or is susceptible to have the disease or disorder associated with administration of the chemotherapeutical drug.

In some embodiments, the severity of the disease or disorder in the use increases after administration of the chemotherapeutical drug.

In some embodiments, before administration of the chemotherapeutical drug, the subject did not have the disease or disorder.

In another aspect, the present application provides a pharmaceutical combination or a kit, comprising: 1) a chemotherapeutical drug; and 2) a thymidine derivative.

In some embodiments of the pharmaceutical combination or the kit, the chemotherapeutical drug and the thymidine derivative are not mixed with each other.

In some embodiments of the pharmaceutical combination or the kit, the chemotherapeutical drug and the thymidine derivative are each independently present in separate containers.

In some embodiments of the pharmaceutical combination or the kit, the thymidine derivative in 2) can prevent or treat a disease or disorder associated with administration of the chemotherapeutical drug in 1).

In some embodiments of the pharmaceutical combination or the kit, the thymidine derivative in 2) does not substantially affect the therapeutical effect of the chemotherapeutical drug in 1).

In some embodiments of the pharmaceutical combination or the kit, the thymidine derivative in 2) is administered before, simultaneously with, or after the administration of the chemotherapeutical drug in 1).

In another aspect, the present application provides a method comprising administering to a subject a thymidine derivative, wherein the subject has been, is being and/or will be administered with a chemotherapeutical drug, and has or is susceptible to disease or disorder associated with administration of the chemotherapeutical drug.

In another aspect, the present application provides a method of preventing or treating a disease or disorder, comprising administering a thymidine derivative to a subject who is susceptible to disease or disorder associated with administration of the chemotherapeutical drug, wherein the subject has been, is being and/or will be administered with the chemotherapeutical drug.

In another aspect, the present application provides a method of preventing or treating a disease or disorder, comprising administering a combination of thymidine derivative and uridine derivative to a subject who is susceptible to the disease or disorder, wherein the disease or disorder is disease or disorder associated with administration of the chemotherapeutical drug.

In some embodiments of the method, the subject has been, is being and/or will be administered with the chemotherapeutical drug.

In another aspect, the present application provides use of a combination of thymidine derivative and uridine derivative in preparation of a drug for preventing or treating a disease or disorder associated with administration of the chemotherapeutical drug in a subject, wherein at least one hydroxyl hydrogen of a deoxyribose in the thymidine derivative is substituted.

In some embodiments, the thymidine derivative comprises a structure of Formula (I): 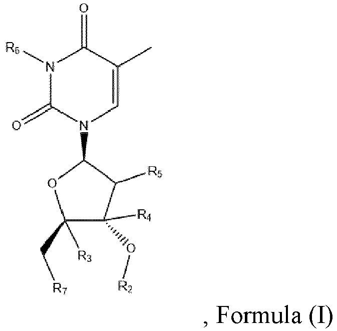 wherein R₂, R₃, R₄, R₅, R₆ and R₇ are not simultaneously hydrogen.

In some embodiments, R₇ is 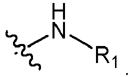 In some embodiments, R₇ is

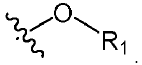

In some embodiments, R₆ is hydrogen.

In some embodiments, R₆ is 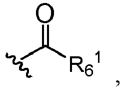 wherein the R₆¹ is C₁-C₆ alkyl. In some embodiments, R₆ is

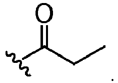

In some embodiments, R₁ is 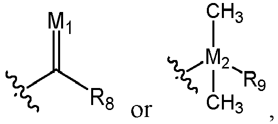 wherein M₁ is oxygen or sulfur, and R₈ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; M₂ is silicon or carbon, and R₉ is selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; R₉ is selected from the group consisting of C₁-C₅ alkyl, C₁-C₅ cycloalkyl, phenyl or benzyl.

In some embodiments, R₂ is 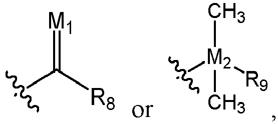 wherein M₁ is oxygen or sulfur, and R₈ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; M₂ is silicon or carbon, and R₉ is selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; R₉ is selected from the group consisting of C₁-C₅ alkyl, C₁-C₅ cycloalkyl, phenyl or benzyl.

In some embodiments, R₃ is 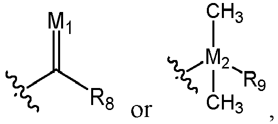 wherein M₁ is oxygen or sulfur, and R₈ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; M₂ is silicon or carbon, and R₉ is selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; R₉ is selected from the group consisting of C₁-C₅ alkyl, C₁-C₅ cycloalkyl, phenyl or benzyl.

In some embodiments, R₁ is selected from the group consisting of -(C=O)-R₈¹, -(C=O)-OR₈², -(C=O)-NR₈³R₈⁴, -(C=S)-R₈⁵, -(C=S)-OR₈⁶ and -(C=S)-NR₈⁷R₈⁸, wherein any one of R₈¹, R₈², R₈³, R₈⁴, R₈⁵, R₈⁶, R₈⁷ and R₈⁸ independently comprises one or more groups selected from the group consisting of hydrogen, substituted or unsubstituted alkyl, substituted or unsubstituted alkynyl, substituted or unsubstituted cycloalkyl, substituted or unsubstituted heterocycloalkyl, substituted or unsubstituted aryl, and substituted or unsubstituted heteroaryl.

In some embodiments, R₂ is selected from the group consisting of -(C=O)-R₈¹, -(C=O)-OR₈², -(C=O)-NR₈³R₈⁴, -(C=S)-R₈⁵, -(C=S)-OR₈⁶ and -(C=S)-NR₈⁷R₈⁸, wherein any one of R₈¹, R₈², R₈³, R₈⁴, R₈⁵, R₈⁶, R₈⁷ and R₈⁸ independently comprises one or more groups selected from the group consisting of hydrogen, substituted or unsubstituted alkyl, substituted or unsubstituted alkynyl, substituted or unsubstituted cycloalkyl, substituted or unsubstituted heterocycloalkyl, substituted or unsubstituted aryl, and substituted or unsubstituted heteroaryl.

In some embodiments, R₃ is selected from the group consisting of -(C=O)-R₈¹, -(C=O)-OR₈², -(C=O)-NR₈³R₈⁴, -(C=S)-R₈⁵, -(C=S)-OR₈⁶ and -(C=S)-NR₈⁷R₈⁸, wherein any one of R₈¹, R₈², R₈³, R₈⁴, R₈⁵, R₈⁶, R₈⁷ and R₈⁸ independently comprises one or more groups selected from the group consisting of hydrogen, substituted or unsubstituted alkyl, substituted or unsubstituted alkynyl, substituted or unsubstituted cycloalkyl, substituted or unsubstituted heterocycloalkyl, substituted or unsubstituted aryl, and substituted or unsubstituted heteroaryl.

In some embodiments, any one of R₈¹, R₈², R₈³, R₈⁴, R₈⁵, R₈⁶, R₈⁷ and R₈⁸ independently comprises one or more groups selected from the group consisting of hydrogen, C₁-C₅ alkyl, C₁-C₅ cycloalkyl, C₁-C₅ heterocycloalkyl, phenyl or benzyl.

In some embodiments, R₈¹ comprises one or more groups selected from the group consisting of C₁-C₅ alkyl, C₁-C₅ cycloalkyl, phenyl, and benzyl. In some embodiments, R₈¹ comprises one or more groups selected from the group consisting of methyl, ethyl, propyl, butyl, cyclopentyl, cyclopropyl, and benzyl.

In some embodiments, R₈² comprises one or more groups selected from the group consisting of C₁-C₅ alkyl, C₁-C₅ cycloalkyl, phenyl, and benzyl. In some embodiments, R₈² comprises one or more groups selected from the group consisting of cyclopropyl, propyl, butyl, pentyl, phenyl, and benzyl.

In some embodiments, R₈³ is hydrogen.

In some embodiments, R₈⁴ comprises one or more groups selected from the group consisting of C₁-C₅ alkyl, C₁-C₅ cycloalkyl, phenyl, and benzyl. In some embodiments, R₈⁴ comprises one or more groups selected from the group consisting of cyclopropyl, cyclopentyl, and phenyl.

In some embodiments, R₉ is butyl.

In some embodiments, R₃ is hydrogen. In some embodiments, R₄ is hydrogen. In some embodiments, R₅ is hydrogen.

In some embodiments, R₁ is 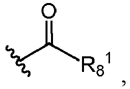 wherein R₈¹ comprises one or more groups selected from the group consisting of C₁-C₆ alkyl, C₁-C₆ silyl, C₁-C₆ alkoxy, C₁-C₆ alkylnitro, C₃-C₇ cycloalkyl, C₃-C₇ cycloalkylnitro, C₄-C₇ aryl, C₄-C₇ alkoxyaryl and/or C₄-C₇ heteroaryl. In some embodiments, R₂ is 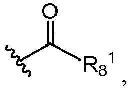 wherein R₈¹ comprises one or more groups selected from the group consisting of C₁-C₆ alkyl, C₁-C₆ silyl, C₁-C₆ alkoxy, C₁-C₆ alkylnitro, C₃-C₇ cycloalkyl, C₃-C₇ cycloalkylnitro, C₄-C₇ aryl, C₄-C₇ alkoxyaryl and/or C₄-C₇ heteroaryl.

In some embodiments, in the structure of Formula (I), R₃, R₄, R₅ and R₆ are all hydrogen, R₇ is 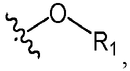 R₁ comprises one or more groups selected from the group consisting of hydrogen, 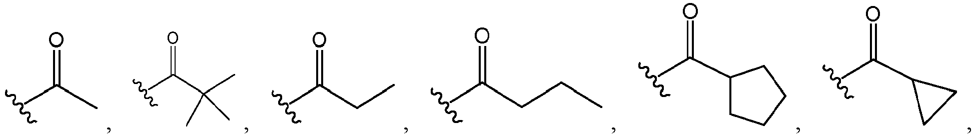 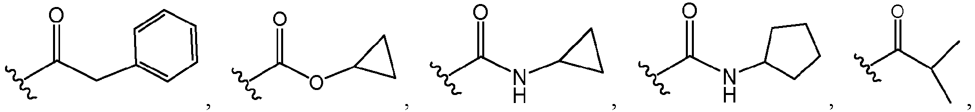 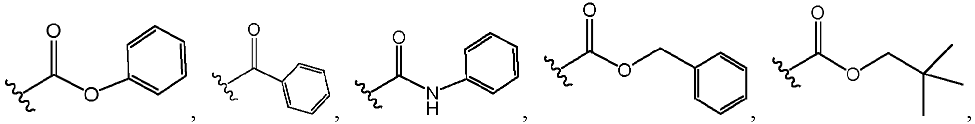 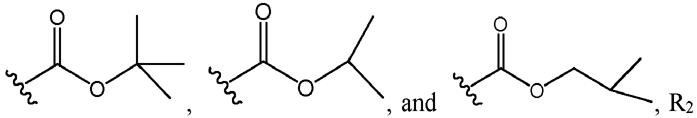 comprises one or more groups selected from the group consisting of hydrogen, 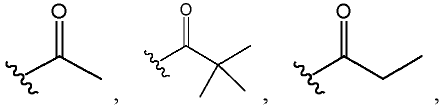 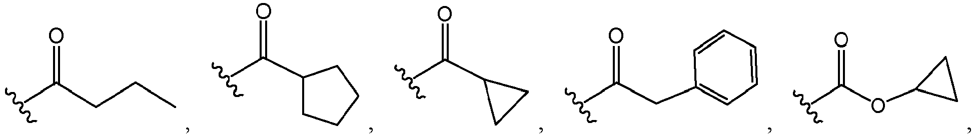 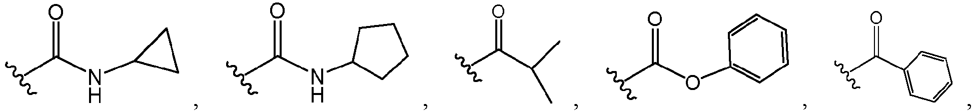 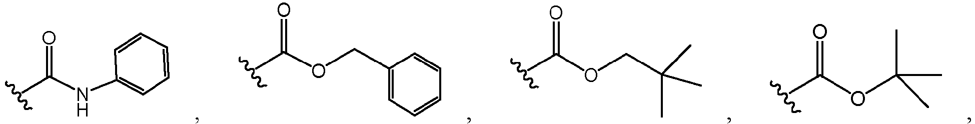 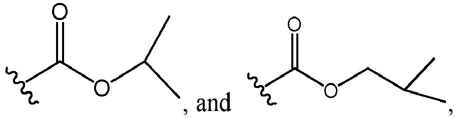 and R₂ and R₁ are not simultaneously hydrogen.

In some embodiments, the thymidine derivative comprises one or more of compound T1 to compound T24 selected from the following group:

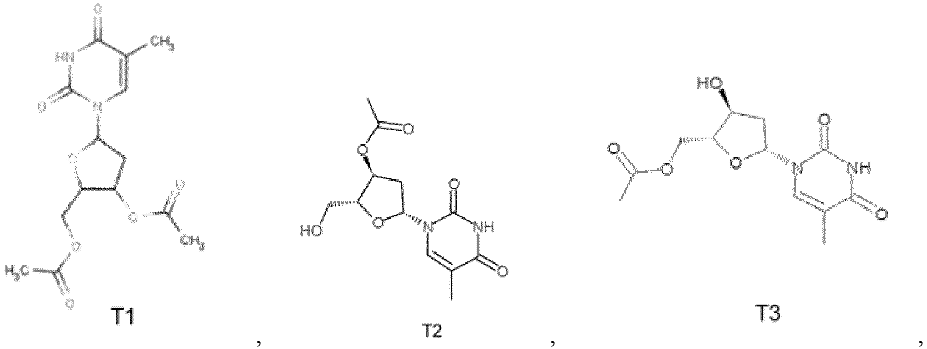

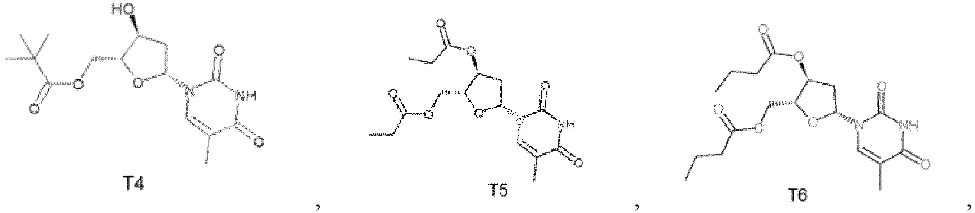

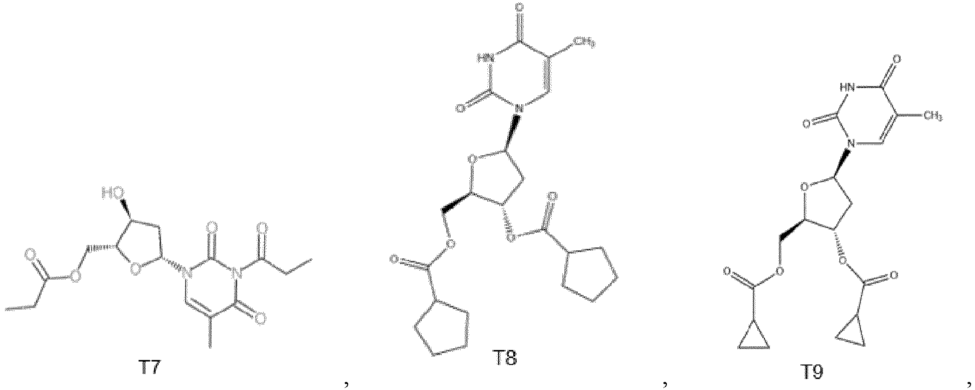

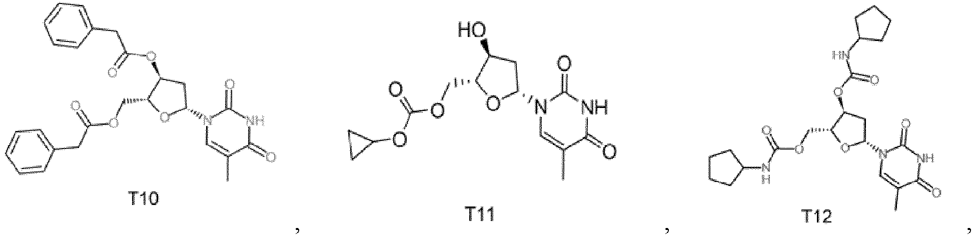

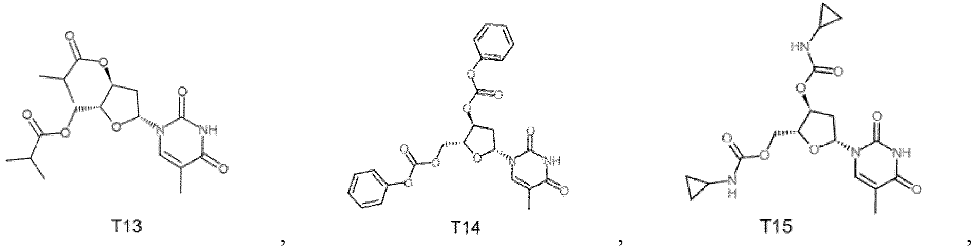

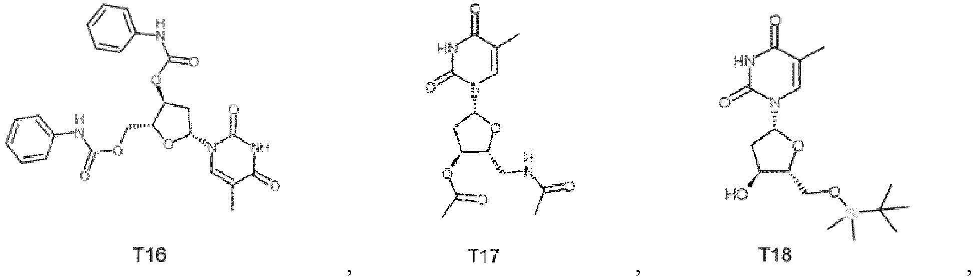

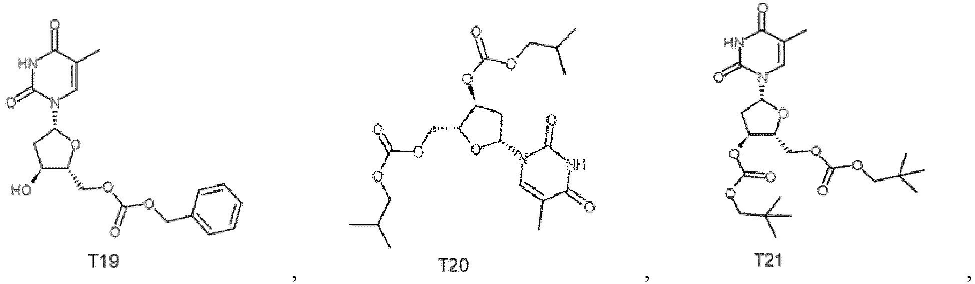

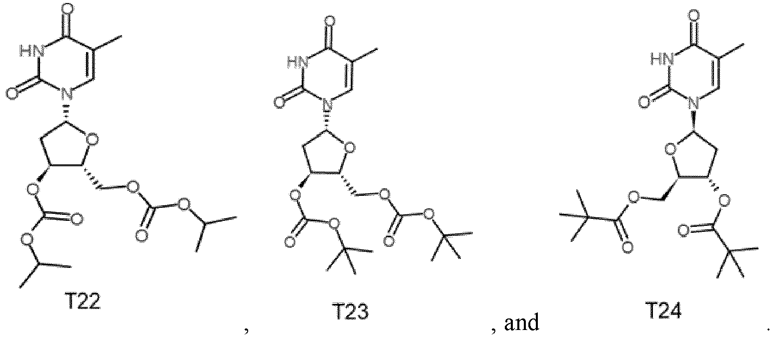

In some embodiments, the thymidine derivative comprises a structure of Formula (II): 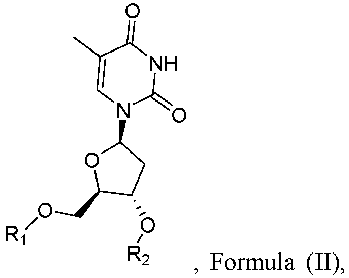 wherein R₁ and/or R₂ comprise/comprises a non-steroidal anti-inflammatory drug (NSAID) moiety.

In some embodiments, the NSAID moiety comprises salicylic acid or a derivative thereof, aryl acetic acid or a derivative thereof, heteroaryl acetic acid or a derivative thereof, indoleacetic acid or a derivative thereof, indene acetic acid or a derivative thereof, anthranilic acid or a derivative thereof and/or enolic acid or a derivative thereof.

In some embodiments, R₁ or R₂ is hydrogen.

In some embodiments, R₁ and R₂ are not simultaneously hydrogen.

In some embodiments, R₁ and/or R₂ are/is 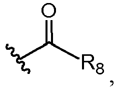 wherein R₈ is Rₛ² or 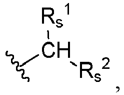 wherein Rₛ¹ is hydrogen or methyl, Rₛ² is 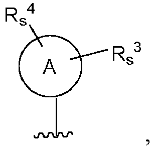 wherein the ring A is C₄-C₇ aryl, C₄-C₇ heteroaryl, indene ring, naphthalene ring, indoline ring, unsaturated polycyclic hydrocarbon and/or heterocyclic polycycle, Rs³ and/or Rs⁴ are/is independently selected from the group consisting of hydrogen, C₁-C₆ alkyl, C₁-C₆ alkyl ester, halogen, C₄-C₇ aryl, C₄-C₇ heteroaryl, and 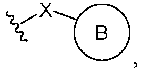 wherein ring B is C₄-C₇ aryl, C₄-C₇ heteroaryl, X is -CH₂, -NH-, -O- or 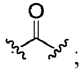 wherein the C₄-C₇ aryl, C₄-C₇ heteroaryl are optionally substituted with one or more substituents selected from the group consisting of halogen, C₁-C₆ alkyl, C₁-C₆ alkynyl, and C₁-C₆ alkenyl.

In some embodiments, ring A is pyrrole ring, Rₛ³ is C₁-C₆ alkyl, Rₛ⁴ is 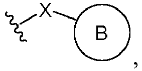 wherein Xis 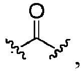 ring B is benzene ring, and the ring B is optionally substituted with one or more C₁-C₆ alkyl.

In some embodiments, Rₛ¹ and/or Rₛ² are/is 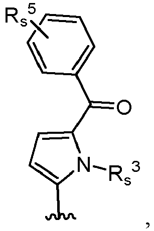 wherein the Rₛ³ is C₁-C₆ alkyl or halogen.

In some embodiments, Rₛ¹ and/or Rₛ² are/is 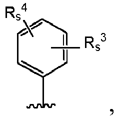 wherein Rₛ³ and/or Rₛ⁴ are/is selected from the group consisting of hydrogen, C₁-C₆ alkyl, C₁-C₆ alkyl ester, halogen, C₄-C₇ aryl, C₄-C₇ heteroaryl, 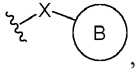 ring B is C₄-C₇ aryl, C₄-C₇ heteroaryl, X is -CH₂, -NH-, -O- or 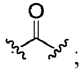 wherein the C₄-C₇ aryl, C₄-C₇ heteroaryl are optionally substituted with one or more substituents selected from the group consisting of halogen, C₁-C₆ alkyl, C₁-C₆ alkynyl, and C₁-C₆ alkenyl.

In some embodiments, Rₛ² is 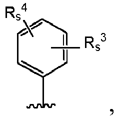 wherein Rₛ³ and/or Rₛ⁴ are/is selected from the group consisting of hydrogen, C₁-C₆ alkyl, 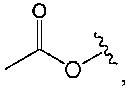 fluorine, chlorine, bromine, benzene ring, and 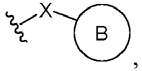 wherein ring B is benzene ring, X is -CH₂, -NH-, -O- or 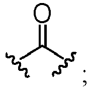 and the benzene ring is optionally substituted with one or more substituents selected from the group consisting of fluorine, chlorine and bromine.

In some embodiments, R₈ is 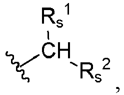 Rₛ¹ is hydrogen or methyl, Rₛ² is selected from the group consisting of

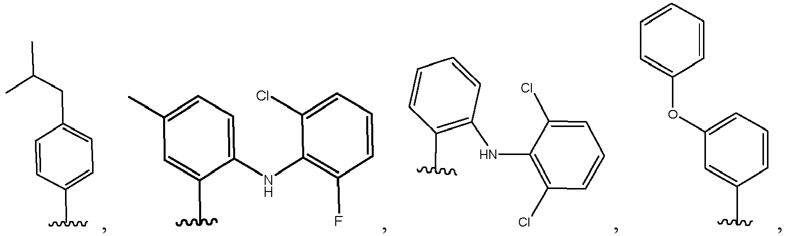

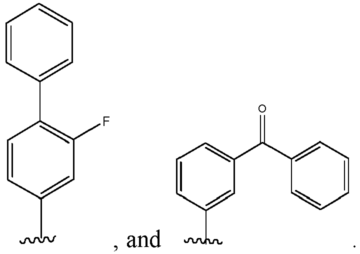

In some embodiments, R₈ is 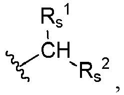 Rₛ¹ is hydrogen or methyl, Rₛ² is 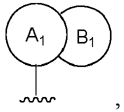 wherein ring A₁ is C₄-C₇ cycloalkyl, C₄-C₇ heterocycloalkyl, C₄-C₇ aryl and/or C₄-C₇ heteroaryl, ring B₁ is C₄-C₇ cycloalkyl, C₄-C₇ heterocycloalkyl, C₄-C₇ aryl, C₄-C₇ heteroaryl or 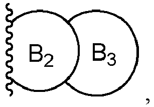 wherein ring B₂ is C₄-C₇ cycloalkyl, C₄-C₇ heterocycloalkyl, C₄-C₇ aryl and/or C₄-C₇ heteroaryl, ring B₃ is C₄-C₇ cycloalkyl, C₄-C₇ heterocycloalkyl, C₄-C₇ aryl and/or C₄-C₇ heteroaryl, wherein the C₄-C₇ cycloalkyl, C₄-C₇ heterocycloalkyl, C₄-C₇ aryl and/or C₄-C₇ heteroaryl are optionally substituted with halogen, 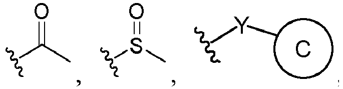 C₁-C₆ alkyl, C₁-C₆ alkyl-substituted ester groups and/or C₁-C₆ alkyl-substituted aldehyde groups, wherein ring C is benzene ring, Y is -CH₂, -NH-, - O- or 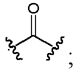 the benzene ring is optionally substituted with one or more substituents selected from the group consisting of fluorine, chlorine, bromine, and 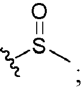 the Y may form a double bond with a ring atom on ring B₂ or ring B₃.

In some embodiments, Rₛ² is 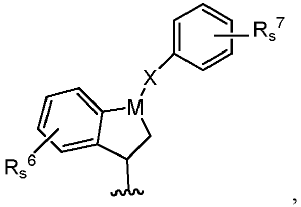 wherein the Rₛ⁶ is fluorine, chlorine or bromine, 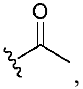 M is nitrogen or carbon, X is carbon or 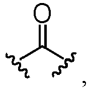 a double bond is optionally formed between X and M, Rₛ⁷ is fluorine, chlorine, bromine or

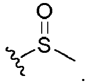

In some embodiments, R₈ is 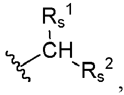 Rₛ¹ is hydrogen or methyl, Rₛ² is 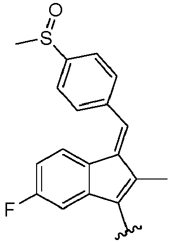 or

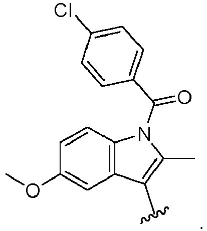

In some embodiments, R₈ is 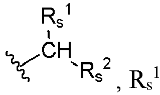 is hydrogen or methyl, Rₛ² is

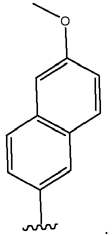

In some embodiments, Rₛ² is 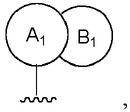 the ring A₁ is benzene ring, ring B₁ is 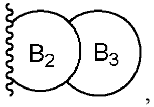 and ring B₂ is pyrrole ring, B₃ is pyran ring, and the pyran ring is optionally substituted with one or more C₁-C₆ alkyl and/or C₁-C₆ alkyl-substituted aldehyde groups.

In some embodiments, R₈ is 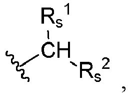 Rₛ¹ is hydrogen or methyl, and Rₛ² is

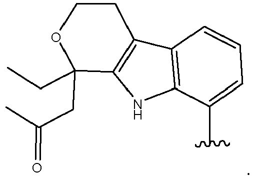

In some embodiments, R₈¹ is

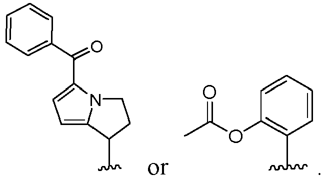

In some embodiments, R₁ and/or R₂ are/is

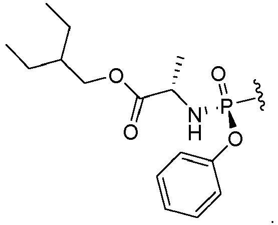

In some embodiments, the R₁ comprises the NSAID moiety, and R₂ is hydrogen.

In some embodiments, the R₁ comprises any one group selected from the following group: 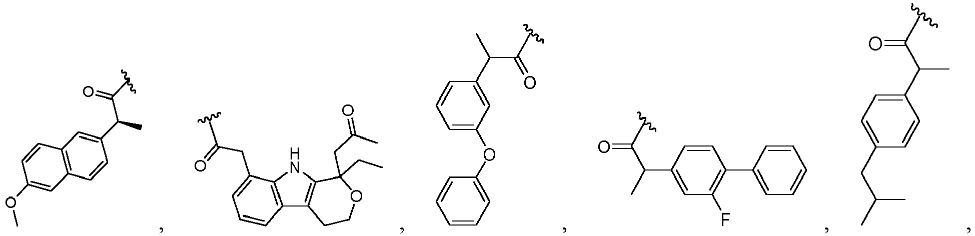 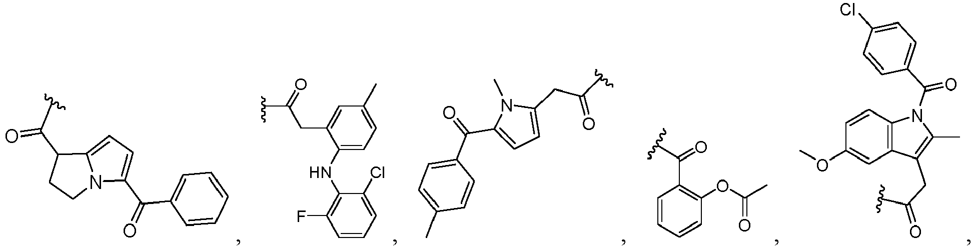 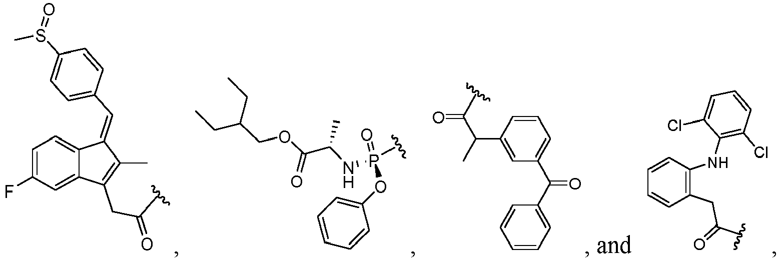 and R₂ is hydrogen.

In some embodiments, the R₂ comprises the NSAID moiety, and R₁ is hydrogen.

In some embodiments, the R₂ is any one group selected from the following group: 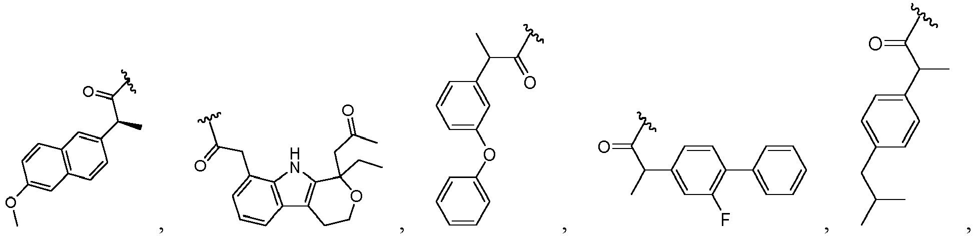 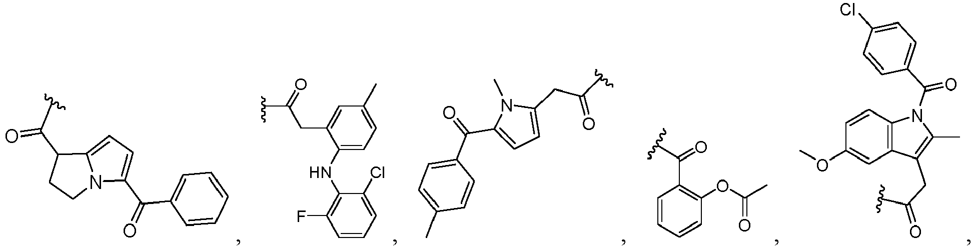 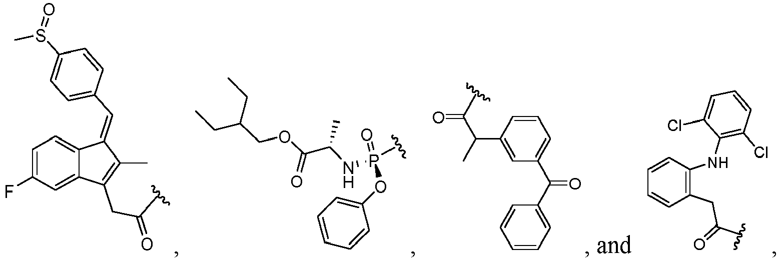 and R₁ is hydrogen.

In some embodiments, the R₁ and R₂ both comprise the NSAID moiety.

In some embodiments, the R₁ and R₂ are each independently any one group selected from the group consisting of:

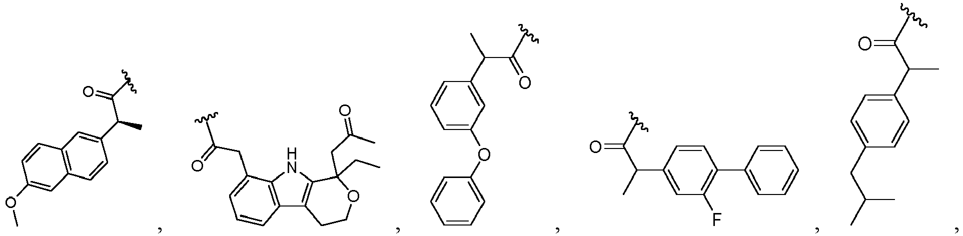

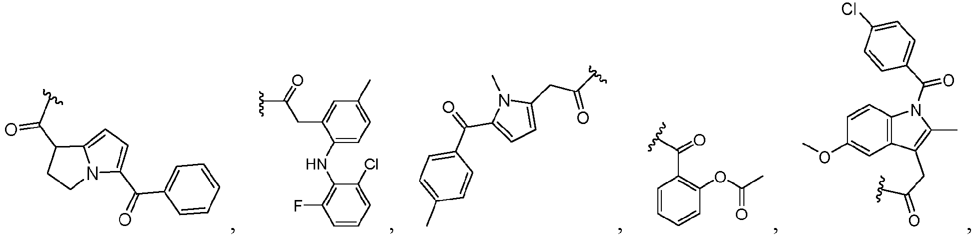

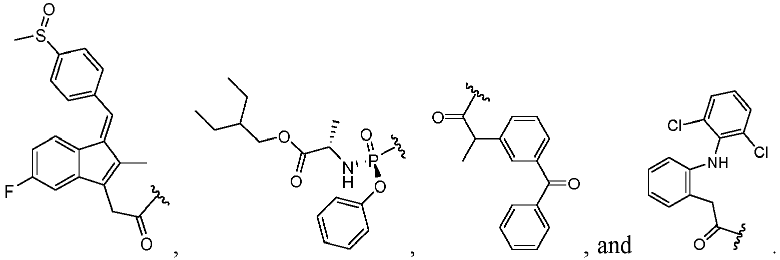

In some embodiments, the thymidine derivative is one or more selected from the group consisting of: 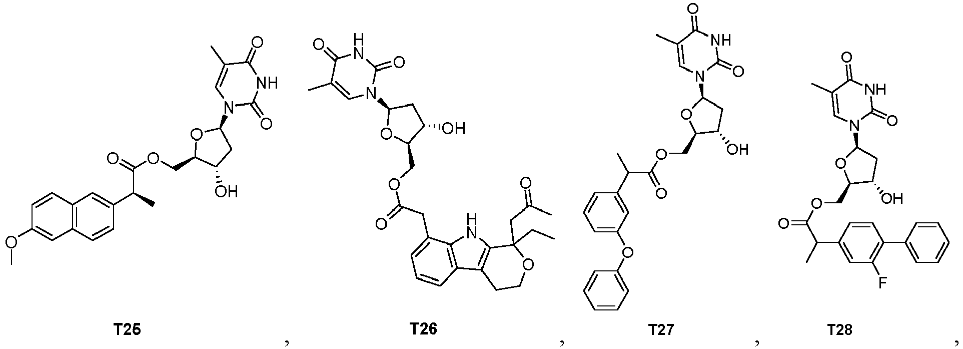 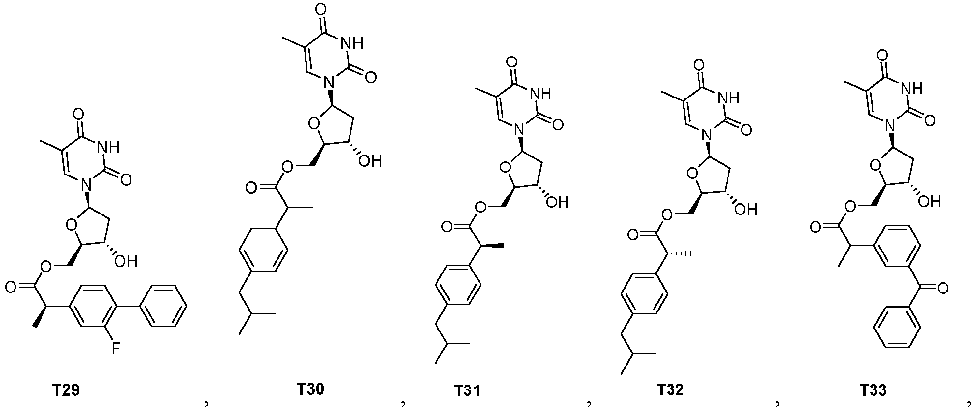 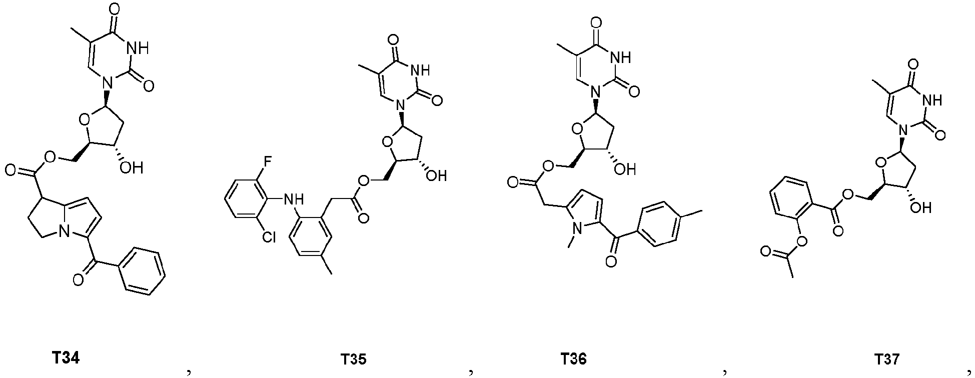 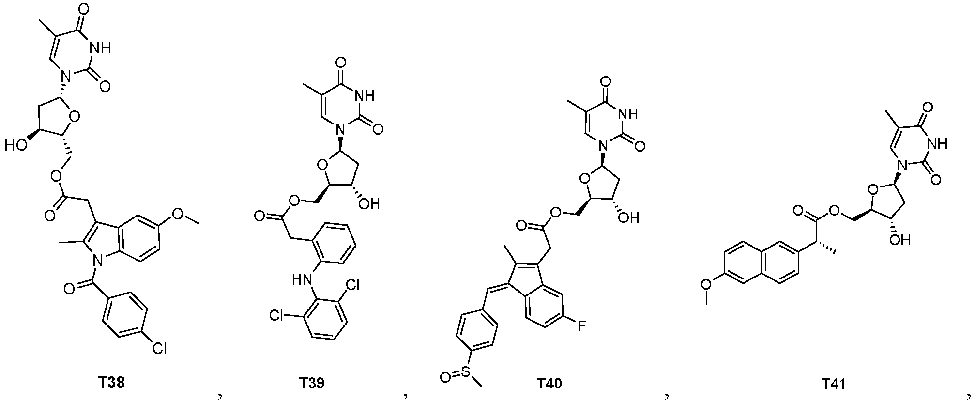 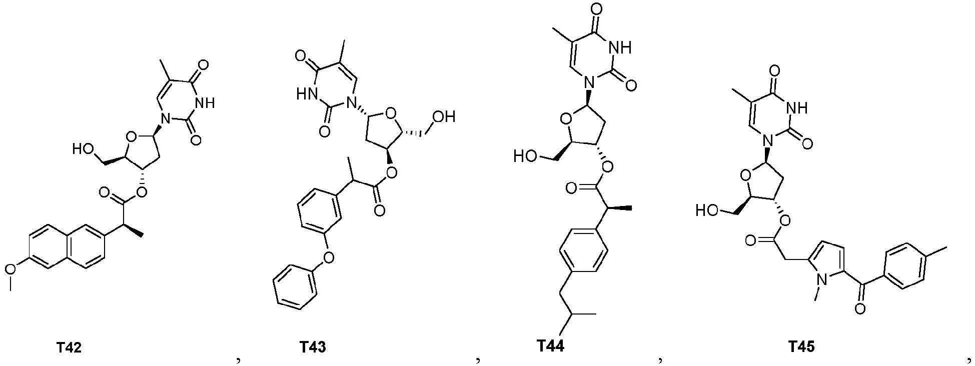 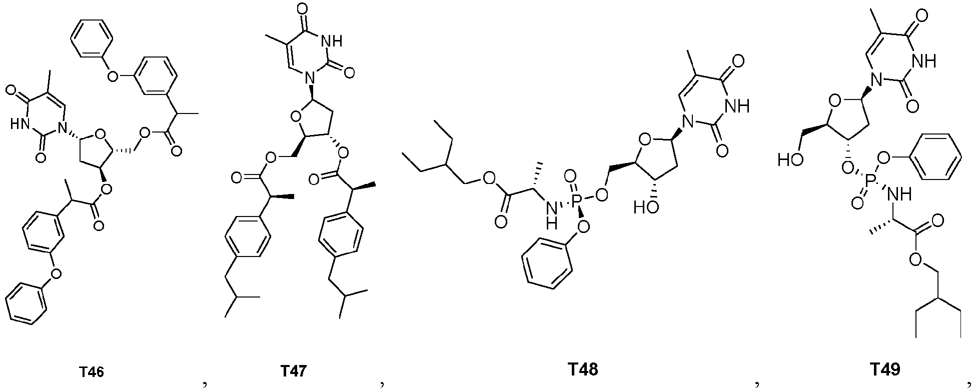 and

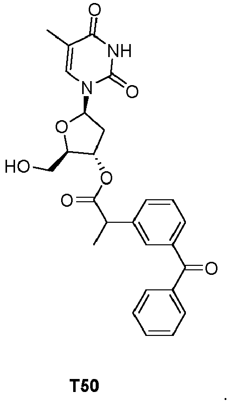

In some embodiments, the uridine derivative can comprise a structure of Formula (III): 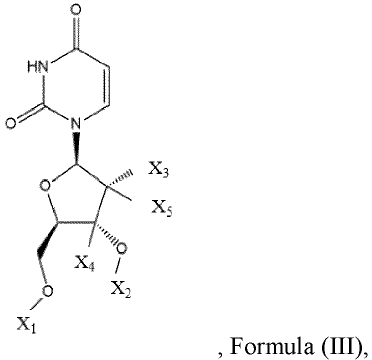 wherein when X₄ and X₅ are both hydrogen, X₁, X₂ and X₃ are not simultaneously hydrogen or are not simultaneously CH₃CO-.

In some embodiments, X₁ is hydrogen or 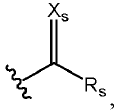 wherein the Xₛ is oxygen or sulfur, Rₛ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted C₁-C₅ cycloalkyl, substituted or unsubstituted heterocycloalkyl, substituted or unsubstituted aryl, and substituted or unsubstituted aralkyl. In some embodiments, Xₛ is oxygen.

In some embodiments, X₂ is hydrogen or 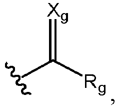 wherein the X_{g} is oxygen or sulfur, R_{g} comprises one or more groups selected from the group consisting of hydrogen, substituted or unsubstituted hydroxyl, substituted or unsubstituted sulfydryl substituted or unsubstituted amino, substituted or unsubstituted C₁-C₅ alkyl, substituted or unsubstituted C₁-C₅ alkynyl, substituted or unsubstituted C₁-C₅ cycloalkyl, substituted or unsubstituted heterocycloalkyl, substituted or unsubstituted aryl, and substituted or unsubstituted aralkyl. In some embodiments, X_{g} is oxygen.

In some embodiments, X₃ is 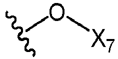 or hydrogen, wherein the X₇ is hydrogen or 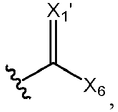 wherein the X₁' is oxygen or sulfur, X₆ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted C₁-C₅ cycloalkyl, substituted or unsubstituted heterocycloalkyl, substituted or unsubstituted aryl, and substituted or unsubstituted aralkyl. In some embodiments, X₁' is oxygen.

In some embodiments, X₃ is 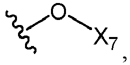 the C₇ is 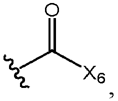 wherein X₆ comprises one or more groups selected from the group consisting of hydrogen, C₁-C₆ alkyl, C₁-C₆ alkoxy, C₃-C₁₀ cycloalkyl, C₃-C₁₀ cycloalkoxy, C₄-C₁₀ aralkyl, C₄-C₁₀ aralkoxy or C₄-C₁₀ aryl.

In some embodiments, the X₃ is hydrogen.

In some embodiments, the X₁ is 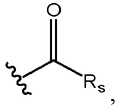 wherein Rₛ comprises one or more groups selected from the group consisting of hydrogen, C₁-C₆ alkyl, C₁-C₆ alkoxy, C₃-C₁₀ cycloalkyl, C₃-C₁₀ cycloalkoxy, C₄-C₁₀ aralkyl, C₄-C₁₀ aralkoxy or C₄-C₁₀ aryl.

In some embodiments, the X₂ is 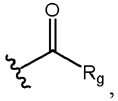 wherein R_{g} comprises one or more groups selected from the group consisting of hydrogen, C₁-C₆ alkyl, C₁-C₆ alkoxy, C₃-C₁₀ cycloalkyl, C₃-C₁₀ cycloalkoxy, C₄-C₁₀ aralkyl, C₄-C₁₀ aralkoxy or C₄-C₁₀ aryl.

In some embodiments, X₄ is hydrogen. In some embodiments, X₅ is hydrogen.

In some embodiments, the uridine derivative is one or more selected from U1 to U13.

In some embodiments, the uridine derivative comprises a compound of Formula (IV): 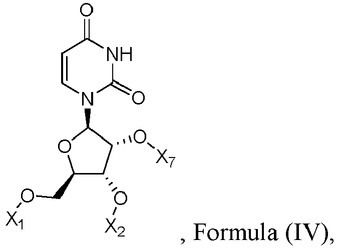 wherein at least one of the X₁, X₂ and X₇ comprises a non-steroidal anti-inflammatory drug (NSAID) moiety.

In some embodiments, the NSAID moiety comprises salicylic acid or a derivative thereof, aryl acetic acid or a derivative thereof, heteroaryl acetic acid or a derivative thereof, indoleacetic acid or a derivative thereof, indene acetic acid or a derivative thereof, anthranilic acid or a derivative thereof and/or enolic acid or a derivative thereof.

In some embodiments, X₁, X₂ or X₇ is hydrogen. In some embodiments, X₁, X₂ and X₇ are not simultaneously hydrogen.

In some embodiments, any one of X₁, X₂ and X₇ is independently 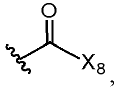 wherein X₈ is Xₛ² or 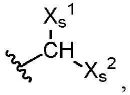 wherein Xₛ¹ is hydrogen or methyl, Xₛ² is 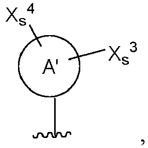 wherein the ring A' is C₄-C₇ aryl, C₄-C₇ heteroaryl, indene ring, naphthalene ring, indoline ring, unsaturated polycyclic hydrocarbon and/or heterocyclic polycycle, Xs³ and/or Xs⁴ are/is independently selected from the group consisting of hydrogen, C₁-C₆ alkyl, C₁-C₆ alkyl ester, halogen, C₄-C₇ aryl, C₄-C₇ heteroaryl, and 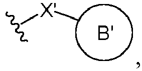 wherein ring B' is C₄-C₇ aryl, C₄-C₇ heteroaryl, X' is -CH₂, -NH-, -O- or 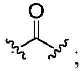 wherein the C₄-C₇ aryl, C₄-C₇ heteroaryl are optionally substituted with one or more substituents selected from the group consisting of halogen, C₁-C₆ alkyl, C₁-C₆ alkynyl, and C₁-C₆ alkenyl.

In some embodiments, X₈ is 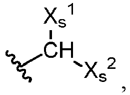 Xₛ¹ is hydrogen or methyl, and Xₛ² is selected from 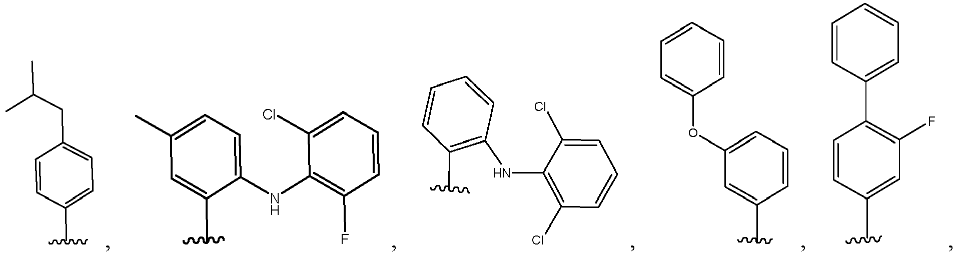 and

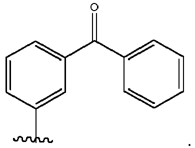

In some embodiments, X₈ is 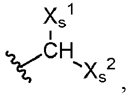 Xₛ¹ is hydrogen or methyl, Xₛ² is 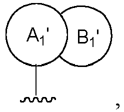 wherein ring A₁' is C₄-C₇ cycloalkyl, C₄-C₇ heterocycloalkyl, C₄-C₇ aryl and/or C₄-C₇ heteroaryl, ring B₁' is C₄-C₇ cycloalkyl, C₄-C₇ heterocycloalkyl, C₄-C₇ aryl, C₄-C₇ heteroaryl or 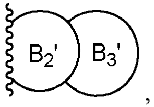 wherein ring B₂' is C₄-C₇ cycloalkyl, C₄-C₇ heterocycloalkyl, C₄-C₇ aryl and/or C₄-C₇ heteroaryl, ring B₃' is C₄-C₇ cycloalkyl, C₄-C₇ heterocycloalkyl, C₄-C₇ aryl and/or C₄-C₇ heteroaryl, wherein the C₄-C₇ cycloalkyl, C₄-C₇ heterocycloalkyl, C₄-C₇ aryl and/or C₄-C₇ heteroaryl are/is optionally substituted with halogen, 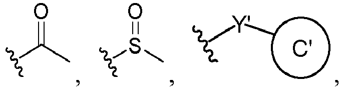 C₁-C₆ alkyl, C₁-C₆ alkyl-substituted ester groups and/or C₁-C₆ alkyl-substituted aldehyde groups, wherein ring C' is benzene ring, Y' is -CH₂, -NH-, - O- or 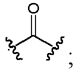 the benzene ring is optionally substituted with one or more substituents selected from the group consisting of fluorine, chlorine, bromine and 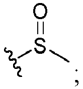 and the Y' may form a double bond with a ring atom on ring B₂ or ring B₃.

In some embodiments, X₈ is 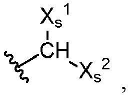 Xₛ¹ is hydrogen or methyl, and Xₛ² is

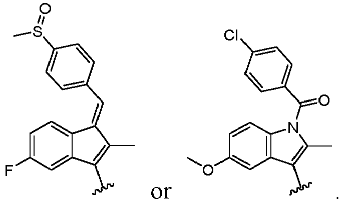

In some embodiments, X₈ is 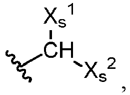 Xₛ¹ is hydrogen or methyl, and Xₛ² is

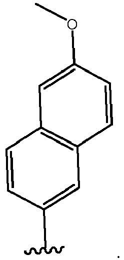

In some embodiments, X₈ is 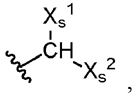 Xₛ¹ is hydrogen or methyl, and Xₛ² is

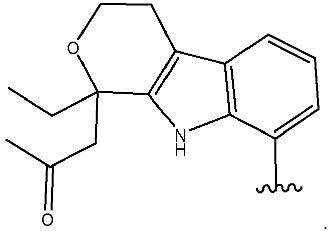

In some embodiments, X₈¹ is

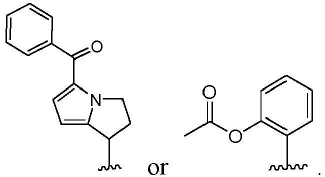

In some embodiments, any one of X₁, X₂ and X₇ is independently

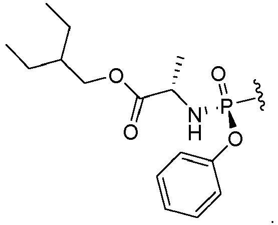

In some embodiments, at least one of X₁, X₂ and X₇ (e.g., X₁, X₂, X₇, X₁ and X₂, X₂ and X₇, X₁ and X₇, alternatively, X₁, X₂ and X₇) comprises a NSAID moiety, with the remaining being hydrogen.

In some embodiments, the X₁ is selected from the group consisting of:

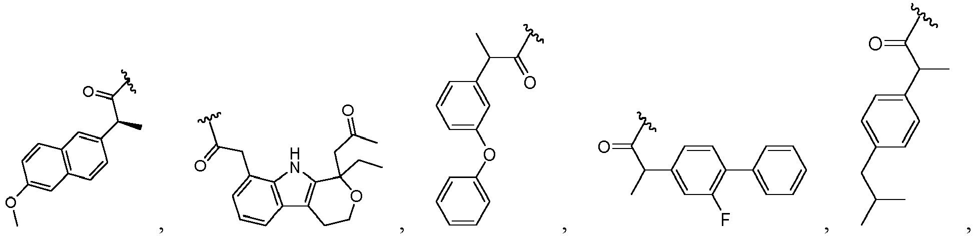

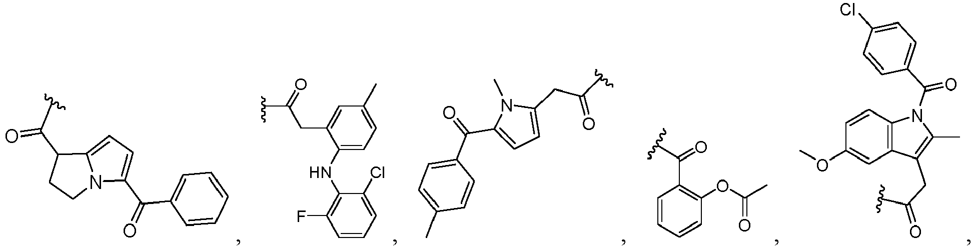

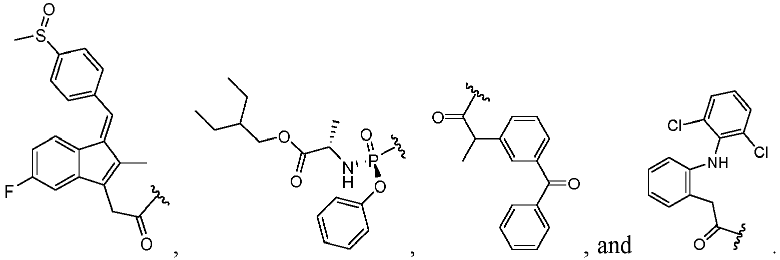

In some embodiments, the X₁ is selected from the group consisting of:

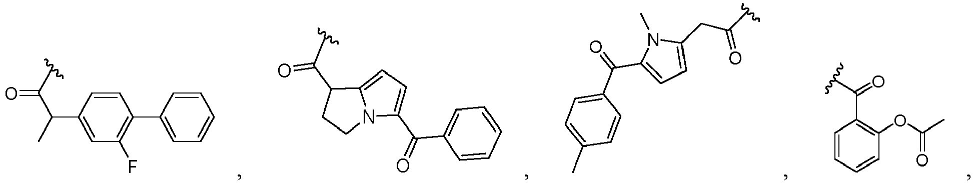

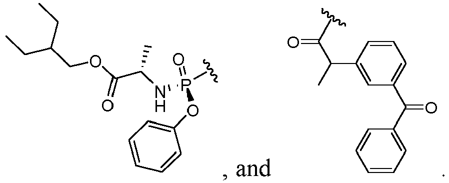

In some embodiments, the X₂ is selected from the group consisting of:

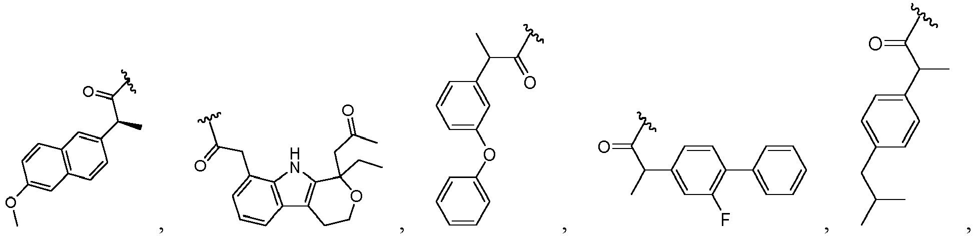

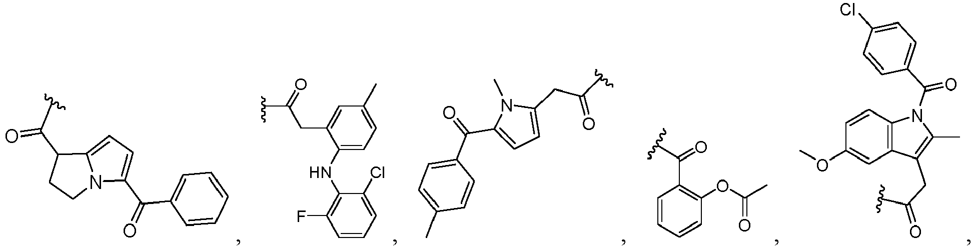

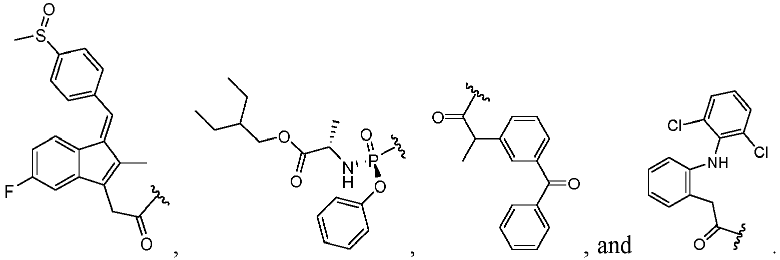

In some embodiments, the X₂ is selected from the group consisting of: hydrogen,

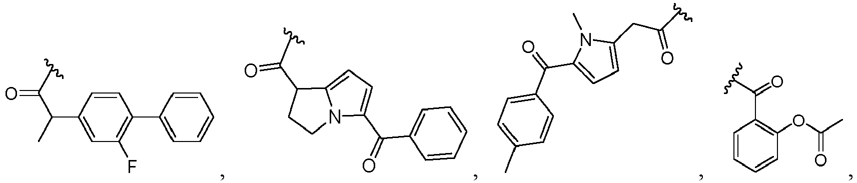

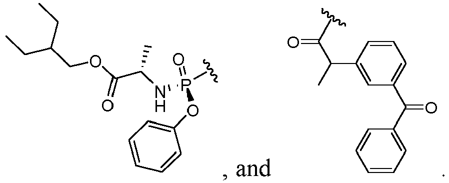

In some embodiments, the X₇ is selected from the group consisting of:

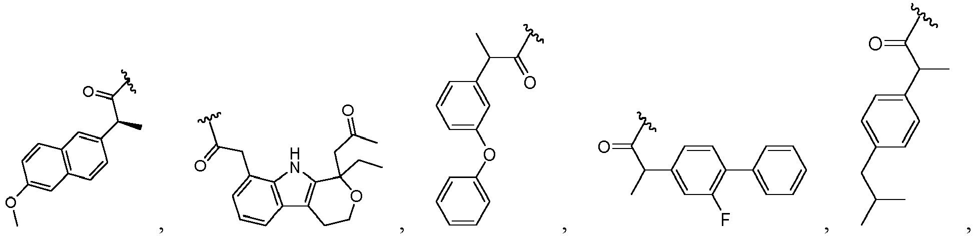

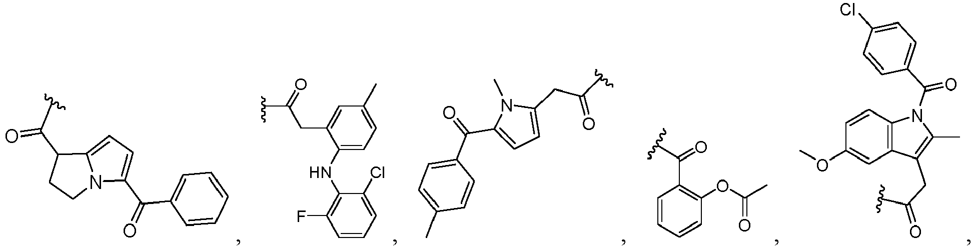

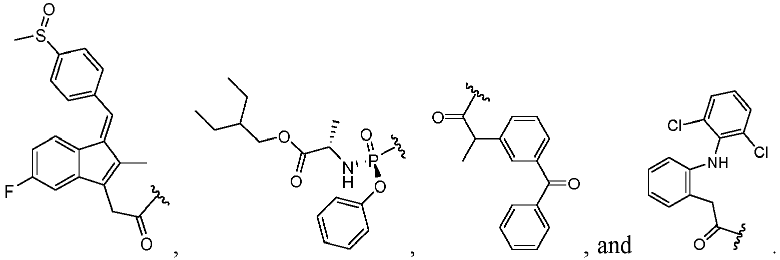

In some embodiments, the X₇ is selected from the group consisting of:

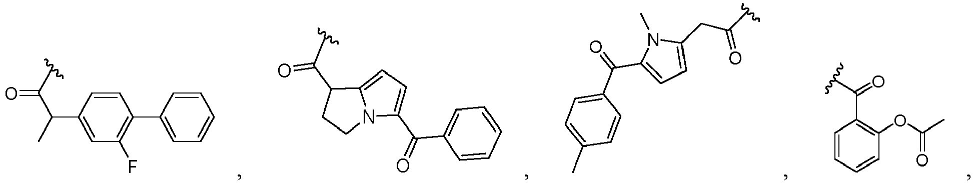

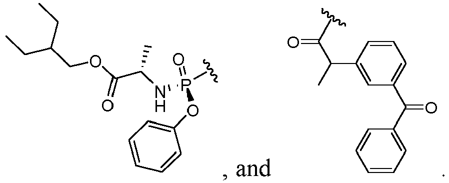

In some embodiments, when one or more of X₁, X₂ or X₇ are each independently selected from specific groups in the above group, those of X₁, X₂ or X₇ not selected from the above group are hydrogen; however, X₁, X₂ or X₇ is not simultaneously hydrogen. In some embodiments, X₁ X₂ or X₇ may be the same group. In some embodiments, two of X₁, X₂ or X₇ are the same group. In some embodiments, X₁, X₂ or X₇ are different groups from each other.

In some embodiments, the uridine derivative is one or more selected from the group consisting of:

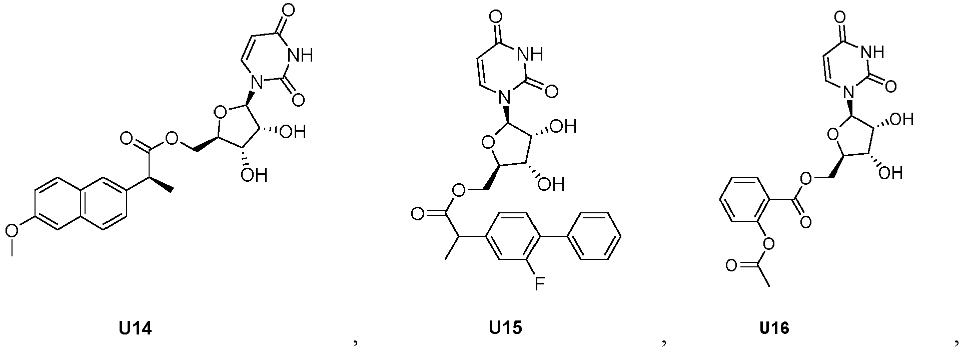

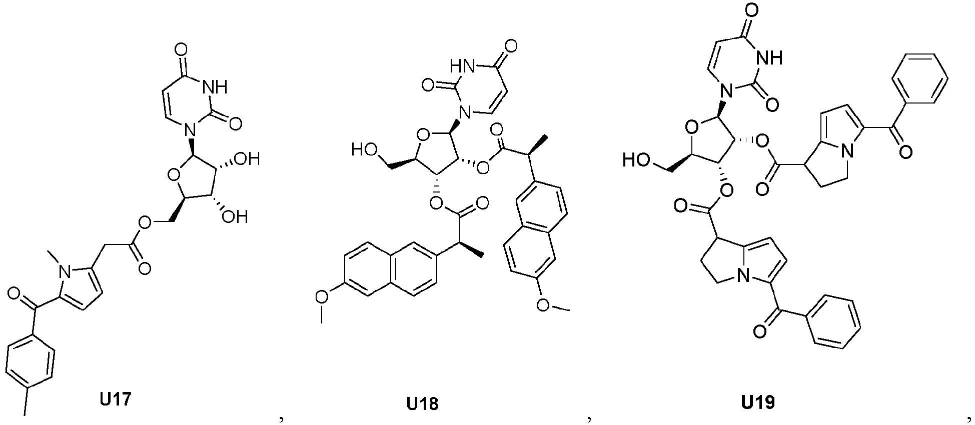

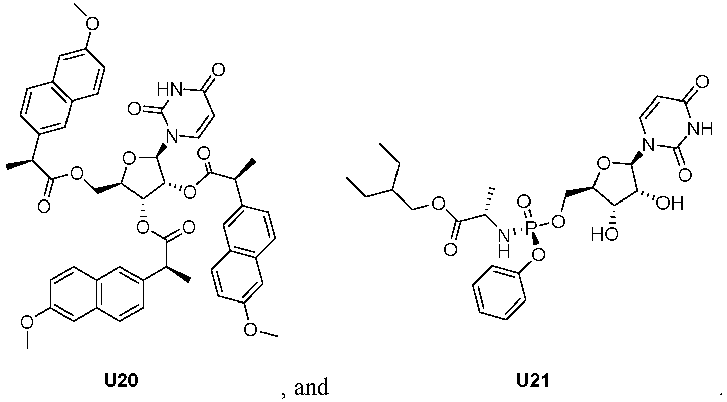

In some embodiments of the use, the chemotherapeutical drug is used for treating cancers.

In some embodiments, the chemotherapeutical drug comprises a pyrimidine nucleoside analog or a prodrug thereof.

In some embodiments, the chemotherapeutical drug comprises one or more selected from the group consisting of capecitabine, cytarabine, docetaxel, adriamycin, fluorouracil (5-FU), floxuridine, tegafur, idarubicin, paclitaxel, epirubicin, acelarin (NUC-1031), doxorubicin, folinic acid, cis-platinum, paclitaxel, cyclophosphamide, vincristine and 5-FU pro-drugs (e.g., tegafur and 5'-deoxyfloxuridine, floxuridine, 2'-deoxyfloxuridine, a pro-drug derivative of floxuridine or a pro-drug derivative of 2'-deoxyfloxuridine, trifluoro-methyl-2'-deoxyuridine, 6-azauridine or 3-deazauridine).

In some embodiments of the use, the disease or disorder is caused by administration of the chemotherapeutical drug.

In some embodiments, the disease or disorder associated with administration of the chemotherapeutical drug comprises skin tissue disease or disorder associated with administration of the chemotherapeutical drug, hemopoietic tissue disease or disorder associated with administration of the chemotherapeutical drug, limb disease or disorder associated with administration of the chemotherapeutical drug, cardiac disease or disorder associated with administration of the chemotherapeutical drug, nervous system disease or disorder associated with administration of the chemotherapeutical drug, facial feature disease or disorder associated with administration of the chemotherapeutical drug and/or gastrointestinal disease or disorder associated with administration of the chemotherapeutical drug.

In some embodiments, the hemopoietic tissue disease or disorder associated with administration of the chemotherapeutical drug comprises marrow disease or disorder associated with administration of the chemotherapeutical drug and blood disease or disorder associated with administration of the chemotherapeutical drug.

In some embodiments, the hemopoietic tissue disease or disorder associated with administration of the chemotherapeutical drug comprises abnormal blood cell proliferative disease or disorder associated with administration of the chemotherapeutical drug.

In some embodiments, the disease or disorder associated with administration of the chemotherapeutical drug comprises epithelial tissue disease or disorder associated with administration of the chemotherapeutical drug.

In some embodiments, the epithelial tissue disease or disorder associated with administration of the chemotherapeutical drug comprises disease or disorder associated with endothelial cell lesion, and/or disease or disorder associated with epithelial cell lesion, and wherein the endothelial cell lesion and/or epithelial cell lesion are/is associated with administration of the chemotherapeutic drug.

In some embodiments, the endothelial cell comprises a vascular endothelial cell.

In some embodiments, the epithelial cell comprises skin epithelial cell, oral epithelial cell, nasal epithelial cell, gastric epithelial cell and/or intestinal epithelial cell.

In some embodiments, the disease or disorder associated with administration of the chemotherapeutical drug comprises rash associated with administration of the chemotherapeutical drug, hand-foot syndrome associated with administration of the chemotherapeutical drug, pruritus associated with administration of the chemotherapeutical drug, erythema associated with administration of the chemotherapeutical drug, dry skin associated with administration of the chemotherapeutical drug, alopecia associated with administration of the chemotherapeutical drug, paronychia associated with administration of the chemotherapeutical drug, pigmentation disorder associated with administration of the chemotherapeutical drug, oral ulcer associated with administration of the chemotherapeutical drug, xerostomia associated with administration of the chemotherapeutical drug, epistaxis associated with administration of the chemotherapeutical drug, nasopharyngitis associated with administration of the chemotherapeutical drug, cheilitis associated with administration of the chemotherapeutical drug, esophagus mucositis associated with administration of the chemotherapeutical drug, gastric mucositis associated with administration of the chemotherapeutical drug, gastric ulcer associated with administration of the chemotherapeutical drug, diarrhea associated with administration of the chemotherapeutical drug, vomiting associated with administration of the chemotherapeutical drug, nausea associated with administration of the chemotherapeutical drug, anorexia associated with administration of the chemotherapeutical drug, constipation associated with administration of the chemotherapeutical drug, or abdominal pain associated with administration of the chemotherapeutical drug, nonspecific chest pain associated with administration of the chemotherapeutical drug, angina pectoris associated with administration of the chemotherapeutical drug, palpitation associated with administration of the chemotherapeutical drug, dyspnea associated with administration of the chemotherapeutical drug, associated with administration of the chemotherapeutical drug diffuse pleuritic chest pain associated with administration of the chemotherapeutical drug, supraventricular arrhythmia associated with administration of the chemotherapeutical drug, hypotension associated with administration of the chemotherapeutical drug, myocardial infarction associated with administration of the chemotherapeutical drug, bradycardia associated with administration of the chemotherapeutical drug, arrhythmia associated with administration of the chemotherapeutical drug, ventricular fibrillation associated with administration of the chemotherapeutical drug, ventricular tachycardia associated with administration of the chemotherapeutical drug, myocarditis associated with administration of the chemotherapeutical drug, heart failure associated with administration of the chemotherapeutical drug, acute pneumonedema associated with administration of the chemotherapeutical drug, cardiac arrest associated with administration of the chemotherapeutical drug, pericarditis associated with administration of the chemotherapeutical drug, leukemia associated with administration of the chemotherapeutical drug, marrow proliferative disease associated with administration of the chemotherapeutical drug, marrow suppression associated with administration of the chemotherapeutical drug, anemia associated with administration of the chemotherapeutical drug, leukopenia associated with administration of the chemotherapeutical drug, granulocytopenia associated with administration of the chemotherapeutical drug, thrombocytopenia associated with administration of the chemotherapeutical drug, distal paresthesia associated with administration of the chemotherapeutical drug, muscle contraction associated with administration of the chemotherapeutical drug, and/or limb stiffness associated with administration of the chemotherapeutical drug.

In some embodiments, the disease or disorder associated with administration of the chemotherapeutical drug comprises hand-foot syndrome associated with administration of the chemotherapeutical drug and/or paronychia associated with administration of the chemotherapeutical drug.

In some embodiments, the severity of the disease or disorder associated with administration of the chemotherapeutical drug is Grade 1 or above, Grade 2 or above, Grade 3 or above, Grade 4 or above, and/or Grade 5 in accordance with NCI-CTCAE V5.0.

In some embodiments of the use, the drug is prepared to be applicable for topical administration. In some embodiments of the use, the drug is prepared to be applicable for oral administration. In some embodiments of the use, the drug is prepared to be applicable for topical administration with limited area.

In some embodiments, the concentration of the thymidine derivative in the drug is about 0.0001% (w/w) to about 50% (w/w). In some embodiments, the concentration of the thymidine derivative in the drug is about 0.1% (w/w) to about 5% (w/w). In some embodiments, the concentration of the thymidine derivative in the drug is about 0.5% (w/w) to about 3% (w/w).

In some embodiments, the administration dose of the thymidine derivative is about 0.5 µM to about 1000 µM. In some embodiments, the administration dose of the thymidine derivative is about 1 µM to about 500 µM. In some embodiments, the administration dose of the thymidine derivative is about 20 µM to about 300 µM. In some embodiments, the administration dose of the thymidine derivative is about 20 µM to about 200 µM. In some embodiments, the administration dose of the thymidine derivative is about 30 µM to about 200 µM.

In some embodiments, the administration dose of the uridine derivative is about 100 µM to about 500 µM. In some embodiments, the administration dose of the uridine derivative is about 0.5 µM to about 50 µM. In some embodiments, the administration dose of the uridine derivative is about 1 µM to about 50 µM. In some embodiments, the administration dose of the uridine derivative is about 1 µM to about 30 µM. In some embodiments, the administration dose of the uridine derivative is about 10 µM to about 50 µM.

In some embodiments, the administration dose of the thymidine derivative and uridine derivative is about 0.5 µM to about 1000 µM. In some embodiments, the administration dose of the thymidine derivative and uridine derivative is about 1 µM to about 500 µM. In some embodiments, the administration dose of the thymidine derivative and uridine derivative is about 0.5 µM to about 25 µM. In some embodiments, the administration dose of the thymidine derivative and uridine derivative is about 0.8 µM to about 25 µM.

In some embodiments, the administration dose of the thymidine derivative is about 15 mpk to about 300 mpk. In some embodiments, the administration dose of the thymidine derivative is about 10 mpk to about 500 mpk.

In some embodiments, the administration dose of the thymidine derivative is about 10 mpk to about 100 mpk. In some embodiments, the administration dose of the thymidine derivative is about 50 mpk to about 100 mpk.

In some embodiments, the administration dose of the thymidine derivative and uridine derivative is about 50 mpk to about 1000 mpk. In some embodiments, the administration dose of the thymidine derivative and uridine derivative is about 300 mpk to about 1000 mpk.

In some embodiments, the drug further comprises one or more active ingredients.

In some embodiments, the drug does not substantially affect the therapeutical effect of the chemotherapeutical drug.

In some embodiments, the subject in the use comprises a cancer patient.

In some embodiments, the subject has been, is being and/or will be administered with the chemotherapeutical drug.

In some embodiments, the subject has or is susceptible to disease or disorder associated with administration of the chemotherapeutical drug.

In some embodiments, the severity of the disease or disorder in the use increases after administration of the chemotherapeutical drug.

In some embodiments, before administration of the chemotherapeutical drug, the subject did not have the disease or disorder.

In another aspect, the present application provides a pharmaceutical combination or a kit, comprising: 1) a chemotherapeutical drug; and 2) a combination of thymidine derivative and uridine derivative.

In some embodiments of the pharmaceutical combination or the kit, the chemotherapeutical drug and the combination of thymidine derivative and uridine derivative are not mixed with each other.

In some embodiments, the chemotherapeutical drug and the combination of thymidine derivative and uridine derivative are each independently present in separate containers.

In some embodiments of the pharmaceutical combination or the kit, the combination of thymidine derivative and uridine derivative in 2) can prevent or treat a disease or disorder associated with administration of the chemotherapeutical drug in 1).

In some embodiments of the pharmaceutical combination or the kit, the combination of thymidine derivative and uridine derivative in 2) does not substantially affect the therapeutical effect of the chemotherapeutical drug in 1).

In some embodiments of the pharmaceutical combination or the kit, the combination of thymidine derivative and uridine derivative in 2) is administered before, simultaneously with, or after the administration of the chemotherapeutical drug in 1).

In another aspect, the present application provides a method comprising administering to a subject a combination of thymidine derivative and uridine derivative, wherein the subject has been, is being and/or will be administered with a chemotherapeutical drug and has or is susceptible to disease or disorder associated with administration of the chemotherapeutical drug.

In another aspect, the present application provides a method of preventing or treating a disease or disorder, comprising administering a combination of thymidine derivative and uridine derivative to a subject who is susceptible to the disease or disorder, wherein the disease or disorder is disease or disorder associated with administration of the chemotherapeutical drug.

In some embodiments of the method, the subject has been, is being and/or will be administered with the chemotherapeutical drug.

Persons skilled in the art can easily perceive other aspects and advantages of the present application from the detailed description below. The following detailed description only shows and describes exemplary embodiments of the present application. As persons skilled in the art will recognize, the content of the present application enables those skilled in the art to modify the disclosed embodiments without departing from the spirit and scope of the invention involved in the present application. Accordingly, the drawings and the description in the specification of the present application is only exemplary, and not restrictive.

### BRIEF DESCRIPTION OF THE DRAWINGS

Specific features of the invention involved in the present application are as shown in the appended claims. By reference to the exemplary embodiments detailed hereinafter and the accompanying drawings, the features and advantages of the invention involved in the present application can be better understood. The drawings are briefly described as follows:

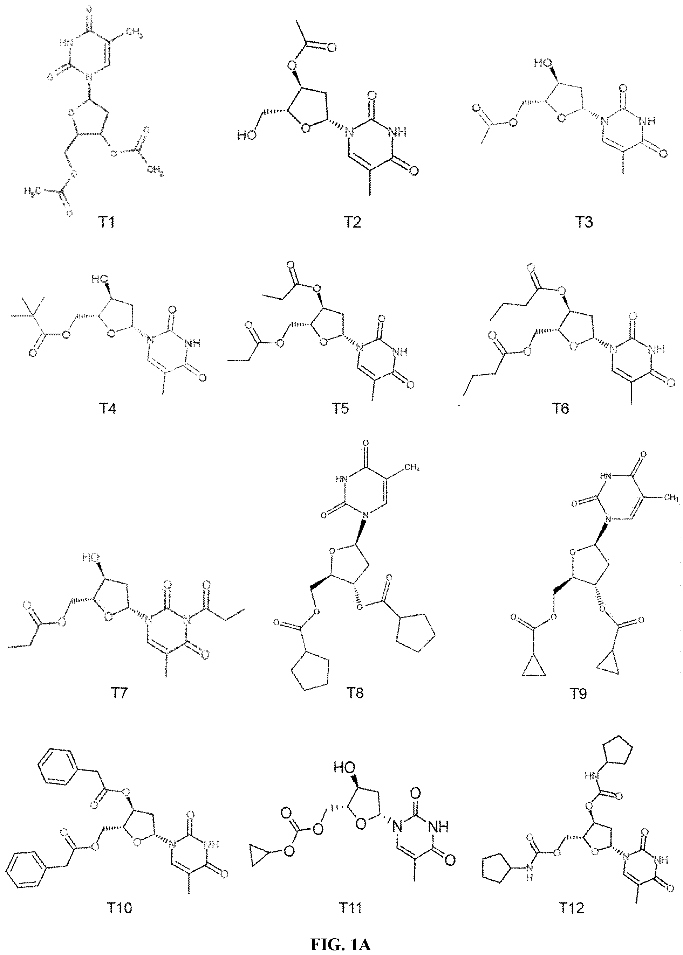

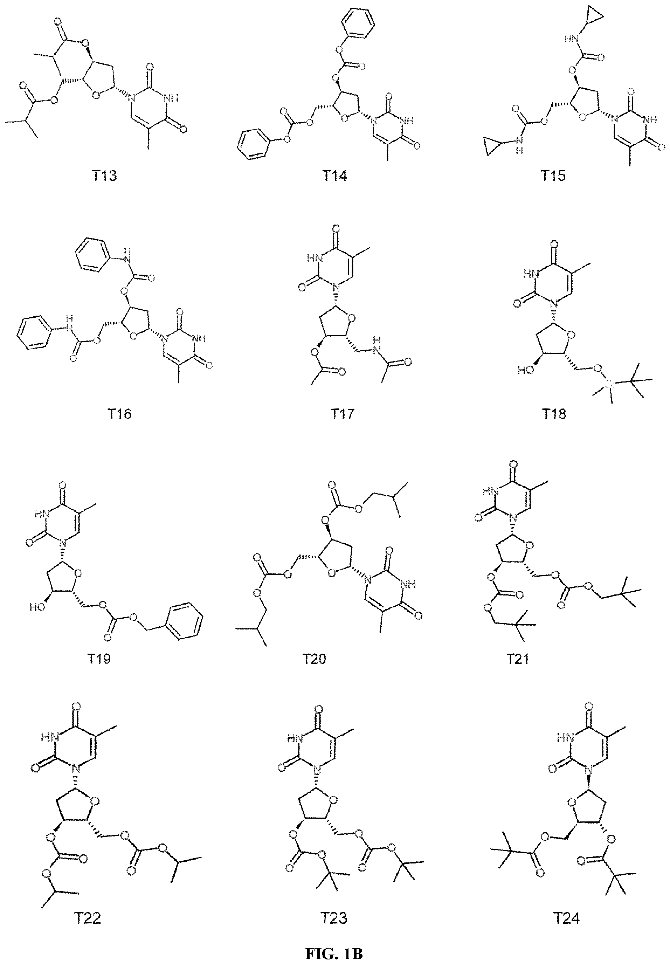

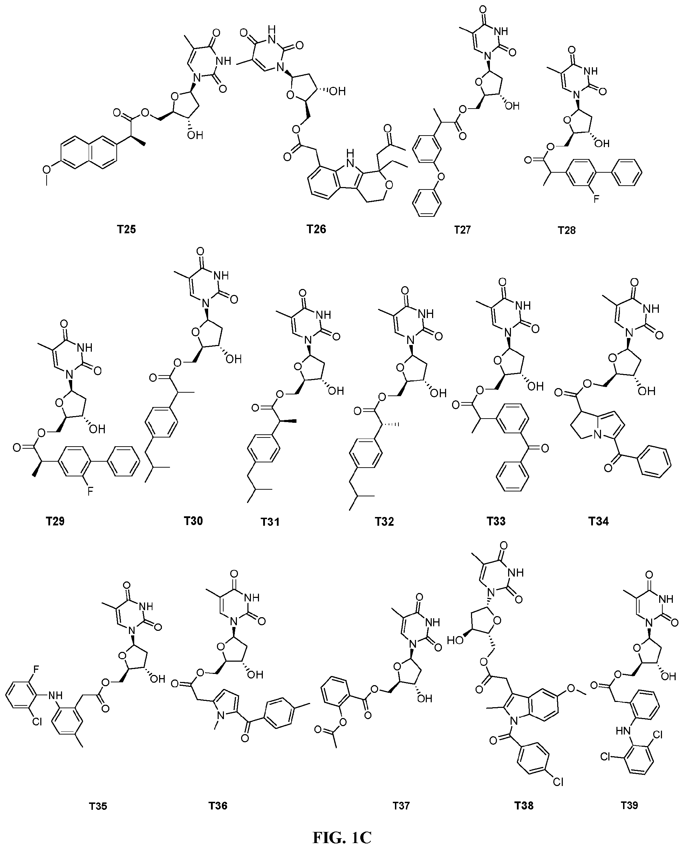

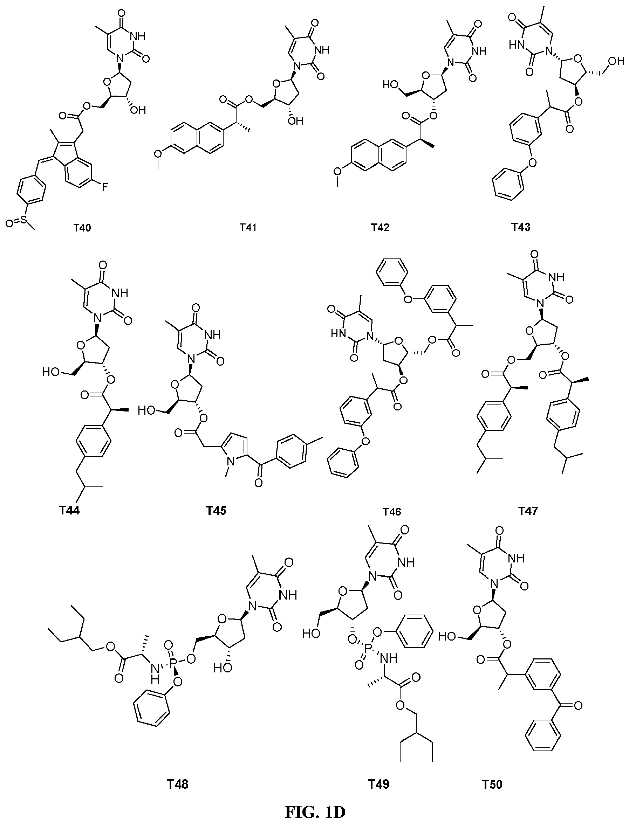

FIGs. 1A to 1D showed the thymidine derivative compounds of the present application and their numbers.

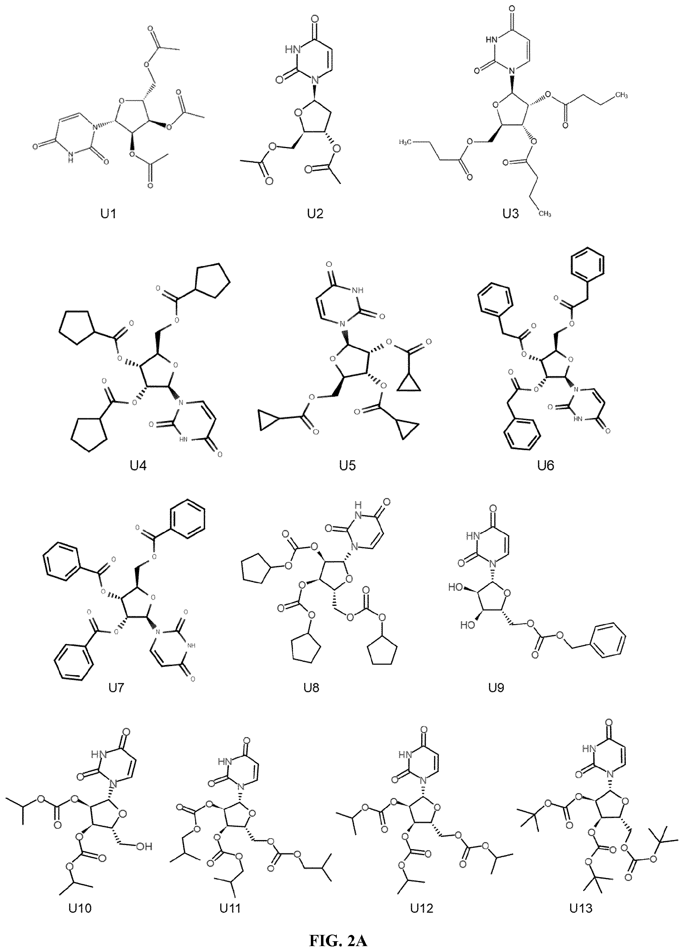

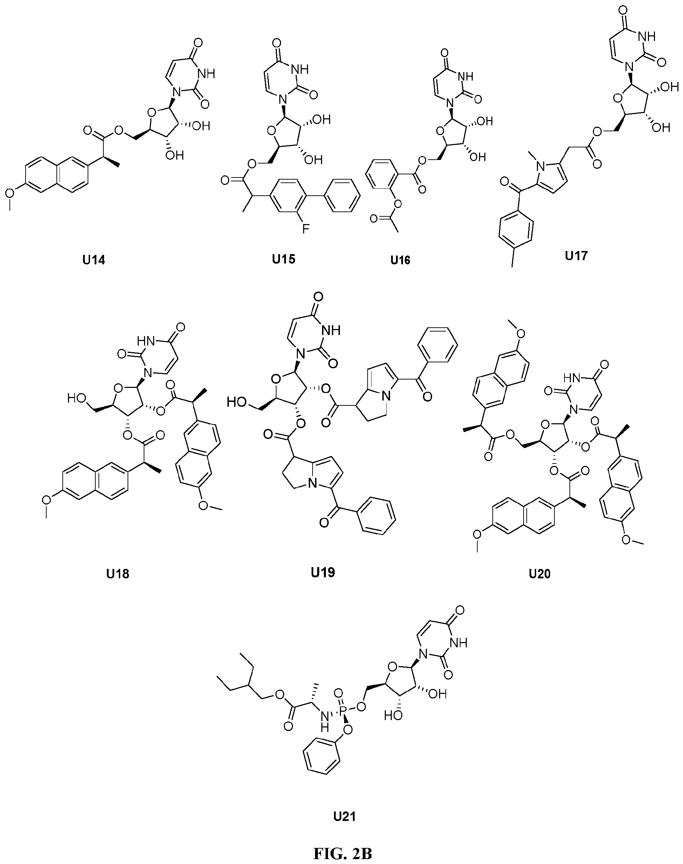

FIGs. 2A to 2B showed the uridine derivative compounds of the present application and their numbers.
FIGs. 3 to 10 showed that the exemplary thymidine derivative and the combination of thymidine derivative and uridine derivative could relieve the toxicity of fluorine-containing drugs in Hacat cells; at the same time, the results showed that the relieving effect of the derivative series of compounds was better than that of uridine.
FIG. 11 showed a model of hand-foot syndrome in rats caused by capecitabine. The results showed that the hand-foot syndrome symptoms are obvious.
FIG. 12 showed the exemplary results of the thymidine derivative and the combination of thymidine derivative and uridine derivative in the prevention and/or treatment of the hand-foot syndrome caused by capecitabine in rats. The results showed that the symptoms after application can be significantly relieved as compared with the model group.
FIG. 13 showed a model of hand-foot syndrome caused by capecitabine in mice.
FIG. 14 showed the exemplary results of the thymidine derivative and the combination of thymidine derivative and uridine derivative in the prevention and/or treatment of the hand-foot syndrome caused by capecitabine in mice. The results showed that the symptoms after application could be significantly relieved as compared with the model group.
FIGs. 15 to 20 showed that the exemplary NSAID-containing thymidine derivative could relieve the toxicity of fluorine-containing drugs in Hacat cells; at the same time, the results showed that the relieving effect of the uridine derivative compound was better than that of thymidine.
FIG. 21 showed that an exemplary combination of the NSAID-containing thymidine derivative and the NSAID-containing uridine derivative could relieve the toxicity of fluorine-containing drugs in Hacat cells.
FIG. 22 showed that the exemplary NSAID-containing thymidine derivative could relieve the inflammatory response in rats with the hand-foot syndrome after administration of a chemotherapeutical drug.
FIG. 23 showed that the exemplary NSAID-containing thymidine derivative and the combination of the NSAID-containing thymidine derivative and the NSAID-containing uridine derivative could relieve the pain in rats with the hand-foot syndrome after administration of a chemotherapeutical drug.
FIG. 24 showed that the exemplary NSAID-containing thymidine derivative and the combination of the NSAID-containing thymidine derivative and the NSAID-containing uridine derivative could relieve the diarrhea after administration of a chemotherapeutical drug.

### DETAILED DESCRIPTION

Hereinafter the embodiments of the invention of the present application are described by specific examples. Persons skilled in the art can easily understand other advantages and effects of the invention of the present application from the disclosure of the present description.

### DEFINITIONS

In the present application, the term "chemotherapeutical drug" refers generally to a drug that can act on different stages of growth and reproduction of tumor cells and inhibit or kill the tumor cells. In some embodiments, the chemotherapeutical drug may comprise a pyrimidine nucleoside analog or a prodrug thereof. For example, the chemotherapeutical drug may comprise one or more selected from the group consisting of capecitabine, cytarabine, docetaxel, adriamycin, fluorouracil (5-FU), floxuridine, tegafur, idarubicin, paclitaxel, epirubicin, acelarin (NUC-1031), doxorubicin, folinic acid, cis-platinum, paclitaxel, cyclophosphamide and vincristine. For example, the chemotherapeutical drug can directly act on DNA and prevent the regeneration of cancer cells. For example, the chemotherapeutical drug can interfere with the synthesis of DNA and RNA. For example, the chemotherapeutical drug can block the proliferation of cancer cells by inhibiting enzymes or mitosis.

In the present application, the term "cancer" refers generally to neoplasms formed by the proliferation of local tissue cells under the action of various carcinogens. These neoplasms are also called neoformations because they are mostly presented as space-occupying lumps. The cancer may be selected from the group consisting of lung cancer, pancreatic cancer, skin cancer, bladder cancer, colon cancer, uterine cancer, breast cancer, bowel cancer, prostate cancer, cervical cancer, ovarian cancer, esophagus cancer, head and neck cancer, stomach cancer and throat cancer. For example, the cancer may be colon cancer.

In the present application, the term "disease or disorder associated with administration of the chemotherapeutical drug" refers generally to a disease or disorder that is relevant to the administration of the chemotherapeutical drug to a subject. For example, the disease or disorder may be caused by administering the chemotherapeutical drug to a subject. The disease or disorder may occur or aggravate after the administration of the chemotherapeutical drug. For example, the disease or disorder associated with administration of the chemotherapeutical drug may be hand-foot syndrome. For example, the disease or disorder associated with administration of the chemotherapeutical drug may be diarrhea.

In the present application, the term "skin tissue disease or disorder" refers generally to a pathological change of morphology, structure and/or function of skin (including hair and nails). For example, the skin tissue disease or disorder may comprise, but is not limited to, rash, hand-foot syndrome, pruritus, erythema, dry skin, alopecia, paronychia, pigmentation disorder, etc.

In the present application, the term "rash" refers generally to a skin change that can affect the color, appearance or texture of skin. Rash may be restricted in a part of the body or affect the entire skin. Rash may also comprise urticaria.

In the present application, the term "hand-foot syndrome" is also called Palmar Plantar Erythrodysesthesia (PPE) or Hand-foot skin reaction (HFSR), which was first described in 1984 by Jacob Lokich and Cery Moor of Harvard Medical School's New England Dekennes Hospital. Typical clinical manifestations are progressive, and clinical manifestations are mainly finger (toe) fever, pain, erythematous swelling, and severe cases develop to desquamation, ulcers and severe pain, etc. Pathological manifestations of HFS comprise vacuolar degeneration of basal keratinocytes, peripheral lymphocyte infiltration of the skin, keratinocyte apoptosis, and skin edema, etc. For example, HFS can comprise insensitivity of palms and vola, or acroerythema caused by chemotherapeutics, etc. In the present application, a cancer patient may develop corresponding symptoms during chemotherapeutics.

In the present application, the term "thymidine derivative" refers generally to a product derived by replacing hydrogen atom(s) of the thymidine with other atom(s) or atomic group(s). In some embodiments, at least one hydroxyl hydrogen of a deoxyribose in the thymidine derivative may be substituted. In some embodiments, the thymidine derivative can prevent and/or treat a subject who has been, is being and/or will be administered with a chemotherapeutical drug, and has or is susceptible to the disease or disorder associated with administration of the chemotherapeutical drug.

In the present application, the term "alkyl" refers generally to a linear or branched, saturated hydrocarbon substituent with 1-20 carbon atoms (e.g., a substituent obtained by removing a hydrogen from hydrocarbon(s)); e.g., 1-12 carbon atoms; in other embodiments, the number of carbon atoms is 1-10; in other embodiments, 1-6 carbon atoms, in other embodiments, 1-4 carbon atoms (such as, 1, 2, 3 or more carbon atoms). Examples of the substituents comprise, e.g., methyl, ethyl, propyl (including *n*-propyl and isopropyl), butyl (including *n*-butyl, isobutyl, *sec*-butyl and *tert*-butyl), pentyl, isopentyl, hexyl, etc. In some cases, the number of carbon atoms in hydrocarbon substituents (i.e., alkyl, alkenyl, cycloalkyl, aryl, etc.) is represented with the prefix "Cₐ-C_{b}", wherein a is the lower limit, b is the upper limit of the number of carbon atoms in the substituent. Thus, for example, "C₁-C₆ alkyl" refers to an alkyl substituent with 1-6 carbon atoms.

In the present application, the term "cycloalkyl" refers generally to a carbocyclyl substituent obtained by removing hydrogen from a saturated carboncycle molecule and having 3-14 carbon atoms. In some embodiments, a cycloalkyl substituent has 3-10 carbon atoms. The cycloalkyl can be monocyclic, and generally comprises 4-7 ring atoms. The cycloalkyl comprises cyclopropyl, cyclobutyl, cyclopentyl and cyclohexyl. The cycloalkyl can also be 2-3 rings fused to each other, e.g., bicyclo [4.2.0] octane and decalin, and can also be called "bicycloalkyl".

In the present application, the term "cycloalkyl" further comprises a substituent fused to C₆-C₁₀ aryl ring or 5-10-membered heteroaryl ring, wherein a group having this fused cycloalkyl as a substituent is attached to a carbon atom of cycloalkyl. When this fused cycloalkyl is substituted with one or more substituents, unless otherwise indicated, the one or more substituents are each bonded to a carbon atom of the cycloalkyl. The fused C₆-C₁₀ aryl ring or 5-10-membered heteroaryl ring can optionally be further substituted.

In the present application, the term "hydrogen" refers generally to a hydrogen substituent, and may be described as -H.

In the present application, the term "oxygen" refers generally to an oxygen substituent and may be described as -O-.

In the present application, the term "hydroxyl" refers generally to -OH. When used in combination with another term, the prefix "hydroxyl" generally means that the substituent involved by the prefix is substituted with one or more hydroxyl substituents. Compounds having carbon attached to one or more hydroxyl substituents comprise e.g., alcohols, enols, and phenols.

In the present application, the term "substituent", "radical" and "group" can be used interchangeably.

If a substituent is described as "optional substituents", the substituent may be: (1) unsubstituted, or (2) substituted. If a carbon of a substituent is described as optionally substituted with one or more substituents, one or more hydrogen on the carbon (if present) may be replaced with independently selected optional substituents, alone and/or together. If nitrogen of a substituent is described as optionally substituted with one or more substituents, one or more hydrogen on the nitrogen (if present) may be each replaced with independently selected optional substituents. An exemplary substituent can be described as -NR'R", wherein R' and R", together with the nitrogen atom to which they are attached, can form a heterocyclyl ring comprising one or two heteroatoms independently selected from the group consisting of oxygen, nitrogen, and sulfur, wherein the heterocycloalkyl moiety can be optionally substituted. The heterocyclyl ring formed by R' and R", together with the nitrogen atom to which they are attached can be partially or completely saturated, or aromatic. In some embodiments, the heterocyclyl ring consists of 4 to 10 atoms.

If a substituent is described as "independently selected from" a group consisting of groups, each substituent is selected independent of other substituents. Thus, each substituent may be the same as or different from other substituents.

In the present application, the terms "Formula (I)", "Formula (II)", "Formula (III)", "Formula (IV)" can be called "the compound of Formula (I)", "the compound of Formula (II)", "the compound of Formula (III)" or "the compound of Formula (IV)". These terms are also defined to comprise all forms of the compound of Formula (I), the compound of Formula (II), the compound of Formula (III) or the compound of Formula (IV), including hydrate, solvate, isomer, crystalline and non-crystalline forms, isomorphism, polymorphism, and metabolite. For example, the compound of Formula (I), the compound of Formula (II), the compound of Formula (III) or the compound of Formula (IV) or a pharmaceutically acceptable salt thereof, the compound of Formula (I), the compound of Formula (II), the compound of Formula (III) or the compound of Formula (IV) or a pharmaceutically acceptable salt thereof, may be present in unsolvated or solvated forms. When the binding power of solvent or water is relatively strong, the coordination compound has a definite stoichiometry that is not affected by humidity. However, if the binding power of solvent or water is relatively weak, e.g., in a channel solvate and hygroscopic compound, the content of water/solvent will depend on the humidity and drying conditions. In this case, non-stoichiometry is normal.

"The compound of Formula (I)", "the compound of Formula (II)", "the compound of Formula (III)" or "the compound of Formula (IV)" may comprise chiral carbon atom(s). In the present application, the carbon-carbon bond in the compound of Formula (I), the compound of Formula (II), the compound of Formula (III) or the compound of Formula (IV) may be represented by solid lines, solid wedges, or dot wedges. Depicting the bond to a chiral carbon atom with solid line means that all the potential stereoisomers (e.g., specific enantiomers, racemates, etc.) on the carbon atoms are comprised. The compounds of the present application may comprise one or more chiral carbon atoms. In these compounds, depicting the bond to a chiral carbon atom with solid lines is intended to indicate that all the potential stereoisomers should be encompassed, e.g., unless otherwise indicated, the compound of Formula (I), the compound of Formula (II), the compound of Formula (III) or the compound of Formula (IV) may be present as enantiomers, diastereomers, or racemates and mixtures. Depicting the bond to one or more chiral carbon atoms in the compound of Formula (I), the compound of Formula (II), the compound of Formula (III) or the compound of Formula (IV) with solid lines and depicting the bond to another chiral carbon atom in the same compound with solid or dot wedges indicate the presence of a mixture of diastereomers.

The compound of the present application can be present as inclusion compound or other coordination compounds. The present invention encompasses complexes, e.g., inclusion compounds, drug-host inclusion complex, wherein in contrast to the aforesaid solvates, drug and host are present in stoichiometric or non-stoichiometric amount. It further comprises a coordination compound of the compound of Formula (I), the compound of Formula (II), the compound of Formula (III) or the compound of Formula (IV) comprising two or more organic and/or inorganic components that can be stoichiometric or non-stoichiometric. The resultant complex can be ionized, partially ionized, or unionized.

The stereoisomer of the compound of Formula (I), the compound of Formula (II), the compound of Formula (III) or the compound of Formula (IV) comprises cis- and trans-isomers, optical isomers, e.g., R and S enantiomers, diastereomers, geometrical isomers, rotamers, conformational isomers and tautomers. The compound of Formula (I), the compound of Formula (II), the compound of Formula (III) or the compound of Formula (IV) comprises the compounds exhibiting one or more of the aforesaid types of isomerism, and mixtures thereof (e.g., racemates and diastereomer pair). It further comprises acid or base addition salts in which the counterion has optical activity, e.g., D-lactate or L-lysine, or racemates, e.g., DL-tartrate or DL-arginine.

When any racemate crystallizes, there may be two different types of crystals. The first type is the racemic compounds (true racemates) as described above, wherein a homogeneous form of crystal is produced and comprises two enantiomeric isomers in equimolar amounts. The second type is a racemic mixture or agglomerate, wherein two forms of crystals are produced in equimolar amounts, each comprising a single enantiomer.

The compound of Formula (I), the compound of Formula (II), the compound of Formula (III) or the compound of Formula (IV) can exhibit tautomerism and structural isomerism. For example, the compound of Formula (I) or Formula (I) may be present in several tautomeric forms, comprising enol and imine forms, and ketones and enamine forms; and geometric isomers and their mixtures. All these tautomeric forms are encompassed within the scope of the compound of Formula (I), the compound of Formula (II), the compound of Formula (III) or the compound of Formula (IV). Tautomeric isomers are present in solution as a mixture of tautomeric isomers. In solid form, one tautomer isomer generally dominates. Even if one tautomer can be described, the present invention also comprises all the tautomers of the compound of Formula (I), the compound of Formula (II), the compound of Formula (III) or the compound of Formula (IV).

The present invention further comprises isotope-labeled compounds that are the same as the compound of Formula (I), the compound of Formula (II), the compound of Formula (III) or the compound of Formula (IV) except that one or more atoms thereof are replaced with atoms having different atomic mass or mass number found in nature. Isotopes which may be incorporated into the compound of Formula (I), the compound of Formula (II), the compound of Formula (III) or the compound of Formula (IV) comprise isotopes of hydrogen, carbon, nitrogen, oxygen, phosphorus, fluorine and chlorine, e.g., but are not limited to: ²H, ³H, ¹³C, ¹⁴C, ¹⁵N, ¹⁸O, ¹⁷O, ³¹P, ³²P, ³⁵S, ¹⁸F, and ³⁶Cl. Some isotope-labeled compounds of Formula (I), Formula (II), Formula (III), or Formula (IV), e.g., into which a radioisotope (e.g., ³H and ¹⁴C) is incorporated, can be used for measuring the tissue dispersion of drug and substrate due to its easy preparation and measurability. Heavier isotopes, such as ²H, can provide certain therapeutic advantages due to its greater metabolic stability, e.g., longer half-life in the body or lower dose requirements. The isotope-labeled compound of Formula (I), Formula (II), Formula (III), or Formula (IV) generally can be prepared with isotope-labeled reagents instead of non-isotope-labeled reagents.

The compound of the present application can be used as salts derived from inorganic or organic acids. Some compounds have advantages, such as, enhanced drug stability at different temperatures and humidities, or desired solubilities in water/oil due to the physical property of one or more salts. In some cases, the salts of the compound can also be adjuvants for use in the separation, purification, and/or resolution of the compound.

In the present application, the term "prodrug" refers generally to a precursor of a given compound which produces the compound in vivo via a chemical or physiological process (e.g., solvolysis or enzymatic decomposition) or under physiological conditions. "Prodrug" refers generally to non-toxic, biologically tolerable prodrugs that are biologically suitable for administration to a subject. Exemplary methods of selecting and preparing suitable prodrug derivatives are described in, e.g., "Design of Prodrugs", H. Bundgaard (Edited), Elsevier, 1985.

In the present application, the term "non-steroidal anti-inflammatory drug (NSAID)" refers generally to a class of drugs with antipyretic and analgesic effects. Most NSAIDs can inhibit the activity of cyclooxygenase (COX, e.g., COX-1 and COX-2), thus further reducing the synthesis of prostaglandin and thromboxane. non-steroidal means non-glucocorticoid. In the present application, the thymidine derivative and/or uridine derivative can comprise a NSAID moiety, and the NSAID can be a common NSAID, e.g., a COX-1 and/or COX-2 inhibitor. The NSAID moiety can be connected to thymidine or uridine via an ester bond. The NSAID can comprise, but not limited to, pyrazolidines, salicylic acids, acetic acid derivatives, oxicams, propanoic acid derivatives, profens and/or fenamic acids. For example, the NSAID may comprise aminoantipyrine, azapropazone, clofezone, kebuzone, feprazone, analginum, mofebutazone, nifenazone, oxyphenbutazone, phenylbutazone, antipyrine, isoantipyrine, sulfinpyrazone, suxibuzone, aspirin (acetylsalicylic acid), aloxiprin, benorilate, carbasalate calcium, diflunisal, diacetylsalicylic acid, ethenzamide, guacetisal, magnesium salicylate, methyl salicylate, salsalate, salicin, salicylamide, sodium salicylate, aceclofenac, acemetacin, alclofenac, amfenac, bendazac, bromfenac, bumadizone, bufexamac, diclofenac sodium, diphenylpyraline, etodolac, felbinac, fentiazac, indometacin, indometacin farnesil, ketorolac, lonazolac, oxametacin, proglumetacine, sulindac, tolmetin, zomepirac, ampiroxicam, droxicam, isoxicam, lornoxicam, meloxicam, piroxicam, tenoxicam, alminoprofen, benoxaprofen, carprofen, dexibuprofen, ketoprofen, fenbufen, fenoprofen, flunoxaprofen, flurbiprofen, ibuprofen, bromhexine, indoprofen, ketoprofen, loxoprofen, miroprofen, naproxen, oxaprozin, pirprofen, suprofen, dalfampridine, tepoxalin, tiaprofenic acid, vedaprofen, naproxcinod, azapropazone, etofenamate, flufenamic acid, flunixin, meclofenamic acid, mefenamic acid, morniflumate, niflumic acid, tolfenamic acid, parecoxib, celecoxib, cimecoxib, deracoxib, Etoricoxib, Firocoxib, Lumiracoxib, mavacoxib, parecoxib, robenacoxib, rofecoxib, valdecoxib, aminopropionitrile, benzydamine, chondroitin sulfate, diacerein, fluproquazone, glucosamine, glycosaminoglycan, magnesium salicylate, nabumetone, nimesulide, oxaceprol, proquazone, superoxide dismutase (Orgotein) and/or Tenidap.

### DETAILED DESCRIPTION OF THE INVENTION

### Chemotherapeutical Drug

In the present application, the chemotherapeutical drug can treat cancers. For example, the chemotherapeutical drug may comprise a pyrimidine nucleoside analog or a prodrug thereof. Alternatively, for example, the chemotherapeutical drug may comprise drugs that can be metabolized to form fluorouridine nucleotides. The floxuridine nucleotides in cells can cause cytotoxicity by interfering with the normal metabolism of the uridine nucleotides. Alternatively, for example, the chemotherapeutical drug may comprise one or more selected from the group consisting of capecitabine, cytarabine, docetaxel, adriamycin, fluorouracil (5-FU), floxuridine, tegafur, idarubicin, paclitaxel, epirubicin, Acelarin (NUC-1031), doxorubicin, folinic acid, cis-platinum, paclitaxel, cyclophosphamide, vincristine and 5-FU pro-drugs (e.g., tegafur and 5'-deoxyfloxuridine, floxuridine, 2'-deoxyfloxuridine, a pro-drug derivative of floxuridine or a pro-drug derivative of 2'-deoxyfloxuridine, trifluoro-methyl-2'-deoxyuridine, 6-azauridine, 3-deazauridine).

For example, the chemotherapeutical drug may comprise alkylating agents, such as, nitrogen mustard, nitrogen mustard N-oxide hydrochloride, chlorambucil, cyclophosphamide, ifosfamide, thiotepa, isothiocyanate, busulfan, nimustine hydrochloride, mitropium bromide, melphalan, dacarbazine, ranimustine, sodium phopofol phosphate, ethylenetriamine, carmustine, lomustine, streptozotocin, pipobroman, ethoglucid, carboplatin, *cis*-platinum, miriplatin, nedaplatin, tinidamine, omustine, dichloropyridine, fupisitan, prednifixine, pumitepa, Ribomustin hydrochloride, temozolomide, diclofenac, trovafloxacin, zinostatin, simvastatin, penem, cystemustine and bizelesin; antimetabolites, such as, mercaptopurine, 6-mercaptopurine nucleoside, thioinosine, methotrexate, pemetrexed, entectine, cytarabine, oxaliplatin, tisabatin hydrochloride, 5-FU and derivatives thereof (e.g., fluorouracil, tegafur, UFT, dosiholu, carmofur, capecitabine, etc.), aminopterin, nazothioamine, calcium leucovorin, microphyllum, calcium folinate, calcium levofate, cladribine, emitoful, fludarabine, gemcitabine, hydroxylurea, pentostatin, piritrexim, iodouridine, nitoguanone, thiazolylfuran, vimalstat and bendamustine; anti-tumor antibiotics, such as, dactinomycin D, dactinomycin C, mitomycin C, chromomycin A3, bleomycin hydrochloride, bleomycin sulfate, cetiamycin hydrochloride, doxorubicin hydrochloride, mitoxantrone hydrochloride and idarubicin hydrochloride; and/or, etoposide, etoposide phosphate, vinblastine sulfate, vincristine sulfate, teniposide, paclitaxel, docetaxel and vinorelbine, and other plant-derived cytotoxic anticancer drugs.

For example, the chemotherapeutical drug may be hormonotherapeutic anticancer agent, that may comprise fusitatine, stilboestrol, chlorinated costen, medroxyprogesterone acetate, megestrol acetate, cyproterone acetate, cyproterone acetate, danazol, allylestrenol, progesterone, mepartricin, raloxifene or meloxifene, levofloxacin, antiestrogen (e.g., tamoxifen citrate, toremifene citrate, etc.), contraceptive, quanliehanwa, testolactone, aminosuccinimide, LH-RH agonists (e.g., goserelin acetate, buserelin, leuprorelin, etc.), droloxifene, epiandrostanol, ethinylestradiol sulfonate, fubenzole hydrochloride, anastrozole, letrozole, exemestane, vorozole, antiandrogen (e.g., flutamide, bicalutamide, nilutamide, etc.), 5α-reductase inhibitor (e.g., finasteride, Epristeride), corticosteroids (e.g., dexamethasone, prednisolone, betamethasone, triamcinolone, etc.) and/or androgen synthesis inhibitor (e.g., abiraterone, etc.).

For example, in some cases, the chemotherapeutical drug may also be immunotherapeutic anticancer agent, that may comprise bubinanie,cretirizine, etofuran, lentinan, ubenmetacin, interferon, interleukin, macrophage colony stimulating factor, granulocyte colony stimulating factor, erythrogenin, lymphotoxin, BCG vaccine, corynebacterium arvum, everolimus, levamisole and/or polysaccharide K, etc.

In the present application, the chemotherapeutical drug may be administered in combination with one or more other therapies. For example, the one or more other therapies may comprise one or more other antitumor therapies. For example, other antitumor therapies may also comprise radiotherapy or surgical therapy.

In the present application, if the chemotherapeutical drug is used in combination with other antitumor therapies, they can be simultaneously administered to the subject, or separately administered at intervals. For example, the other antitumor therapies may be part of a single drug, which is mixed with the chemotherapeutical drug to form a single composition. Alternatively, for example, the other antitumor therapies may be separate agents that are administered separately from the chemotherapeutical drug. In the present application, if the other antitumor therapies and the chemotherapeutical drug form a single composition, the chemotherapeutical drug may be present and/or administered in a dose level of about 1-99% (e.g., about 5-95%, about 1%, about 5%, about 10%, about 20%, about 30%, about 40%, about 50%, about 60%, about 70%, about 80%, about 90%, about 95% or about 99%) based on the total weight.

### Disease or disorder associated with administration of the chemotherapeutical drug

In the present application, the disease or disorder associated with administration of the chemotherapeutical drug may be statistically significantly associated with the administration of the chemotherapeutical drug. In the present application, the disease or disorder associated with administration of the chemotherapeutical drug may be caused by the action of the chemotherapeutical drug. For example, the disease or disorder associated with administration of the chemotherapeutical drug may comprise skin tissue disease or disorder associated with administration of the chemotherapeutical drug, hemopoietic tissue disease or disorder associated with administration of the chemotherapeutical drug, limb disease or disorder associated with administration of the chemotherapeutical drug, cardiac disease or disorder associated with administration of the chemotherapeutical drug, nervous system disease or disorder associated with administration of the chemotherapeutical drug, facial feature disease or disorder associated with administration of the chemotherapeutical drug and/or gastrointestinal disease or disorder associated with administration of the chemotherapeutical drug.

For example, the skin tissue disease or disorder, the limb disease or disorder, the facial feature disease or disorder and/or the gastrointestinal disease or disorder associated with administration of the chemotherapeutical drug may comprise the disease or disorder associated with administration of the chemotherapeutical drug in skin, limbs, facial features and/or gastrointestinal tract. For example, the epithelial tissue disease or disorder associated with administration of the chemotherapeutical drug in the skin, limbs, facial features and/or gastrointestinal tract comprises disease or disorder associated with endothelial cell lesion, and/or disease or disorder associated with epithelial cell lesion, and wherein the endothelial cell lesion and/or epithelial cell lesion are/is associated with administration of the chemotherapeutic drug.

For example, the endothelial cell may comprise a vascular endothelial cell. The lesion of the vascular endothelial cell may comprise endothelial dysfunction. For example, the vascular endothelial cell lesion may comprise degenerative vascular disease (e.g., atherosclerosis, medial arteriosclerosis and arteriolosclerosis (e.g., hyaline degenerative arteriolosclerosis and proliferative arteriolosclerosis)), inflammatory vascular disease (e.g., infective arteritis, syphilitic arteritis, giant cell arteritis, thromboangiitis obliterans and rheumatic arteritis), functional vascular disease (e.g., Raynaud's gangrene, acrocyanosis and erythema acrodynia) and/or congenital vascular disease (e.g., congenital arteriovenous fistula), etc.

For example, the epithelial cell may comprise skin epithelial cell, oral epithelial cell, nasal epithelial cell, gastric epithelial cell and/or intestinal epithelial cell. For example, the epithelial cell lesion may comprise skin epithelial cell lesion (e.g., rash, acne, rosacea, atopic dermatitis, contact dermatitis, seborrheic dermatitis, lupus, scleroderma, pemphigus, pigmentation, black spot, leukoderma, urticaria, tinea corporis, skin itching, alopecia, hair change, erythema, paronychia and schizonychia, dry skin, hypersensitive reaction and psoriasis), oral epithelial cell lesion (e.g., pemphigus, herpes labialis, herpetic stomatitis, granulomatous cheilitis, oral ulcer, pemphigoid, Sjogren's syndrome, Behcet syndrome and oral sarcoidosis, etc.), nasal epithelial cell lesion (epistaxis, nasosinusitis, nasal furuncle and nasal polyp, etc.), gastric epithelial cell lesion (e.g., gastritis, intestinal metaplasia, gastric perforation, gastric fistula, gastric ulcer and gastrointestinal polyp) and/or intestine epithelial cell lesion (e.g., enteritis, Crohn's disease, enterobrosis, intestinal fistula, intestinal ulcer, ulcerative colitis and NSAIDs intestinal disease), etc.

For example, the skin tissue disease or disorder associated with administration of the chemotherapeutical drug may comprise side effects or adverse reactions caused by the administration of the chemotherapeutical drug. For example, the skin tissue disease or disorder associated with administration of the chemotherapeutical drug may comprise rash associated with administration of the chemotherapeutical drug, hand-foot syndrome associated with administration of the chemotherapeutical drug, pruritus associated with administration of the chemotherapeutical drug, erythema associated with administration of the chemotherapeutical drug, dry skin associated with administration of the chemotherapeutical drug, alopecia associated with administration of the chemotherapeutical drug, paronychia associated with administration of the chemotherapeutical drug, pigmentation disorder associated with administration of the chemotherapeutical drug, oral ulcer associated with administration of the chemotherapeutical drug, xerostomia associated with administration of the chemotherapeutical drug, epistaxis associated with administration of the chemotherapeutical drug, nasopharyngitis associated with administration of the chemotherapeutical drug, cheilitis associated with administration of the chemotherapeutical drug, esophagus mucositis associated with administration of the chemotherapeutical drug, gastric mucositis associated with administration of the chemotherapeutical drug, gastric ulcer associated with administration of the chemotherapeutical drug, diarrhea associated with administration of the chemotherapeutical drug, vomiting associated with administration of the chemotherapeutical drug, nausea associated with administration of the chemotherapeutical drug, anorexia associated with administration of the chemotherapeutical drug, constipation associated with administration of the chemotherapeutical drug, and/or abdominal pain associated with administration of the chemotherapeutical drug, distal paresthesia associated with administration of the chemotherapeutical drug, muscle contraction associated with administration of the chemotherapeutical drug, and/or limb stiffness associated with administration of the chemotherapeutical drug.

In the present application, the skin tissue disease or disorder, the facial feature disease or disorder and/or the gastrointestinal disease or disorder associated with administration of the chemotherapeutical drug may comprise the epithelial tissue disease or disorder associated with administration of the chemotherapeutical drug in the skin tissue, facial features and/or gastrointestinal tract.

In the present application, the term "skin tissue disease or disorder" refers generally to the pathologic changes of morphology, structure and/or function of skin (including hair and nails). For example, the skin tissue disease or disorder may comprise, but is not limited to rash, hand-foot syndrome, pruritus, erythema, dry skin, alopecia, paronychia, pigmentation disorder, etc.

In the present application, the term "rash" refers generally to a skin change that may affect the color, appearance or texture of skin. Rash may be restricted in a part of the body, or affect the entire skin. Rash may also comprise urticaria.

In the present application, the term "hand-foot syndrome" is also called Palmar Plantar Erythrodysesthesia (PPE) or Hand-foot skin reaction (HFSR), which was first described in 1984 by Jacob Lokich and Cery Moor of Harvard Medical School's New England Dekennes Hospital. Typical clinical manifestations are progressive, and clinical manifestations are mainly finger (toe) fever, pain, erythematous swelling, and severe cases develop to desquamation, ulcers and severe pain, etc. Pathological manifestations of HFS mainly comprise for example vacuolar degeneration of basal keratinocytes, peripheral lymphocyte infiltration of the skin, keratinocyte apoptosis, and skin edema, etc. For example, HFS may comprise insensitivity of palms and vola, or acroerythema caused by chemotherapy, etc. A cancer patient may develop corresponding symptoms during chemotherapy or molecular targeting therapy (for example, chemotherapeutical drugs).

Currently, the hand-foot syndrome (HFS) may be classified by a variety of methods, in which the National Cancer Institute (NCI) classification criteria are commonly used. This classification criteria divides the hand-foot syndrome into three grades: Grade 1 involves minimal skin changes or dermatitis (e.g., disappearance of fingerprints, pigmentation, erythema, skin peeling, paresthesia, insensitivity, skin numbness, etc.) and unaffected daily activities; Grade 2 involves the same degree of skin changes as Grade 1 with pain, slightly affected daily activities, and intact skin surface; and Grade 3 involves ulcerative dermatitis or skin changes with severe pain, severely affected daily life, obvious tissue destruction (e.g., desquamation, blisters, bleeding, edema, etc.).

In addition, the World Health Organization (WHO) rclassified the HFS as four grades: Grade 1 involves insensitivity of hands and feet, paresthesia or tingling sensation; Grade 2 involves discomfort in walking and/ or in holding objects, painless swelling or erythema; Grade 3 involves painful erythema, edema of palms and soles, erythema around nails and swelling; and Grade 4 involves peeling, ulceration, blistering and severe pain.

In the present application, the term "gastrointestinal disease or disorder" refers generally to a pathological change of morphology, structure and/or function of gastric or intestinal tissue (e.g., the digestive tract tissue from the gastric pylorus to the anus). For example, the gastrointestinal disease or disorder may comprise, but is not limited to diarrhea, vomiting, nausea, anorexia, constipation and/or abdominal pain, etc.

For example, the hemopoietic system disease or disorder associated with administration of the chemotherapeutical drug may comprise marrow disease or disorder associated with administration of the chemotherapeutical drug and blood disease or disorder associated with administration of the chemotherapeutical drug. For example, the hemopoietic tissue disease and disorder associated with administration of the chemotherapeutical drug may comprise abnormal blood cell proliferative disease or disorder associated with administration of the chemotherapeutical drug. For example, the hemopoietic system disease associated with administration of the chemotherapeutical drug may comprise leukemia associated with administration of the chemotherapeutical drug, marrow proliferative disease associated with administration of the chemotherapeutical drug, marrow suppression associated with administration of the chemotherapeutical drug, anemia associated with administration of the chemotherapeutical drug, leukopenia associated with administration of the chemotherapeutical drug, and/or thrombocytopenia associated with administration of the chemotherapeutical drug.

In the present application, the hemopoietic system disease associated with administration of the chemotherapeutical drug may be thrombocytopenia associated with administration of the chemotherapeutical drug. In the present application, the term "thrombocytopenia" generally means that an antitumor chemotherapeutical drug produces an inhibition effect on megakaryocytes, so that the blood platelet count in the peripheral blood is less than 100 × 10⁹/L. By reference to Common Adverse Reaction Terminology Standards Version 5.0 (November, 2017), thrombocytopenia is generally divided to four grades. Of those, 7.5 × 10¹⁰/L to the lower limit of normal value corresponds to Grade 1, 5 × 10¹⁰/L to 7.5 × 10¹⁰/L corresponds to Grade 2, 2.5 × 10¹⁰/L to 5 × 10¹⁰/L corresponds to Grade 3, and less than 2.5 × 10¹⁰/L corresponds to Grade 4.

In the present application, the term "marrow inhibition" refers generally to a phenomenon that a chemotherapeutical drug can kill many normal marrow cells while killing many tumor cells. In some embodiments, some tumor patients develop gradually increasing marrow inhibition with the increase of the cumulative amount of chemotherapeutical drug in the body. For example, the marrow inhibition can comprise, but is not limited to, leukopenia.

In the present application, the term "marrow proliferative disease" refers generally to a group of relevant diseases caused by abnormal hemopoietic cells growing in the marrow. In some cases, these cells may become cancerous, and become a type of leukemia. With the accumulation of chemotherapeutical drugs, the incidence and severity of anemia will increase and worsen.

Referring to the anemia grade criteria of the National Cancer Institute (NCI) and the World Health Organization (WHO), in combination with China's national conditions, anemia associated with tumor chemotherapy is rated and evaluated for the severity thereof (the lower limit of normal value: 120 g/L for male; and 110 g/ L for female). See Table 1.

**Table 1 Grades of Severity of Tumor Anemia (g/L)**

| Hemoglobin | Chinese Criteria | NCI Criteria | WHO Criteria |
|---|---|---|---|
| Grade 0 (Normal) | > the lower limit of normal value | ≥ the lower limit of the normal value | ≥110 |
| Grade 1 (Mild) | 90 to the lower limit of the normal value | 100 to the lower limit of the normal value | 95-110 |
| Grade 2 (Moderate) | 60-90 | 80-100 | 80-95 |
| Grade 3 (Severe) | 30-60 | <80 | 65-80 |
| Grade 4 (Extremely Severe) | <30 | Life-threatening | <65 |

In the present application, the term "anemia" refers generally to a common clinic symptom that the erythrocyte volume in the peripheral blood decreases below the lower limit of the normal range. In some embodiments, the chemotherapeutical drug may directly affect the marrow hematopoiesis by blocking the synthesis of erythroid precursor cells.

For example, the cardiac disease or disorder associated with administration of the chemotherapeutical drug may comprise the disease or disorder of heart associated with administration of the chemotherapeutical drug. For example, the cardiac disease or disorder associated with administration of the chemotherapeutical drug may comprise nonspecific chest pain associated with administration of the chemotherapeutical drug, angina pectoris associated with administration of the chemotherapeutical drug, palpitation associated with administration of the chemotherapeutical drug, dyspnea associated with administration of the chemotherapeutical drug, diffuse pleuritic chest pain associated with administration of the chemotherapeutical drug associated with administration of the chemotherapeutical drug, supraventricular arrhythmia associated with administration of the chemotherapeutical drug, myocardial infarction associated with administration of the chemotherapeutical drug, bradycardia associated with administration of the chemotherapeutical drug, arrhythmia associated with administration of the chemotherapeutical drug, ventricular fibrillation associated with administration of the chemotherapeutical drug, ventricular tachycardia associated with administration of the chemotherapeutical drug, myocarditis associated with administration of the chemotherapeutical drug, heart failure associated with administration of the chemotherapeutical drug, cardiac arrest associated with administration of the chemotherapeutical drug and/or pericarditis associated with administration of the chemotherapeutical drug.

In the present application, the chemotherapeutical drug may be used to treat tumors. For example, the site of the disease or disorder is different from that of the tumor.

In the present application, the severity of the disease or disorder associated with administration of the chemotherapeutical drug may be Grade 1 or above, Grade 2 or above, Grade 3 or above, Grade 4 or above, and/or Grade 5 or above in accordance with NCI-CTCAE V5.0.

The disease or disorder may occur or aggravate after the administration of the chemotherapeutical drug.

### Thymidine derivative and uridine derivative

The present application provides use of a thymidine derivative in preparation of a drug for preventing or treating a disease or disorder associated with administration of a chemotherapeutical drug in a subject, wherein the thymidine may be acetyl derivatives of thymidine (e.g., monoacetyl derivatives of thymidine, diacetyl derivatives of thymidine or triacetyl derivatives of thymidine).

In the present application, the thymidine derivative can comprise a structure of Formula (I): 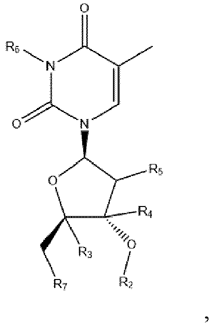 wherein R₂, R₃, R₄, R₅ and/or R₆ may be hydrogen.

In the present application, the thymidine derivatives may be any one or more selected from T1 to T24.

The present application provides use of a thymidine derivative containing a NSAID moiety in preparation of a drug for preventing or treating a disease or disorder associated with administration of the chemotherapeutical drug (e.g., hand-foot syndrome, or diarrhea) in a subject.

In the present application, the thymidine derivative comprises a structure of Formula (II): 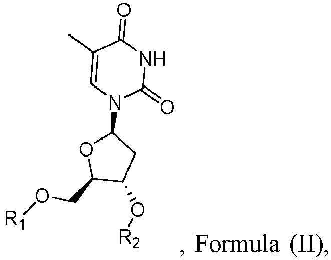 wherein at least one of R₁ and R₂ comprises a non-steroidal anti-inflammatory drug (NSAID) moiety. For example, R₁ in Formula (II) comprises a NSAID moiety. For example, R₂ in Formula (II) comprises a NSAID moiety. When one of R₁ and R₂ comprises a non-steroidal anti-inflammatory drug (NSAID) moiety, the other may be hydrogen.

For example, both R₁ and R₂ in Formula (II) comprise NSAID moieties and can comprise the same or different NSAID moieties.

In the present application, the NSAID moiety may comprise, but is not limited to, pyrazolidines, salicylic acids, acetic acid derivatives, oxicams, propanoic acid derivatives, profens and/or fenamic acids. In the present application, the NSAID moiety may comprise salicylic acid or a derivative thereof, aryl acetic acid or a derivative thereof, heteroaryl acetic acid or a derivative thereof, indoleacetic acid or a derivative thereof, indene acetic acid or a derivative thereof, anthranilic acid or a derivative thereof and/or enolic acid or a derivative thereof. The NSAID moiety can be connected to thymidine via an ester bond.

For example, the NSAID-containing thymidine derivative may be one or more selected from T25 to T50.

In the present application, the thymidine derivative can be used together with the uridine derivative for treating a disease or disorder associated with administration of the chemotherapeutical drug in a subject. For example, the disease or disorder can be hand-foot syndrome or diarrhea.

In the present application, the uridine derivative comprises a structure of Formula (III): 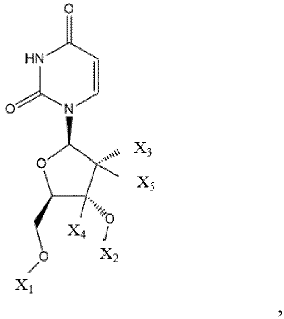 wherein when X₄ and X₅ are both hydrogens, X₁, X₂ and X₃ are not simultaneously hydrogen or are not simultaneously CH₃CO-. For example, the uridine derivative is one or more selected from U1 to U13.

In the present application, the thymidine derivative can comprise a NSAID moiety. For example, the thymidine derivative containing a NSAID moiety can comprise a compound of Formula (IV): 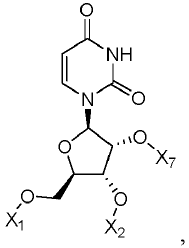 wherein at least one of the X₁, X₂ and X₇ comprises a non-steroidal anti-inflammatory drug (NSAID) moiety. For example, X₁ in Formula (IV) can comprise a NSAID moiety. For example, X₂ in Formula (II) comprises a NSAID moiety. When one of X₁ and X₂ comprises a non-steroidal anti-inflammatory drug (NSAID) moiety, the other may be hydrogen.

For example, both X₁ and X₂ in Formula (IV) comprise NSAID moieties and can comprise the same or different NSAID moieties.

In the present application, the NSAID moiety may comprise, but is not limited to, pyrazolidines, salicylic acids, acetic acid derivatives, oxicams, propanoic acid derivatives, profens and/or fenamic acids. In the present application, the NSAID moiety may comprise salicylic acid or a derivative thereof, aryl acetic acid or a derivative thereof, heteroaryl acetic acid or a derivative thereof, indoleacetic acid or a derivative thereof, indene acetic acid or a derivative thereof, anthranilic acid or a derivative thereof and/or enolic acid or a derivative thereof. The NSAID can be connected to thymidine via an ester bond.

For example, the NSAID-containing uridine derivative may be one or more selected from U14 to U21.

In the present application, the NSAID-containing thymidine derivative and the NSAID-containing uridine derivative can be used for preventing or treating a disease or disorder associated with administration of the chemotherapeutical drug (e.g., hand-foot syndrome and/or diarrhea) in a subj ect.

### Methods of preventing and/or treating disease and relevant uses

In an aspect, the present application provides use of a thymidine derivative in preparation of a drug for preventing or treating a disease or disorder associated with administration of the chemotherapeutical drug (e.g., diarrhea or hand-foot syndrome) in a subject. For example, the thymidine derivative may comprise the compound of Formula (I) herein. For example, the thymidine derivative may comprise the compounds T1 to T24 herein. For example, the thymidine derivative may comprise the compound of Formula (II) herein. For example, the thymidine derivative may comprise the compounds T25 to T50 herein.

In another aspect, the present application provides use of thymidine derivative and uridine derivative in preparation of a drug for preventing or treating a disease or disorder associated with administration of the chemotherapeutical drug in a subject. For example, the thymidine derivative may comprise the compound of Formula (I) herein, and the thymidine derivative may comprise the compound of Formula (III) herein. For example, the thymidine derivative may comprise compounds T1 to T24 herein, and the thymidine derivative may comprise compounds U1 to U13. For example, the thymidine derivative may comprise the compound of Formula (II) herein, and the thymidine derivative may comprise the compound of Formula (IV) herein. For example, the thymidine derivative may comprise compounds T25 to T50 herein, and the thymidine derivative may comprise compounds U14 to U21.

In the present application, the thymidine derivative and/or uridine derivative can be used for preventing or treating hand-foot syndrome caused by the chemotherapeutical drug. For example, the thymidine derivative and/or uridine derivative can be used for preventing or treating hand-foot syndrome caused by 5-FU or 5-FU prodrugs. For example, the thymidine derivative and/or uridine derivative can be used for preventing or treating hand-foot syndrome caused by capecitabine or 5-FU. In the present application, the thymidine derivative and/or uridine derivative can be used for preventing or treating diarrhea caused by chemotherapeutical drug. For example, the thymidine derivative and/or uridine derivative can be used for preventing or treating diarrhea caused by 5-FU or 5-FU prodrugs. For example, the thymidine derivative and/or uridine derivative can prevent or treat diarrhea caused by 5-FU.

In the present application, the thymidine derivative and/or uridine derivative can comprise a NSAID moiety, and the thymidine derivative and/or uridine derivative containing a NSAID moiety can relieve, treat and/or prevent pyrimidine nucleoside analogs or prodrugs thereof (e.g., 5-FU or capecitabine), and compared with the thymidine derivative and/or uridine derivative not containing NSAID, they can relieve both pain and inflammatory responses in the subject, indicating a synergistic effect by retaining the dual effects of the uridine and/or thymidine moieties and the NSAID moiety.

In the present application, the drug may be prepared to be applicable for topical or oral administration. For example, the administration site of topical administration cannot be the site of cancer or the potential site of cancer metastasis. For example, the administration site cannot be the primary site of cancer. Alternatively, for example, the administration site cannot be the metastatic site of cancer. For example, the metastatic site may comprise the metastatic site of cancer caused by lymphatic metastasis, vascular metastasis and/or seeding metastasis. For example, the metastatic site may comprise bone, brain, liver, stomach and/or lung. Alternatively, for example, the administration site cannot be the recurrence site of cancer. In the drug of the present application, the concentration of thymidine derivative may be about 0.0001% (w/w) to about 50% (w/w), e.g., it may vary within a range of about 0.0001% (w/w) to about 10% (w/w), about 0.0001% (w/w) to about 9.5% (w/w), about 0.0001% (w/w) to about 9% (w/w), about 0.0001% (w/w) to about 8.5% (w/w), about 0.0001% (w/w) to about 8% (w/w), about 0.0001% (w/w) to about 7.5% (w/w), about 0.0001% (w/w) to about 7% (w/w), about 0.0001% (w/w) to about 6.5% (w/w), about 0.0001% (w/w) to about 6% (w/w), about 0.0001% (w/w) to about 5.5% (w/w), about 0.0001% (w/w) to about 5% (w/w), about 0.0001% (w/w) to about 4.5% (w/w), about 0.0001% (w/w) to about 4% (w/w), about 0.0001% (w/w) to about 3.5% (w/w), about 0.0001% (w/w) to about 3% (w/w), about 0.0001% (w/w) to about 2.5% (w/w), about 0.0001% (w/w) to about 2% (w/w), about 0.0001% (w/w) to about 1.5% (w/w), about 0.0001% (w/w) to about 1% (w/w), about 0.0001% (w/w) to about 0.5% (w/w), about 0.0001% (w/w) to about 0.01% (w/w) or less. In the drug of the present application, the concentration of thymidine derivative may vary within a range of about 0.0001% (w/w) to about 1% (w/w), about 0.0001% (w/w) to about 0.9% (w/w), about 0.0001% (w/w) to about 0.6% (w/w), about 0.05% (w/w) to about 0.5% (w/w), about 0.05% (w/w) to about 0.4% (w/w), about 0.05% (w/w) to about 0.3% (w/w), about 0.05% (w/w) to about 0.2% (w/w), about 0.1% (w/w) to about 0.2% (w/w) or less. For example, the concentration of thymidine derivative may be about 0.2% (w/w). Alternatively, the concentration of thymidine derivative may be about 0.1% (w/w).

In the present application, the drug comprising the thymidine derivative and/or uridine derivative may not substantially affect the therapeutical effect of the chemotherapeutical drug. For example, the administration of the drug comprising the thymidine derivative and/or uridine derivative substantially does not require an increased administration dose of the chemotherapeutical drug to achieve substantially the same therapeutic effect.

For example, the drug may be prepared to be applicable for oral administration.

In the present application, the drug is oral formulation. Oral formulation may comprise, but is not limited to, capsules, sachets, pills, tablets, lozenges (generally comprising a flavoring base of sucrose and acacia or tragacanth), powders, granules, aqueous or nonaqueous solutions or suspensions, water-in-oil or oil-in-water emulsions, elixirs or syrups, troches (for inert bases, such as, gelatin, glycerin, sucrose, or acacia) and/or mouthwashes or the like.

Oral solid formulations (e.g., capsules, tablets, pills, dragees, powders, or granules, etc.) may comprise an active ingredient and one or more pharmaceutically acceptable adjuvants, such as, sodium citrate or dicalcium phosphate, and/or the following substances: (1) fillers or extenders, e.g., starch, lactose, sucrose, glucose, mannitol and/or silicic acid; (2) binders, e.g., carboxymethylcellulose, alginates, gelatin, polyvinylpyrrolidone, sucrose and/or acacia; (3) humectants, e.g., glycerol; (4) disintegrating agents, e.g., agar, calcium carbonate, potato or tapioca starch, alginic acid, certain silicates and/or sodium carbonate; (5) blocker solution, e.g., paraffin; (6) absorption accelerator, e.g., quaternary ammonium compounds; (7) lubricants, e.g., acetyl alcohol and/or glycerol monostearate; (8) absorbers, e.g., kaolin and/or bentonite; (9) glidants, e.g., talc, calcium stearate, magnesium stearate, solid PEG, sodium lauryl sulfate, and mixtures thereof; and (10) colorants.

Oral liquid formulations may comprise pharmaceutically acceptable emulsions, microemulsions, solutions, suspensions, syrups, and elixirs, etc. In addition to active substances, the liquid dosage forms may comprise common inert diluents, e.g., water or other solvents, solubilizers, and emulsifiers, such as, ethanol, isopropanol, ethyl carbonate, ethyl acetate, benzyl alcohol, benzyl benzoate, propanediol, 1,3-butanediol, oils (in particular, cottonseed oil, peanut oil, corn oil, olive oil, castor oil and sesame oil), glycerol, tetrahydrofurfuryl alcohol, PEG, and fatty acid sorbitol ester, and mixtures of two or more of the above. In addition to inert diluents, the oral liquid formulations may comprise adjuvants, e.g., humectants, emulsifiers, suspending agents, sweetening agents, flavoring agents, pigments, perfumes, or preservatives.

For example, the drug may be prepared to be applicable for topical administration.

For example, the drug may be prepared to be applicable for local administration to skin. For example, the drug may be ointment, lotion or cream.

In the present application, the drug may further comprise one or more active ingredients. For example, the active ingredients may refer to a monomeric compound having a medical effect or physiological activity. For example, the other active ingredients may be selected from the group consisting of anti-inflammatory agents, analgesics, topical anesthetics, antibiotics, antihistamines, preservatives, immunosuppressant and anti-bleeding agents.

In the present application, the drug may further comprise pharmaceutically acceptable carriers. For example, the pharmaceutically acceptable carriers may be selected from the group consisting of fillers, binders, disintegrants, buffers, preservatives, lubricants, flavoring agents, thickeners, colorants, and emulsifiers.

In the present application, the subject may comprise a cancer patient.

In the present application, the subject has been, is being and/or will be administered with the chemotherapeutical drug.

In the present application, the subject may have or be susceptible to have the disease or disorder associated with administration of the chemotherapeutical drug.

In the present application, the severity of the disease or disorder may increase after administration of the chemotherapeutical drug.

In the present application, the subject did not have the disease or disorder before administration of the chemotherapeutical drug.

In the present application, the administration dose of the thymidine derivative may be about 0.0001 µM to about 1500 µM, e.g., about 0.001 µM to about 1500 µM, about 1 µM to about 1500 µM, about 1 µM to about 500 µM, about 1 µM to about 100 µM, about 30 µM to about 900 µM, about 10 µM to about 1000 µM, about 10 µM to about 500 µM.

In the present application, the administration dose of the uridine derivative may be about 0.0001 µM to about 1500 µM, e.g., about 0.001 µM to about 1500 µM, about 1 µM to about 1500 µM, about 1 µM to about 500 µM, about 1 µM to about 100 µM, about 30 µM to about 900 µM, about 10 µM to about 1000 µM, about 10 µM to about 500 µM.

In the present application, the administration dose of the thymidine derivative and uridine derivative may be about 0.0001 µM to about 1500 µM, e.g., about 0.001 µM to about 1500 µM, about 1 µM to about 1500 µM, about 1 µM to about 500 µM, about 1 µM to about 100 µM, about 30 µM to about 900 µM, about 10 µM to about 1000 µM, about 10 µM to about 500 µM.

In the present application, the ratio of the concentration of the thymidine derivative to that of the uridine derivative in the drug is about 1:10 to about 10:1, e.g., about 1:8 to about 8:1, about 1:6 to about 6:1, about 1:4 to about 4:1, about 1:2 to about 2:1. For example, the ratio of the concentration of the compound of T1 to T24 to that of the compound of U1 to U13 is about 1:10, about 1:8, about 1:6, about 1:4, about 1:2, about 2:1, about 4:1, about 6:1, about 8:1 or about 10:1.

In the present application, the ratio of the administration dose of the thymidine derivative to that of the uridine derivative in the method is about 1:10 to about 10:1, e.g., about 1:8 to about 8:1, about 1:6 to about 6:1, about 1:4 to about 4:1, about 1:2 to about 2:1. For example, the ratio of the concentration of the compound of T1 to T24 to that of the compound of U1 to U13 is about 1:10, about 1:8, about 1:6, about 1:4, about 1:2, about 2:1, about 4:1, about 6:1, about 8:1 or about 10:1.

In another aspect, the present application provides a method for treating and/or preventing hand-foot syndrome associated with administration of a chemotherapeutical drug with a compound of T1 to T24, wherein the compound is administered topically. For example, the chemotherapeutical drug is 5-FU or capecitabine. For example, the administration concentration of the compound of T1 to T24 is about 0.5% (wt%) to about 5.0% (wt%) or about 0.5% (wt%) to about 2.0% (wt%).

In another aspect, the present application provides a method for treating and/or preventing hand-foot syndrome associated with administration of the chemotherapeutical drug with a compound of T1 to T24, wherein the compound is administered topically. For example, the chemotherapeutical drug is 5-FU or capecitabine. For example, the administration concentration of the compound of T1 to T24 is about 1 µM to about 1000 µM, about 10 µM to about 1000 µM, about 30 µM to about 500 µM, about 1 µM to about 10 µM, about 1 µM to about 100 µM, about 1 µM to about 200 µM or about 10 µM to about 500 µM.

In another aspect, the present application provides a method for treating and/or preventing diarrhea associated with administration of the chemotherapeutical drug with a compound of T1 to T24, wherein the compound is administered orally. For example, the chemotherapeutical drug is 5-FU or capecitabine. For example, the administration concentration of the compound of T1 to T24 is about 30 mpk to about 150 mpk.

In another aspect, the present application provides a method for treating and/or preventing hand-foot syndrome associated with administration of the chemotherapeutical drug with the combination of a compound of T1 to T24 and a compound of U1 to U13, wherein the compound is administered topically. For example, the chemotherapeutical drug is 5-FU or capecitabine. For example, the ratio of the concentration of the compound of T1 to T24 to that of the compound of U1 to U13 is about 1:10 to about 10:1, e.g., about 1:8 to about 8:1, about 1:6 to about 6:1, about 1:4 to about 4:1, about 1:2 to about 2:1. For example, the ratio of the concentration of the compound of T1 to T24 to that of the compound of U1 to U13 is about 1:10, about 1:8, about 1:6, about 1:4, about 1:2, about 2:1, about 4:1, about 6:1, about 8:1 or about 10:1.

In another aspect, the present application provides a method for treating and/or preventing hand-foot syndrome associated with administration of the chemotherapeutical drug with a combination of a compound of T1 to T24 and a compound of U1 to U13, wherein the compounds are administered topically. For example, the chemotherapeutical drug is 5-FU or capecitabine. For example, the administration concentration of the compound of T1 to T24 is about 0.5% (wt%) to about 5.0% (wt%), the administration concentration of the compound of U1 to U13 is about 0.5% (wt%) to about 5.0% (wt%), and the concentration ratio is 1:10 to about 10:1. For example, the administration concentration of the compound of T1 to T24 is about 1.0% (wt%) to about 5.0% (wt%), the administration concentration of the compound of U1 to U13 is about 1.0% (wt%) to about 5.0% (wt%), and the concentration ratio is 1:10 to about 10:1.

In another aspect, the present application provides a method for treating and/or preventing hand-foot syndrome associated with administration of the chemotherapeutical drug or diarrhea with a combination of a compound of T1 to T24 and a compound of U1 to U13, wherein the compounds are administered orally. For example, the chemotherapeutical drug is 5-FU or capecitabine. For example, the administration concentration of the compound of T1 to T24 is about 100 mpk to about 300 mpk, the administration concentration of the compound of U1 to U13 is about 200 mpk to about 300 mpk, and the concentration ratio is 1:3 to about 3:1.

In another aspect, the present application provides a method for treating and/or preventing diarrhea associated with administration of the chemotherapeutical drug with a combination of a compound of T1 to T24 and a compound of U1 to U13, wherein the compounds are administered orally. For example, the chemotherapeutical drug is 5-FU or capecitabine. For example, the administration concentration of the compound of T1 to T24 is about 30 mpk to about 150 mpk, the administration concentration of the compound of U1 to U13 is about 1 mpk to about 50 mpk, and the concentration ratio is 1:3 to about 3:1.

In another aspect, the present application provides a method for treating and/or preventing hand-foot syndrome associated with administration of the chemotherapeutical drug with a combination of a compound of T1 to T24 and a compound of U1 to U13, wherein the compounds are administered orally. For example, the chemotherapeutical drug is 5-FU or capecitabine. For example, the administration concentration of the compound of T1 to T24 is about 30 mpk to about 150 mpk, the administration concentration of the compound of U1 to U13 is about 1 mpk to about 50 mpk, and the concentration ratio is 1:1 to about 2: 1.

In another aspect, the present application provides a method for treating and/or preventing hand-foot syndrome associated with administration of the chemotherapeutical drug with a compound of T25 to T50, wherein the compound is administered topically. For example, the chemotherapeutical drug is 5-FU or capecitabine. For example, the concentration of the compound of T25 to T50 is about 0.5 µM to about 12.5 µM, about 0.5 µM to about 50 µM, 0.5 µM to about 100 µM, 3 µM to about 100 µM, 10 µM to about 100 µM, 5 µM to about 200 µM, 2 µM to about 1000 µM, or 5 µM to about 500 µM.

In another aspect, the present application provides a method for treating and/or preventing hand-foot syndrome associated with administration of the chemotherapeutical drug with a compound of T25 to T50, wherein the compound is administered topically. For example, the chemotherapeutical drug is 5-FU or capecitabine. For example, the administration concentration of the compound of T25 to T50 is about 0.01% (wt%) to about 5.0% (wt%). For example, the administration concentration of the compound of T25 to T50 is about 0.1% (wt%) to about 5.0% (wt%). For example, the administration concentration of the compound of T25 to T50 is about 1.01% (wt%) to about 5.0% (wt%). For example, the administration concentration of the compound of T25 to T50 is about 3.0% (wt%) to about 5.0% (wt%). For example, the administration concentration of the compound of T25 to T50 is about 1.0 (wt%) to about 3.0% (wt%).

In another aspect, the present application provides a method for treating and/or preventing hand-foot syndrome associated with administration of the chemotherapeutical drug with a compound of T25 to T50, wherein the compound is administered orally. For example, the chemotherapeutical drug is 5-FU or capecitabine. For example, the administration concentration of the compound of T251 to T50 is about 100 mpk to about 500 mpk. For example, the administration concentration of the compound of T251 to T50 is about 200 mpk to about 300 mpk.

In another aspect, the present application provides a method for treating and/or preventing diarrhea associated with administration of the chemotherapeutical drug with a compound of T25 to T50, wherein the compound is administered orally. For example, the chemotherapeutical drug is 5-FU or capecitabine. For example, the administration concentration of the compound of T25 to T50 is about 10 mpk to about 50 mpk. For example, the administration concentration of the compound of T25 to T50 is about 1 mpk to about 100 mpk.

In another aspect, the present application provides a method for treating and/or preventing hand-foot syndrome associated with administration of the chemotherapeutical drug with a combination of a compound of T25 to T50 and a compound of U14 to U21, wherein the compounds are administered topically. For example, the chemotherapeutical drug is 5-FU or capecitabine. For example, the ratio of the concentration of the compound of T25 to T50 to that of the compound of U14 to U21 is about 1: 10 to about 10:1, e.g., about 1:8 to about 8:1, about 1:6 to about 6:1, about 1:4 to about 4:1, about 1:2 to about 2:1. For example, the ratio of the concentration of the compound of T1 to T24 to that of the compound of U1 to U13 is about 1:10, about 1:8, about 1:6, about 1:4, about 1:2, about 2:1, about 4:1, about 6:1, about 8:1 or about 10:1.

In another aspect, the present application provides a method for treating and/or preventing hand-foot syndrome associated with administration of the chemotherapeutical drug with a combination of a compound of T25 to T50 and a compound of U14 to U21, wherein the compounds are administered orally. For example, the chemotherapeutical drug is 5-FU or capecitabine. For example, the administration concentration of the compound of T25 to T50 is about 0.5 µM to about 25 µM, the administration concentration of the compound of U14 to U21 is about 0.5 µM to about 25 µM, and the concentration ratio of the compound of T25 to T50 to the compound of U14 to U21 is about 1:10 to about 10:1. For example, the administration concentration of the compound of T25 to T50 is about 0.5 µM to about 15 µM, the administration concentration of the compound of U14 to U21 is about 0.5 µM to about 15 µM, and the concentration ratio of the compound of T25 to T50 to the compound of U14 to U21 is about 1:3 to about 3:1.

In another aspect, the present application provides a method for treating and/or preventing hand-foot syndrome associated with administration of the chemotherapeutical drug with a combination of a compound of T25 to T50 and a compound of U14 to U21, wherein the compounds are administered topically. For example, the chemotherapeutical drug is 5-FU or capecitabine. For example, the administration concentration of the compound of T25 to T50 is about 0.01% (wt%) to about 5.0% (wt%), the administration concentration of the compound of U14 to U21 is about 1% (wt%) to about 5.0% (wt%) and the concentration ratio of the compound of T25 to T50 to the compound of U14 to U21 is about 1:3 to about 3:1. For example, the chemotherapeutical drug is 5-FU or capecitabine. For example, the administration concentration of the compound of T25 to T50 is about 1.0% (wt%) to about 5.0% (wt%), the administration concentration of the compound of U14 to U21 is about 1.0% (wt%) to about 5.0% (wt%), and the concentration ratio of the compound of T25 to T50 to the compound of U14 to U21 is about 1:3 to about 3:1.

In another aspect, the present application provides a method for treating and/or preventing hand-foot syndrome associated with administration of the chemotherapeutical drug with a combination of a compound of T25 to T50 and a compound of U14 to U21, wherein the compounds are administered orally. For example, the chemotherapeutical drug is 5-FU or capecitabine. For example, the administration concentration of the compound of T25 to T50 is about 30 mpk to about 150 mpk, the administration concentration of the compound of U14 to U21 is about 1 mpk to about 100 mpk, and the concentration ratio of the compound of T25 to T50 to the compound of U14 to U21 is about 1:3 to about 3:1.

In another aspect, the present application provides a method for treating and/or preventing diarrhea associated with administration of the chemotherapeutical drug with a combination of a compound of T25 to T50 and a compound of U14 to U21, wherein the compounds are administered orally. For example, the chemotherapeutical drug is 5-FU or capecitabine. For example, the administration concentration of the compound of T25 to T50 is about 10 mpk to about 50 mpk, the administration concentration of the compound of U14 to U21 is about 1 mpk to about 100 mpk, and the concentration ratio of the compound of T25 to T50 to the compound of U14 to U21 is about 1:3 to about 3:1.

In another aspect, the present application provides a pharmaceutical combination or a kit, comprising: 1) the chemotherapeutical drug; and 2) the thymidine derivative and/or uridine derivative.

In the pharmaceutical combination or the kit of the present application, the chemotherapeutical drug and the thymidine derivative may be each independently present in separate containers.

In the pharmaceutical combination or the kit of the present application, the thymidine derivative in 2) can prevent or treat a disease or disorder associated with administration of the chemotherapeutical drug in 1).

In the pharmaceutical combination or the kit of the present application, the thymidine derivative in 2) does not substantially affect the therapeutical effect of the chemotherapeutical drug in 1).

In the pharmaceutical combination or the kit of the present application, the thymidine derivative in 2) may be administered before, simultaneously with, or after the administration of the chemotherapeutical drug in 1).

In another aspect, the present application provides a method comprising administering to a subject thymidine derivative, wherein the subject has been, is being and/or will be administered with the chemotherapeutical drug and has or is susceptible to the disease or disorder associated with administration of the chemotherapeutical drug.

In the present application, the subject has been, is being and/or will be administered with the chemotherapeutical drug.

In the present application, the subject may comprise human or non-human animals. For example, the non-human animals may be selected from the group consisting of monkeys, chickens, geese, cats, dogs, mice, and rats. Moreover, non-human animals may also comprise any animal species other than humans, e.g., livestocks, or rodents, or primates, or domestic animals, or poultry animals.

For example, the thymidine derivative and/or uridine derivative may be administered before, simultaneously with, or after the administration of the chemotherapeutical drug to the subject. In the embodiments in which the thymidine derivative and/or uridine derivative and the chemotherapeutical drug are administered at intervals, the thymidine derivative and/or uridine derivative may be administered at intervals before or after the administration of the chemotherapeutical drug. The time intervals may be 1 minute, 2 minutes, 5 minutes, 10 minutes, 20 minutes, 30 minutes, 45 minutes, 1 hour, 2 hours, 3 hours, 4 hours, 5 hours, 6 hours, 12 hours, 18 hours, 1 day, 2 days, 3 days, 1 week, 2 weeks, 3 weeks, 1 month, 2 months, 3 months or longer.

Without being limited by any theory, the following examples merely illustrate the fusion protein, preparation method, and use of the present application, and are not intended to limit the scope of the invention of the present application.

### EXAMPLES

In the results of the examples of the present application, ** represents P<0.01; * represents P<0.05; *** represents P<0.001, by t-test statistical test.

### Example 1: Synthesis of T6 (((2R,3S,5R)-3-(butyrytoxy)-5-(5-methyt-2,4-dioxo-3,4-dihydropyrimidin-1(2H)-yl) tetrahydrofuran-2-yl) methyl butyrate)

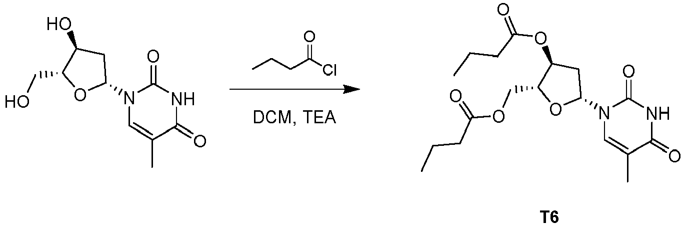

Uridine thymidine (2.0 g, 8.26 mmol) and triethylamine (1.67 g, 16.5mmol) were dissolved in 10 mL of dichloromethane, and butyryl chloride (1.76 g, 16.5 mmol) was added at 0°C. The reaction mixture was stirred at 25°C for 2 hrs. TLC chromatography showed that the reaction was completed. The reaction mixture was poured into 20 ml of water and 20 ml of dichloromethane. After extraction, the organic phase was separated, washed with saturated brine (20 ml), dried over anhydrous sodium sulfate, and rotary evaporated to give a crude product. The crude product was purified by reverse phase HPLC (Column: Xtimate C18 150 × 40 mm × 10 µm; Mobile phase: water (0.225%FA) - ACN; B%: 20-50; 10 min) to give the product T6 (40.0 mg, 98.14% purity). The results of structural assay show that 1H NMR (400 MHz, DMSO-d6) δ 11.34 (br s, 1H), 7.40 (d, 1H, J = 7.6 Hz), 6.18 (br t, 1H, J = 6.8 Hz), 5.45 (br s, 1H), 4.26 (br d, 2H, J = 3.6 Hz), 4.19 (br s, 1H), 3.9-3.9 (m, 1H), 3.60 (td, 1H, J = 7.2, 10.9 Hz), 3.39 (s, 1H), 2.5-2.6 (m, 1H), 2.48 (br s, 1H), 2.0-2.2 (m, 2H), 1.7-1.8 (m, 4H), 1.4-1.5 (m, 2H), 0.7-0.9 (m, 6H). LCMS(M+H) +, 383.0.

The following compounds were synthesized by the similar method as T6, and the results of structural assay are shown in Table 2.

**Table 2 Results of structural assay of thymidine derivatives**

| Compound Name | LCMS (M+H)⁺ | Compound Structure |
|---|---|---|
| T1 | 327.1 | 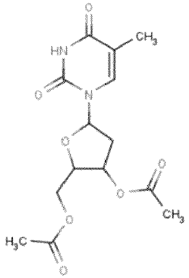 |
| T2 | 317.1 | 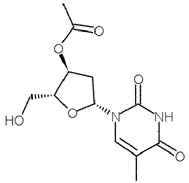 |
| T3 | 317.1 | 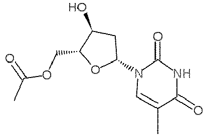 |
| T4 | 327.2 | 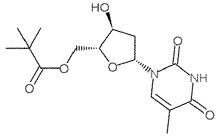 |
| T5 | 355.1 | 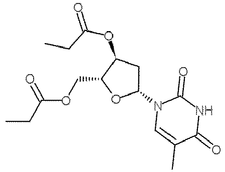 |
| T7 | 299.1 | 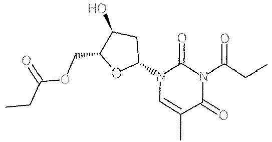 |
| T8 | 435.3 | 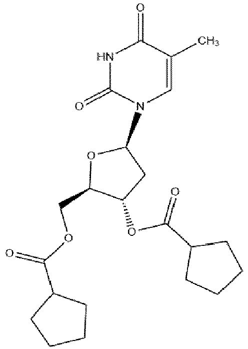 |
| T9 | 379.1 | 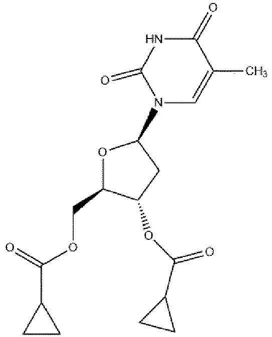 |
| T10 | 479.2 | 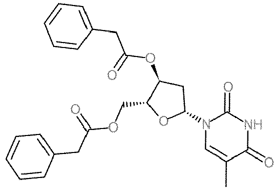 |
| T11 | 327.1 | 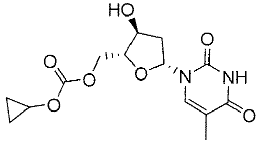 |
| T12 | 465.2 | 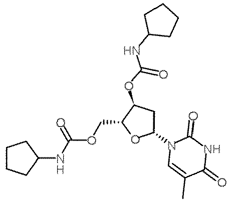 |
| T13 | 383.2 | 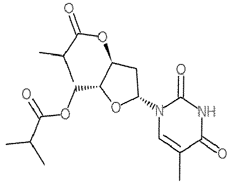 |
| T14 | 483.1 | 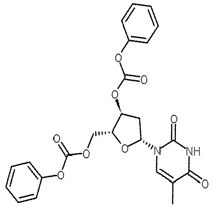 |
| T15 | 409.2 | 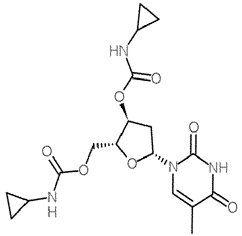 |
| T16 | 481.2 | 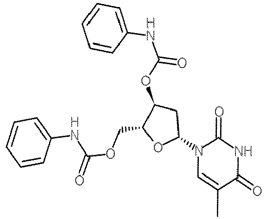 |
| T17 | 326.2 | 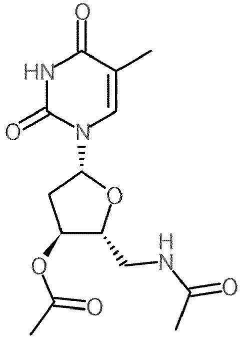 |
| T18 | 357.2 | 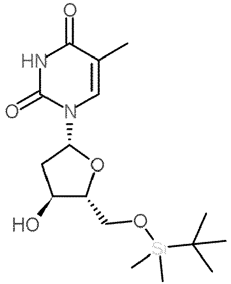 |
| T19 | 377.1 | 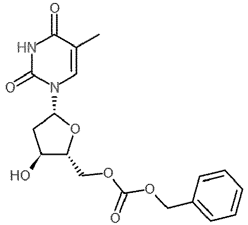 |
| T20 | 443.2 | 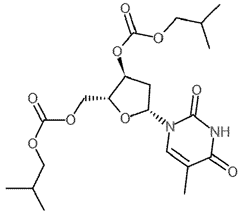 |
| T21 | 471.2 | 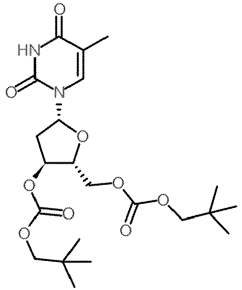 |
| T22 | 415.2 | 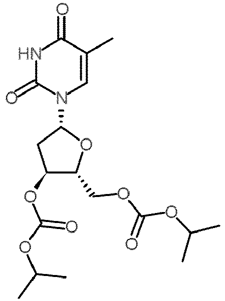 |
| T23 | 443.2 | 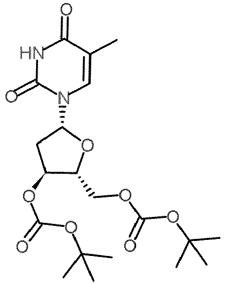 |
| T24 | 411.2 | 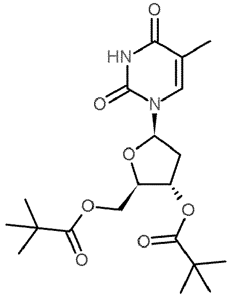 |

### Examples 2-29: Effect of thymidine derivative or a combination of thymidine derivative and uridine derivative on relieving proliferative toxicity of chemotherapeutical drug to HaCaT cells

The cultured skin cell HaCaTs were digested, counted, and inoculated into a 96-well plate with 5000 - 10000 cells in each well. After 24h of culture, the wells were divided into Group of blank solvent control, Group of 5-FU, Group of 5-FU + thymidine derivative, Group of 5-FU + the combination of thymidine derivative and uridine derivative. Group of 5-FU: adding 1 µL of a solution of 5-FU (the final concentration is shown in Table 3, the solutions of 5-FU are all DMSO solutions); Group of 5-FU + thymidine derivative: adding a solution of 5-FU and a solution of thymidine derivative (the final concentrations of 5-FU and thymidine derivative are shown in Table 3, the solution of thymidine is an aqueous solution, while the solutions of thymidine derivatives are all DMSO solutions); Group of 5-FU + the combination of thymidine derivative and uridine derivative: adding a solution of 5-FU and a mixed solution of thymidine derivative and uridine derivative (the final concentrations of the solution of 5-FU and the mixed solution of thymidine derivative + uridine derivative are shown in Table 3, the solution of thymidine is an aqueous solution, while the mixed solutions of thymidine derivative + uridine derivative are all DMSO solutions); Group of blank solvent control: adding the same type of solution as those of the corresponding Group of 5-FU or Group of 5-FU + thymidine derivative (or the mixed solution of thymidine derivative +uridine derivative) in equal volume. Group of blank solvent control was used as control to exclude the influence of the solvents in Group of 5-FU and Group of 5-FU + thymidine derivative (or the mixed solution of thymidine derivative + uridine derivative) on the results. After additional 48 hours of culture, a Cell Counting Kit-8 (CCK-8) detection kit (C0037, purchased from Shanghai Beyotime Biochnology) is used to determine the cell survival rate, thereby calculating the proliferative toxicity of 5-Fu/5-DFUR on cells and the relieving effect of the thymidine derivative (or the mixed solution of thymidine derivative + uridine derivative) on the proliferative toxicity. Using GraphPad Prism 6.0 software, the results were statistically analyzed by t-test and plotted.

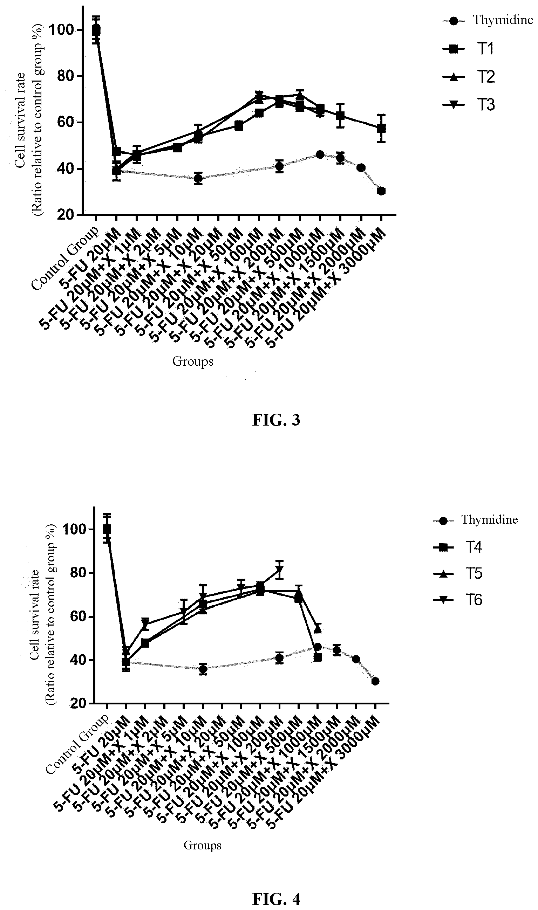

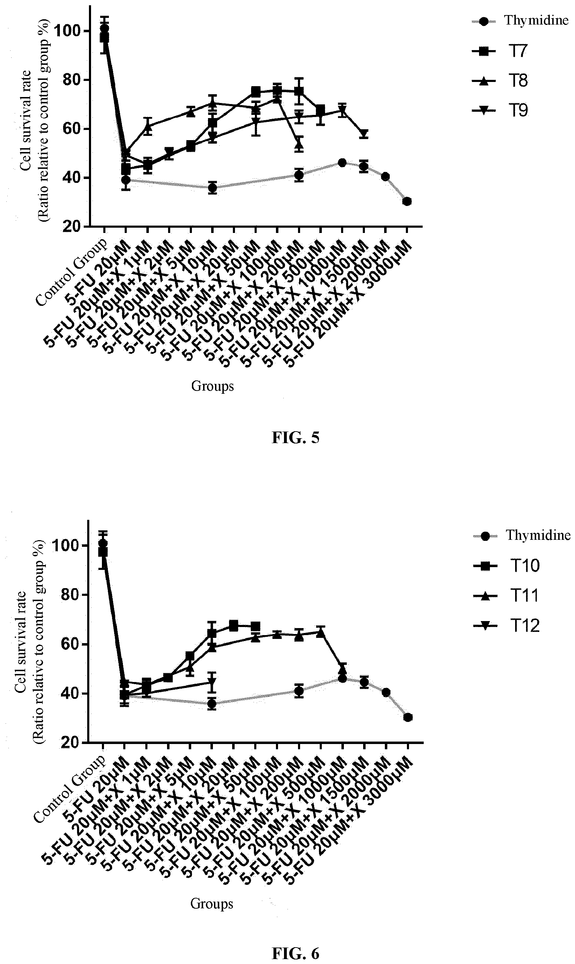

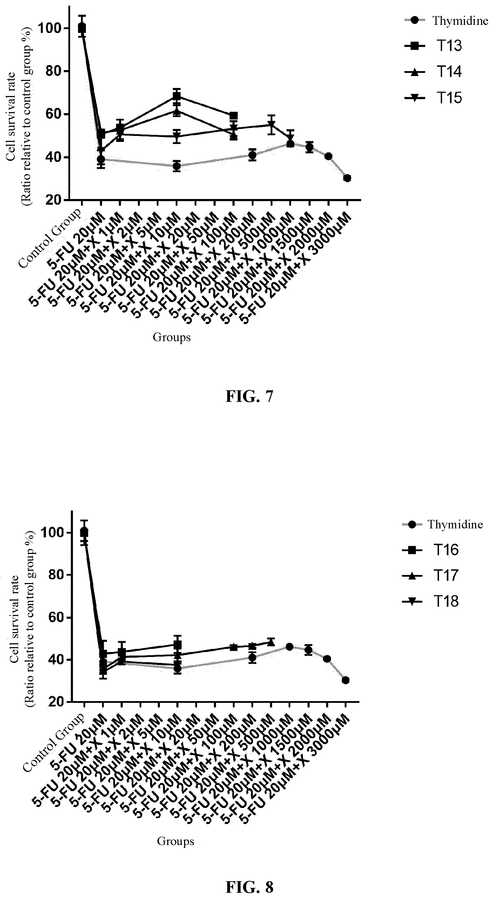

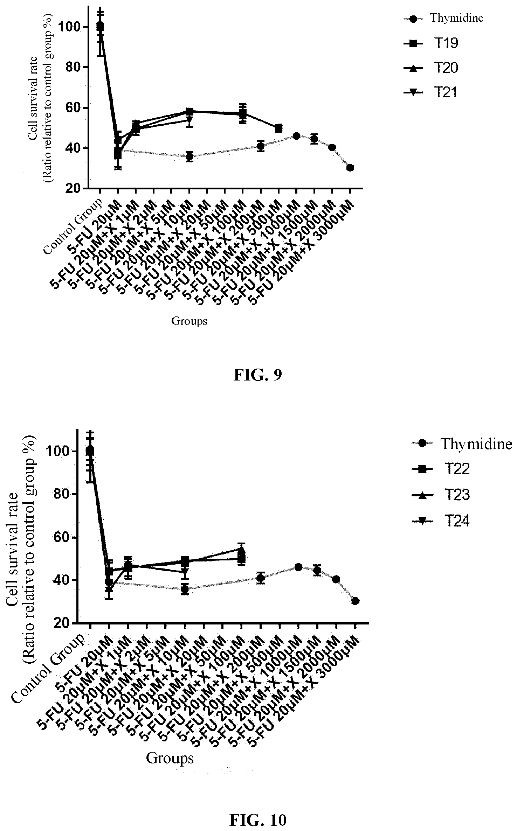

Table 3 lists the combinations of 5-FU and thymidine derivative (or the mixed solution of thymidine derivative + uridine derivative), and the corresponding experimental results (wherein the data in the column of cell survival indicate the corresponding increased percentage of the cell survivals of Group of 5-FU + thymidine derivative or Group of 5-FU + the combination of thymidine derivative and uridine derivative compared with the Group of 5-FU). FIGS. 3-10 list the experimental results of several representative compounds.

**Table 3 Experimental Conditions and Results of Examples 2-29**

| Example No. | 5-FU | Final Concentration | Selected Compound | Final Concentration | Cell Survival Rate |
|---|---|---|---|---|---|
| 2 | 5-FU | 20 µM | thymidine | 10 µM | -5% - -10% |
| | | | | 200 µM | 3%-8% |
| | | | | 1000 µM | 15%-23 |
| | | | | 1500 µM | 11%-17% |
| | | | | 2000 µM | 1%-6% |
| | | | | 3000 µM | -25%-20% |
| 3 | | | T1 | 5 µM | 1%-6% |
| | | | | 10 µM | 10%-16% |
| | | | | 50 µM | 21%-26% |
| | | | | 100 µM | 31%-37% |
| | | | | 200 µM | 42%-48% |
| | | | | 500 µM | 36%-41% |
| | | | | 1000 µM | 35%-42% |
| | | | | 1500 µM | 29%-33% |
| | | | | 3000 µM | 17%-23% |
| 4 | | | T2 | 1 µM | 14%-18% |
| | | | | 10 µM | 35%-45% |
| | | | | 100 µM | 70%-80% |
| | | | | 500 µM | 75%-85% |
| | | | | 1000 µM | 60%-70% |
| 5 | | | T3 | 1 µM | 13%-18% |
| | | | | 10 µM | 30-38% |
| | | | | 100 µM | 80%-86% |
| | | | | 500 µM | 69%-75% |
| | | | | 1000 µM | 58%-65% |
| 6 | | | T4 | 1 µM | 20%-36% |
| | | | | 10 µM | 65%-72% |
| | | | | 100 µM | 80%-90% |
| | | | | 500 µM | 70%-77% |
| | | | | 1000 µM | 2%-7% |
| 7 | | | T5 | 1 µM | 18%-24% |
| | | | | 10 µM | 55%-65% |
| | | | | 100 µM | 77%-84% |
| | | | | 500 µM | 78%-85% |
| | | | | 1000 µM | 35%-45% |
| 8 | | | T6 | 1 µM | 27%-34% |
| | | | | 5 µM | 40%-48% |
| | | | | 10 µM | 55%-65% |
| | | | | 50 µM | 65%-75% |
| | | | | 100 µM | 68%-75% |
| | | | | 200 µM | 85%-95% |
| 9 | | | T7 | 1 µM | 2%-6% |
| | | | | 5 µM | 18%-24% |
| | | | | 10 µM | 40%-47% |
| | | | | 50 µM | 65%-75% |
| | | | | 100 µM | 70%-80% |
| | | | | 200 µM | 68%-76% |
| | | | | 500 µM | 50%-60% |
| 10 | | | T8 | 1 µM | 15%-25% |
| | | | | 5 µM | 27%-37% |
| | | | | 10 µM | 35%-45% |
| | | | | 50 µM | 32%-39% |
| | | | | 100 µM | 40%-47% |
| | | | | 200 µM | 3%-9% |
| 11 | | | T9 | 1 µM | -10% - -3% |
| | | | | 10 µM | 10%-18% |
| | | | | 50 µM | 24%-31% |
| | | | | 200 µM | 27%-34% |
| | | | | 500 µM | 30%-38% |
| | | | | 1000 µM | 35%-40% |
| | | | | 1500 µM | 13%-20% |
| 12 | | | T10 | 1 µM | 5%-10% |
| | | | | 2 µM | 14%-22% |
| | | | | 5 µM | 35%-45% |
| | | | | 10 µM | 60%-70% |
| | | | | 20 µM | 65%-75% |
| | | | | 50 µM | 65%-75% |
| 13 | | | T11 | 1 µM | -5%-0% |
| | | | | 5 µM | 11%-16% |
| | | | | 10 µM | 27%-33% |
| | | | | 50 µM | 35%-45% |
| | | | | 100 µM | 38%-45% |
| | | | | 200 µM | 38%-46% |
| | | | | 500 µM | 40%-50% |
| | | | | 1000 µM | 7%-14% |
| 14 | | | T12 | 1 µM | 0%-5% |
| | | | | 10 µM | 9%-16% |
| 15 | | | T13 | 1 µM | 15%-25% |
| | | | | 10 µM | 40%-50% |
| | | | | 100 µM | 25%-35% |
| 16 | | | T14 | 1 µM | 12%-20% |
| | | | | 10 µM | 30%-40% |
| | | | | 100 µM | 8%-15% |
| 17 | | | T15 | 1 µM | 13%-20% |
| | | | | 10 µM | 13%-20% |
| | | | | 100 µM | 20%-30% |
| | | | | 500 µM | 25%-35% |
| | | | | 1000 µM | 9%-16% |
| 18 | | | T16 | 1 µM | 0%-5% |
| | | | | 10 µM | 7%-14% |
| 19 | | | T17 | 1 µM | 15%-25% |
| | | | | 10 µM | 18%-26% |
| | | | | 100 µM | 25%-35% |
| | | | | 200 µM | 30%-40% |
| | | | | 500 µM | 35%-45% |
| 20 | | | T18 | 1 µM | 6%-12% |
| | | | | 10 µM | 7%-15% |
| 21 | | | T19 | 1 µM | 35%-45% |
| | | | | 10 µM | 60%-70% |
| | | | | 100 µM | 58%-67% |
| | | | | 500 µM | 35%-45% |
| 22 | | | T20 | 1 µM | 48%-58% |
| | | | | 10 µM | 58%-66% |
| | | | | 100 µM | 55%-65% |
| 23 | | | T21 | 1 µM | 5%-13%% |
| | | | | 10 µM | 19%-26% |
| 24 | | | T22 | 1 µM | 2%-7% |
| | | | | 10 µM | 10%-17% |
| | | | | 100 µM | 9%-16% |
| 25 | | | T23 | 1 µM | 2%-7% |
| | | | | 10 µM | 5%-11% |
| | | | | 100 µM | 20%-26% |
| 26 | | | T24 | 1 µM | 27%-35% |
| | | | | 10 µM | 21%-27% |
| 27 | | | T1+U1 | 30 µM+250 µM | 20%-27% |
| | | | | 150 µM+250 µM | 83%-93% |
| | | | | 300 µM+250 µM | 107%-117% |
| | | | | 450 µM+250 µM | 112%-122% |
| | | | | 600 µM+250 µM | 127%-137% |
| | | | | 900 µM+250 µM | 98%-108% |
| | | | | 1500 µM+250 µM | 63%-83% |
| | | | | 30 µM+500 µM | 48%-55% |
| | | | | 150 µM+500 µM | 110%-120% |
| | | | | 300 µM+500 µM | 120%-130% |
| | | | | 450 µM+500 µM | 135%-145% |
| | | | | 600 µM+500 µM | 120%-130% |
| | | | | 900 µM+500 µM | 70%-80% |
| 28 | | | T2+U4 | 1 µM+100 µM | 30%-40% |
| | | | | 10 µM+100 µM | 70%-80% |
| | | | | 100 µM+100 µM | 90%-100% |
| | | | | 500 µM+100 µM | 110%-120% |
| | | | | 1000 µM+100 µM | 90%-100% |
| | | | | 1 µM+500 µM | 45%-55% |
| | | | | 10 µM+500 µM | 60%-70% |
| | | | | 100 µM+500 µM | 100%-110% |
| | | | | 500 µM+500 µM | 115%-125% |
| | | | | 1000 µM+500 µM | 95%-105% |
| 29 | | | T7+U6 | 5 µM+100 µM | 40%-50% |
| | | | | 10 µM+100 µM | 60%-70% |
| | | | | 50 µM+100 µM | 95%-105% |
| | | | | 100 µM+100 µM | 105%-112% |
| | | | | 500 µM+100 µM | 82%-92% |
| | | | | 5 µM+200 µM | 55%-65% |
| | | | | 10 µM+200 µM | 78%-86% |
| | | | | 50 µM+200 µM | 105%-115% |
| | | | | 100 µM+200 µM | 128%-138% |
| | | | | 500 µM+200 µM | 106%-114% |

The results in Table 3 showed that fluorinated drugs have certain proliferative toxicity to the skin cell HaCaTs, while the thymidine derivative or the combination of thymidine derivative and uridine derivative has a significant relieving effect on the proliferative toxicity caused by the fluorinated drugs. The addition of the thymidine derivative or the combination of thymidine derivative and uridine derivative increases the cell survival rate and has a certain advantage over the thymidine.

### Examples 30-40: Thymidine derivative or a combination of thymidine derivative and uridine derivative can prevent/treat hand-foot syndrome caused by a chemotherapeutical drug in a rat model

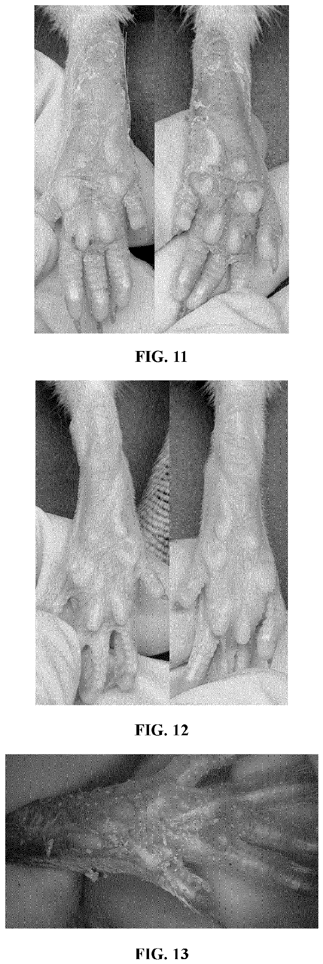

A rat animal model was established. 6-week aged female SD rats were administered with capecitabine by daily gavage. After a few days, rats' paws developed hand-foot syndrome symptoms (photographs are shown in Figure 11). The degree of hand-foot syndrome in paws is similar, and there was no difference between the left and right paws. Similar to the human body, rats developed hand-foot syndrome after oral administration of capecitabine. The causes of the two are the same, and the disorder is also very similar. Therefore, rats are very good animal models for simulating the hand-foot syndrome caused by capecitabine.

After SD rats were fed and adapted for one week (about 200 g), the rats were divided into groups, with 10 rats in each group. Then, the rats were subject to gavage administration. Capecitabine was dissolved in a 1:1 mixed solution of castor oil and ethanol and diluted for three times with PBS buffer. Each rat was subject to gavage in an amount of 1 mL/100 g, and the administration dose is shown in Table 4. After gavage, the rats were fixed with a fixed cylinder, and a gel of thymidine derivative (or a compounded gel of thymidine derivative and uridine derivative) was applied to double hind paws (about 1 cm * 3 cm) of the rats in group of administration (the types and concentrations are shown in Table 4), and a blank gel was applied to the group of blank (as a blank control). After approximately 4 hours of application, the rats were released after 4 hours, wiped with clean water to remove any residual drug at the application site, and allowed back to the cage. The frequency of capecitabine gavage is shown in Table 4. The thymidine derivative gel (or the compounded gel of thymidine derivative and uridine derivative) was administered with gavage every day. The gavage and application tests are repeated daily until the group of blank has developed obvious hand-foot syndrome. At this time, the number of rats of which the paw skins remained normal or had significantly less serious symptoms as compared with the hand-foot syndrome in group of blank was calculated as the number of rats in which the hand-foot syndrome was effectively inhibited.

Table 4 lists the combinations of animal experiments of capecitabine and the thymidine derivative gel (or the compounded gel of thymidine derivative and uridine derivative) and the corresponding experimental results (wherein, the data in the column of control rate = the occurrence rate of hand-foot syndrome in group of blank - the occurrence rate of hand-foot syndrome in group of administration).

**Table 4 Experimental Conditions and Results of Examples 30-40**

| Example No. | Chemotherapeutical Drug | Dose | Frequency | Application | Concentration wt% | Control | Days | Control Rate |
|---|---|---|---|---|---|---|---|---|
| 30 | Capecitabine | 4000 mg/k g | Once daily | T1 | 0.5% | Betwe en groups | 40 | 20.00 % |
| 31 | | | | T1 | 2% | Betwe en groups | 40 | 27.50 % |
| 32 | | | | T1 | 5% | Betwe en groups | 40 | 56.67 % |
| 33 | | | | T2 | 1% | Betwe en groups | 40 | 23.33 % |
| 34 | | | | T3 | 1% | Betwe en groups | 40 | 27.50 % |
| 35 | | | | T4 | 2% | Betwe en groups | 40 | 32.86 % |
| 36 | | | | T5 | 2% | Betwe en groups | 40 | 30.00 % |
| 37 | | | | T1+U1 | 1%+3% | Betwe en groups | 40 | 45.56 % |
| 38 | | | | T1+U1 | 2%+6% | Betwe en groups | 40 | 70.00 % |
| 39 | | | | T3+U5 | 1%+2% | Between groups | 40 | 45.56 % |
| 40 | | | | T10+U7 | 0.5%+2% | Betwe en groups | 40 | 56.67 % |

The results (Table 4 and FIG. 12) showed that different concentrations or different types of the thymidine derivative or a combination of thymidine derivative and uridine derivative can both relieve the hand-foot syndrome to an extent.

### Examples 41-52: Thymidine derivative or a combination of thymidine derivative and uridine derivative can prevent/treat hand-foot syndrome caused by a chemotherapeutic drug in a mouse model.

A mouse animal model was established. 6-week aged male Balb/c mice were administered with capecitabine by daily gavage. After a few days, mice's paws developed hand-foot syndrome symptoms (similar to the results of the rat model, such as, in FIG. 13). Similar to the human body, mice developed hand-foot syndrome in the forepaws and hind paws after oral administration of capecitabine. The causes of the two are the same, and the disorder is also very similar. Therefore, mice are very good animal models for simulating the hand-foot syndrome caused by capecitabine.

After Balb/c mice were fed and adapted for one week (about 20 g), the mice were divided into groups, with 10 mice in each group. Then, the mice were subject to gavage administration. Capecitabine was dissolved in normal saline with pH=8, and the gavage dose was not greater than 0.3 mL at each time. The administration dose is shown in Table 5. After gavage, the blank gel was applied to the forepaws and the hind paws (about 4 * 0.5 cm * 0.5 cm) in group of blank gel, and gels containing different drugs were applied to the forepaws and the hind paws (about 4 * 0.5cm * 0.5cm) in group of administration. After application, the mice were fixed in Elizabethan collar for 4 hours. After 4 hours, the mice were released, wiped with clean water to remove any residual drug at the application site, and allowed back to the cage. The frequency of capecitabine gavage is shown in Table 5. The gavage and application tests are repeated daily until the group of blank has developed obvious hand-foot syndrome. At this time, the number of rats of which the paw skins remained normal or had significantly less serious symptoms in the group of administration as compared with the hand-foot syndrome in group of blank was calculated as the number of rats in which the hand-foot syndrome was effectively inhibited. Additionally, the thymidine derivative and the combination of thymidine derivative and uridine derivative were orally administered for observing the relief of the hand-foot syndrome. In examples 41-48, thymidine derivative and/or uridine derivative were/was applied, and in examples 49-52, thymidine derivative and/or uridine derivative were/was administered orally.

Table 5 lists the combinations of animal experiments of capecitabine and thymidine derivative (or a combination of thymidine derivative and uridine derivative) and the corresponding experimental results (wherein the data in the column of control rate = the occurrence rate of hand-foot syndrome in group of blank - the occurrence rate of hand-foot syndrome in group of administration).

**Table 5 Experimental Conditions and Results of Examples 41-52**

| | | | | | Concentrati on wt% | | | Control Rate | |
|---|---|---|---|---|---|---|---|---|---|
| Example No. | Chemotherap eutical Drug | Dose | Frequ ency | Applica tion | | Contr ol | Day s | Forep aws | Hind Paws |
| 41 | Capecitabine | 1000 mg/kg | Once daily | T1 | 0.5% | Betwe en group s | 15 | 30% | 20% |
| 42 | | | | T1 | 2% | Betwe en group s | 15 | 45.56 % | 34.44 % |
| 43 | | | | T1 | 5% | Betwe en group s | 15 | 67.78 % | 78.89 % |
| 44 | | | | T2 | 2% | Betwe en group s | 15 | 40% | 27.5 % |
| 45 | | | | T3 | 2% | Betwe en group s | 15 | 20% | 30% |
| 46 | | | | T1+U1 | 1.5%+4.5% | Betwe en group s | 15 | 40% | 15% |
| 47 | | | | T1+U1 | 5%+5% | Betwe en group s | 15 | 67.78 % | 56.67 % |
| 48 | | | | T2+U3 | 1%+3% | Betwe en group s | 15 | 56.67 % | 67.78 % |
| 49 | | | | T1 | 300 mpk, twice per day | Betwe en group s | 8 | 80.0 % | 90% |
| 50 | | | | T1+U1 | 300 mpk+300 mpk, twice per day | Betwe en group s | 8 | 78.89 % | 67.78 % |
| 51 | | | | T6 | 200 mpk, twice per day | Betwe en group s | 8 | 65% | 77.5 % |
| 52 | | | | T5+U9 | 100 mpk+200 mpk, twice per day | Betwe en group s | 8 | 78.89 % | 67.78 % |

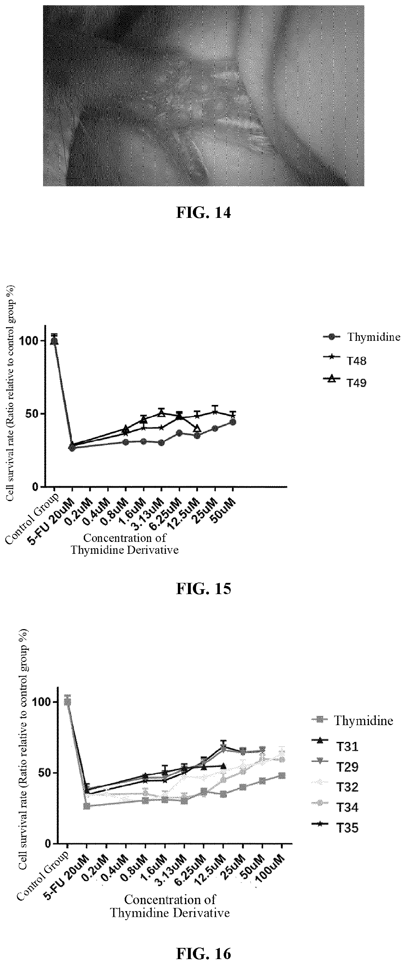

The results showed that (Table 5 and FIG. 14), different concentrations or different types of thymidine derivative and a combination of thymidine derivative and uridine derivative can all relieve the hand-foot syndrome to an extent; while oral administration of thymidine derivative and a combination of thymidine derivative and uridine derivative can all reduce the occurrence of hand-foot syndrome when using capecitabine. Also, it is found in the experiments that some experimental mice in the model group also develop paronychia or other symptoms, but those in the oral administration group do not develop the symptoms of other severe skin side effects like paronychia, etc. (other than hand-foot syndrome).

### Examples 53-61: Effects of thymidine derivative or a combination of thymidine derivative and uridine derivative for relieving 5-FU-induced diarrhea in a mouse model

Male Balb/c mice (7-8 weeks, 23-25 g) were i.p. injected at a dose of 70 mg/kg/d, and the injection was performed for three days. The mice in the group of blank was injected with 0.9% NaCl, and evaluated for the effect by observing the change of diarrhea grade, food uptake, and weight change. The severity of diarrhea was scored in accordance with the following standards: 0: normal feces, 1: soft feces, 2: slightly wet, soft feces, 3: wet, unformed feces, with moderate coloration in perianal region, 4: watery feces with heavy coloration of hair in perianal region. The severity of diarrhea was evaluated by occurrence scores of various grades of diarrhea (Scores 0 to 4) and average score of diarrhea. It was finally found that all the established diarrhea models were scored around 3. The models were stable, and satisfied the experimental requirements.

The male Balb/c mice (7-8 weeks, 23-25 g) were fed and adapted for 1 week, and then divided into groups, with 10 mice in each group for administration test. 5-FU was dissolved in a 0.9%NaCl solution, and the mice in the model group and the treatment group were all i.p. injected at a dose of 70mg/kg/d. The administration doses are shown in Table 6, and the modeling was completed in three days. While modeling of injection administration of 5-FU, the thymidine derivative or the combination of thymidine derivative and uridine derivative was administered by gavage, a series of compounds were dissolved in a PBS solution containing 5%DMSO+1%HPMC. The administration frequency and dose of the selected compounds are shown in Table 6. The mice were daily observed for counting the diarrhea status, food uptake, weight change (Δ%BW) (the weight changes recorded in the table is the value on Day 9) and other information. After 12 days, the experiments were completed and statistically analyzed for the results.

**Table 6 Statistical table of diarrhea status, food uptake, survival rate, and weight change (Δ%BW) of mice after administration of thymidine derivative or combination of thymidine derivative and uridine derivative**

| Exam ples | Drug | Dose | Compou nds to test | Administr ation Dose | Exper iment al Time | Avera ge Diarr hea Grade | Survi val Rate | Foo d Upt ake | Δ%BW On Day 9 |
|---|---|---|---|---|---|---|---|---|---|
| 53 | 5-FU | 70 mg/kg /d, injecti on was lasted for three days | Blank | 200 µl/20g | 12 | 2.4 | 70% | 74 | 29.4%±1.2 |
| 54 | | | T1 | 37 mpk, BID, PO | 12 | 1.4 | 100% | 155 | 4.3%±3.7** |
| 55 | | | T1 | 75 mpk, BID, PO | 12 | 1.3 | 80% | 100 | 3.8%±1.6** |
| 56 | | | T1 | 150 mpk, BID, PO | 12 | 1.5 | 80% | 89 | 16.3%±6.5** |
| 57 | | | T2 | 75 mpk, BID, PO | 12 | 1.4 | 100% | 123 | 6.6%±3.1** |
| 58 | | | T3 | 50 mpk, BID, PO | 12 | 1.6 | 70% | 109 | 8.4%±3.2** |
| 59 | | | T1+U1 | 75 mpk+37 mpk, BID, PO | 12 | 1.6 | 100% | 159 | 9.2%±5.9** |
| 60 | | | T1+U1 | 150 mpk+70 mpk, BID, PO | 12 | 1.3 | 80% | 113 | 15.7%±3.2 |
| 61 | | | T2+U3 | 75 mpk+37 mpk, BID, PO | 12 | 1.5 | 80% | 96 | 13.9%±2.8** |

Δ%BW = (BW (day0) -BW (day9))BW (day0). The results indicate the averages of diarrhea, survival number, food uptake, and body weight (BW) ± SEM, wherein *P < 0.05, **P < 0.01, ***P < 0.001; that is, the mice in the treatment group are compared with those in the model group. Since death of animals will successively occur after Day 9, so that the data are not stable, the weight change is analyzed on Day 9.

The results showed that the thymidine derivative alone or the combination of thymidine derivative and uridine derivative can both effectively decrease the diarrhea grade caused by 5-FU drug, while the survival status and food uptake of the mice in the administration group are also improved dramatically compared with those in the model group. It can be seen from the data that the oral administration of thymidine derivative alone or the combination of thymidine derivative and uridine derivative can obviously ameliorate the weight change of mice.

After completion of the experiments, the mice in the 5-FU control group and the experimental group were subject to blood collection at orbital. Blood cells were counted. It is found that 5-FU can reduce all the blood cells that could be detected, but the treatment of the thymidine derivative or the combination of thymidine derivative and uridine derivative can substantially increase the neutrophilic granulocytes and blood platelets in animals as compared with the group in which 5-FU is administered alone.

### Example 62: Effect of thymidine derivative on therapeutical effect of capecitabine

ABALB/C nude mouse (human colon cancer cell HCT116 xenograft) model was established. After the model was stable, the model mice were divided into four groups (in which the average tumor size of each of the 4 groups was kept as consistent as possible). Except the blank group (containing 5 mice), there were 10 mice in each group, which were subject to gavage and application experiments.

Capecitabine was dissolved in a mixed solution of castor oil: ethanol = 1: 1 (volume ratio). Prior to gavage, the mixture was diluted with PBS to the desired concentration (diluted for about 3 times with PBS solution). The gavage volume did not exceed 0.2mL. The mice underwent gavage administration for 5 days a week, and the administration dose gradually increased. In addition to the group of blank, other three groups of tumor-bearing mice were given capecitabine orally to control or reduce tumors. At the same time, a gel comprising the derivative as primary ingredient was applied onto the back of mice via transdermal administration. It was particularly performed as follows:
A: Group of blank: 5 mice with tumors, no gavage and no drug application; B: blank group: 10 mice with tumors, oral gavage with capecitabine (1.5 mmol/kg), application with blank gel on the back (once per day for 14 days); C: 0.5% T1 group: 10 mice with tumors, oral gavage of capecitabine, application with 0.5% T1 gel (administration method and frequency are the same as those in group B); D: 2% T1 gel group: 10 mice with tumors, oral gavage of capecitabine, application of 2% T1 gel (administration method and frequency are the same as those in group B). An application area of about 5.8 cm² was marked with a marker. Moreover, this application area cannot be an area that can be touched by the mouth of the mice, nor can it be an area immediately adjacent to the tumor. After the daily gavage of the Experimental Groups B, C, and D, the corresponding ointments were applied with cotton swabs on the marked areas on the back of the model mouse, ensuring that the ointment was applied evenly and the skin was hydrated. After application, each mouse was held in a relatively independent space for 4 hours to ensure the transdermal absorption of the drug applied onto the back. After 4 hours, the residual ointment was gently wiped off with paper towel or a water-soaked paper towel from the back of the mice; and then the mice were allowed back to the previous feeding cage for normal activities. The tumor size was measured and recorded every 2 days. After 14 days of the experiment, the mice were dissected to take out the tumor, which was weighed and recorded. Various experimental groups were observed for the changes of tumor volumes.

The results showed that the volume of tumor tissue in Groups B, C, and D (Group with capecitabine gavage) was significantly smaller than that in Group A (Group without capecitabine gavage); the tumor volume of Group of T1 gel (Groups C, D) was close to or slightly smaller than that of Group of blank gel (Group B). It can be seen that the gel of the thymidine derivative will not affect the therapeutic effect of capecitabine on tumors.

### Examples 63-90: Effect of thymidine derivative or a combination of thymidine derivative and uridine derivative on relieving proliferative toxicity of chemotherapeutical drug to HaCaT cells

The effect of thymidine derivative or a combination of thymidine derivative and uridine derivative on relieving the proliferative toxicity of a chemotherapeutical drug (5-FU) to HaCaT cells was tested according to the method in Examples 2-29. The test thymidine derivative or the combination of thymidine derivative and uridine derivative, as well as the final concentration of the test compounds were shown in Table 7.

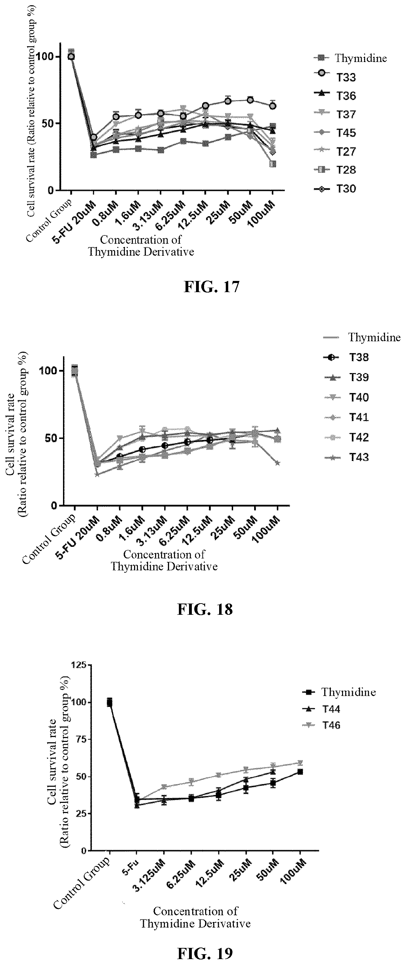

Table 7 lists the combinations of 5-FU and thymidine derivative (or the mixed solution of thymidine derivative + uridine derivative), and the corresponding experimental results (wherein the data in the column of cell survival indicate the corresponding increased percentage of the cell survivals of Group of 5-FU + thymidine derivative or Group of 5-FU + the combination of thymidine derivative and uridine derivative compared with the Group of 5-FU). FIGS. 15-21 list the experimental results of several exemplary compounds.

**Table 7 Experimental Conditions and Results of Examples 63-90**

| Example No. | Chemotherapeutical drug | Final Concentration | Selected Compound | Final concentration (µM) | Cell Survival Rate |
|---|---|---|---|---|---|
| 63 | 5-Fu | 20 µM | T31 | 0.8 | 23%-30% |
| | 5-Fu | 20 µM | | 1.6 | 29%-38% |
| | 5-Fu | 20 µM | | 3.13 | 37%-43% |
| | 5-Fu | 20 µM | | 6.25 | 41%-47% |
| | 5-Fu | 20 µM | | 12.5 | 44%-51% |
| 64 | 5-Fu | 20 µM | T29 | 0.8 | 19%-25% |
| | 5-Fu | 20 µM | | 1.6 | 19%-27% |
| | 5-Fu | 20 µM | | 3.13 | 35%-42% |
| | 5-Fu | 20 µM | | 6.25 | 43%-50% |
| | 5-Fu | 20 µM | | 12.5 | 69%-76% |
| | 5-Fu | 20 µM | | 25 | 54%-62% |
| | 5-Fu | 20 µM | | 50 | 48%-54% |
| 65 | 5-Fu | 20 µM | T32 | 3.13 | 35%-41% |
| | 5-Fu | 20 µM | | 6.25 | 29%-36% |
| | 5-Fu | 20 µM | | 12.5 | 46%-52% |
| | 5-Fu | 20 µM | | 25 | 53%-60% |
| | 5-Fu | 20 µM | | 50 | 59%-66% |
| | 5-Fu | 20 µM | | 100 | 78%-85% |
| 66 | 5-Fu | 20 µM | T34 | 12.5 | 28%-35% |
| | 5-Fu | 20 µM | | 25 | 43%-51% |
| | 5-Fu | 20 µM | | 50 | 69%-77% |
| | 5-Fu | 20 µM | | 100 | 67%-74% |
| 67 | 5-Fu | 20 µM | T35 | 0.8 | 23%-31% |
| | 5-Fu | 20 µM | | 1.6 | 25%-33% |
| | 5-Fu | 20 µM | | 3.13 | 42%-48% |
| | 5-Fu | 20 µM | | 6.25 | 64%-70% |
| | 5-Fu | 20 µM | | 12.5 | 95%-101% |
| | 5-Fu | 20 µM | | 25 | 84%-91 % |
| | 5-Fu | 20 µM | | 50 | 85%-92% |
| | 5-Fu | 20 µM | | 100 | 39%-47% |
| 68 | 5-Fu | 20 µM | T48 | 0.8 | 26%-34% |
| | 5-Fu | 20 µM | | 1.6 | 38%-46% |
| | 5-Fu | 20 µM | | 3.13 | 41%-48% |
| | 5-Fu | 20 µM | | 6.25 | 66%-73% |
| | 5-Fu | 20 µM | | 12.5 | 68%-76% |
| | 5-Fu | 20 µM | | 25 | 77%-85% |
| | 5-Fu | 20 µM | | 50 | 69%-76% |
| 69 | 5-Fu | 20 µM | T49 | 0.8 | 35%-42% |
| | 5-Fu | 20 µM | | 1.6 | 56%-63% |
| | 5-Fu | 20 µM | | 3.13 | 72%-79% |
| | 5-Fu | 20 µM | | 6.25 | 68%-74% |
| | 5-Fu | 20 µM | | 12.5 | 36%-44% |
| 70 | 5-Fu | 20 µM | T33 | 0.8 | 36%-42% |
| | 5-Fu | 20 µM | | 1.6 | 37%-44% |
| | 5-Fu | 20 µM | | 3.13 | 39%-49% |
| | 5-Fu | 20 µM | | 6.25 | 36%-42% |
| | 5-Fu | 20 µM | | 12.5 | 55%-63% |
| | 5-Fu | 20 µM | | 25 | 64%-71% |
| | 5-Fu | 20 µM | | 50 | 66%-73% |
| | 5-Fu | 20 µM | | 100 | 55%-63% |
| 71 | 5-Fu | 20 µM | T36 | 3.13 | 27%-35% |
| | 5-Fu | 20 µM | | 6.25 | 38%-46% |
| | 5-Fu | 20 µM | | 12.5 | 52%-60% |
| | 5-Fu | 20 µM | | 25 | 53%-61% |
| | 5-Fu | 20 µM | | 50 | 49% -57% |
| 72 | 5-Fu | 20 µM | T37 | 0.8 | 36%-44% |
| | 5-Fu | 20 µM | | 1.6 | 55%-62% |
| | 5-Fu | 20 µM | | 3.13 | 61%-69% |
| | 5-Fu | 20 µM | | 6.25 | 68%-76% |
| | 5-Fu | 20 µM | | 12.5 | 52%-60% |
| | 5-Fu | 20 µM | | 25 | 49%-57% |
| | 5-Fu | 20 µM | | 50 | 52%-59% |
| 73 | 5-Fu | 20 µM | T45 | 0.8 | 34%-41% |
| | 5-Fu | 20 µM | | 1.6 | 39%-46% |
| | 5-Fu | 20 µM | | 3.13 | 63%-70% |
| | 5-Fu | 20 µM | | 6.25 | 59%-67% |
| | 5-Fu | 20 µM | | 12.5 | 48%-55% |
| | 5-Fu | 20 µM | | 25 | 44%-51 % |
| | 5-Fu | 20 µM | | 50 | 39%-46% |
| 74 | 5-Fu | 20 µM | T27 | 1.6 | 39%-45% |
| | 5-Fu | 20 µM | | 3.13 | 51%-58% |
| | 5-Fu | 20 µM | | 6.25 | 54%-61 % |
| | 5-Fu | 20 µM | | 12.5 | 51%-59% |
| | 5-Fu | 20 µM | | 25 | 49%-57% |
| | 5-Fu | 20 µM | | 50 | 46%-53% |
| 75 | 5-Fu | 20 µM | T28 | 3.13 | 46%-53% |
| | 5-Fu | 20 µM | | 6.25 | 49%-56% |
| | 5-Fu | 20 µM | | 12.5 | 39%-47% |
| | 5-Fu | 20 µM | | 25 | 43%-49% |
| 76 | 5-Fu | 20 µM | T30 | 3.13 | 39%-47% |
| | 5-Fu | 20 µM | | 6.25 | 45%-53% |
| | 5-Fu | 20 µM | | 12.5 | 54%-61 % |
| | 5-Fu | 20 µM | | 25 | 44%-51 % |
| | 5-Fu | 20 µM | | 50 | 39%-46% |
| 77 | 5-Fu | 20 µM | T44 | 6.25 | 13%-20% |
| | 5-Fu | 20 µM | | 12.5 | 27%-35% |
| | 5-Fu | 20 µM | | 25 | 53%-60% |
| | 5-Fu | 20 µM | | 50 | 69%-76% |
| 78 | 5-Fu | 20 µM | T46 | 3.13 | 27%-34% |
| | 5-Fu | 20 µM | | 6.25 | 34%-41% |
| | 5-Fu | 20 µM | | 12.5 | 48%-55% |
| | 5-Fu | 20 µM | | 25 | 61%-68% |
| | 5-Fu | 20 µM | | 50 | 65%-72% |
| | 5-Fu | 20 µM | | 100 | 75%-83% |
| | 5-Fu | 20 µM | | 200 | 54%-62% |
| 79 | 5-Fu | 20 µM | T38 | 0.8 | 13%-21% |
| | 5-Fu | 20 µM | | 1.6 | 29%-38% |
| | 5-Fu | 20 µM | | 3.13 | 38%-47% |
| | 5-Fu | 20 µM | | 6.25 | 46%-55% |
| | 5-Fu | 20 µM | | 12.5 | 53%-60% |
| | 5-Fu | 20 µM | | 25 | 58%-65% |
| | 5-Fu | 20 µM | | 50 | 67%-75% |
| | 5-Fu | 20 µM | | 100 | 55%-63% |
| 80 | 5-Fu | 20 µM | T39 | 0.8 | 36%-43% |
| | 5-Fu | 20 µM | | 1.6 | 58%-66% |
| | 5-Fu | 20 µM | | 3.13 | 64%-71% |
| | 5-Fu | 20 µM | | 6.25 | 67%-74% |
| | 5-Fu | 20 µM | | 12.5 | 65%-72% |
| | 5-Fu | 20 µM | | 25 | 69%-77% |
| | 5-Fu | 20 µM | | 50 | 69%-77% |
| | 5-Fu | 20 µM | | 100 | 75%-83% |
| 81 | 5-Fu | 20 µM | T40 | 0.8 | 42%-50% |
| | 5-Fu | 20 µM | | 1.6 | 57%-65% |
| | 5-Fu | 20 µM | | 3.13 | 45%-52% |
| | 5-Fu | 20 µM | | 6.25 | 47%-56% |
| | 5-Fu | 20 µM | | 12.5 | 49%-54% |
| | 5-Fu | 20 µM | | 25 | 58%-65% |
| | 5-Fu | 20 µM | | 50 | 48%-55% |
| 82 | 5-Fu | 20 µM | T41 | 12.5 | 39%-46% |
| | 5-Fu | 20 µM | | 25 | 47%-55% |
| | 5-Fu | 20 µM | | 50 | 44%-51% |
| 83 | 5-Fu | 20 µM | T42 | 0.8 | 38%-45% |
| | 5-Fu | 20 µM | | 1.6 | 59%-67% |
| | 5-Fu | 20 µM | | 3.13 | 85%-93% |
| | 5-Fu | 20 µM | | 6.25 | 85%-92% |
| | 5-Fu | 20 µM | | 12.5 | 58%-66% |
| | 5-Fu | 20 µM | | 25 | 68%-75% |
| | 5-Fu | 20 µM | | 50 | 66%-74% |
| 84 | 5-Fu | 20 µM | T43 | 0.8 | 23%-31% |
| | 5-Fu | 20 µM | | 1.6 | 49%-56% |
| | 5-Fu | 20 µM | | 3.13 | 72%-79% |
| | 5-Fu | 20 µM | | 6.25 | 91%-98% |
| | 5-Fu | 20 µM | | 12.5 | 125%-133% |
| | 5-Fu | 20 µM | | 25 | 99%-106% |
| | 5-Fu | 20 µM | | 50 | 100%-107% |
| | 5-Fu | 20 µM | | 100 | 34%-41% |
| 85 | 5-Fu | 20 µM | T47 | 8 | 21%-28% |
| | 5-Fu | 20 µM | | 16 | 35%-42% |
| | 5-Fu | 20 µM | | 32 | 49%-56% |
| | 5-Fu | 20 µM | | 62.5 | 63%-71% |
| | 5-Fu | 20 µM | | 125 | 64%-72% |
| | 5-Fu | 20 µM | | 250 | 44%-51% |
| | 5-Fu | 20 µM | | 500 | 24%-31% |
| 86 | 5-Fu | 20 µM | T50 | 2 | 13%-20% |
| | 5-Fu | 20 µM | | 4 | 26%-33% |
| | 5-Fu | 20 µM | | 8 | 36%-43% |
| | 5-Fu | 20 µM | | 16 | 47%-53% |
| | 5-Fu | 20 µM | | 32 | 68%-76% |
| | 5-Fu | 20 µM | | 62.5 | 74%-82% |
| | 5-Fu | 20 µM | | 125 | 75%-85% |
| | 5-Fu | 20 µM | | 250 | 75%-82% |
| | 5-Fu | 20 µM | | 500 | 65%-73% |
| | 5-Fu | 20 µM | | 1000 | 58%-65% |
| 87 | 5-Fu | 20 µM | T33 + U14 | 0.8+6.25 | 63%-69% |
| | 5-Fu | 20 µM | | 1.6+6.25 | 65%-70% |
| | 5-Fu | 20 µM | | 3.13+6.25 | 89%-96% |
| | 5-Fu | 20 µM | | 6.25+6.25 | 86%-91% |
| | 5-Fu | 20 µM | | 12.5+6.25 | 79%-85% |
| | 5-Fu | 20 µM | | 25+6.25 | 103%-111% |
| | 5-Fu | 20 µM | | 50+6.25 | 123%-130% |
| 88 | 5-Fu | 20 µM | T33 + U21 | 0.8+12.5 | 65%-71% |
| | 5-Fu | 20 µM | | 1.6+12.5 | 69%-78% |
| | 5-Fu | 20 µM | | 3.13+12.5 | 82%-90% |
| | 5-Fu | 20 µM | | 6.25+12.5 | 88%-95% |
| | 5-Fu | 20 µM | | 12.5+12.5 | 97%-105% |
| | 5-Fu | 20 µM | | 25+12.5 | 104%-112% |
| 89 | 5-Fu | 20 µM | T26 + U21 | 0.8+12.5 | 58%-66% |
| | 5-Fu | 20 µM | | 1.6+12.5 | 66%-75% |
| | 5-Fu | 20 µM | | 3.13+12.5 | 67%-77% |
| | 5-Fu | 20 µM | | 6.25+12.5 | 73%-80% |
| | 5-Fu | 20 µM | | 12.5+12.5 | 80%-89% |
| | 5-Fu | 20 µM | | 25+12.5 | 83%-91% |
| 90 | 5-Fu | 20 µM | T26 + U14 | 0.8+6.25 | 67%-75% |
| | 5-Fu | 20 µM | | 1.6+6.25 | 77%-85% |
| | 5-Fu | 20 µM | | 3.13+6.25 | 85%-94% |
| | 5-Fu | 20 µM | | 6.25+6.25 | 99%-107% |
| | 5-Fu | 20 µM | | 12.5+6.25 | 108%-116% |
| | 5-Fu | 20 µM | | 25+6.25 | 93%-99% |

The results from Table 7 showed that fluorinated drugs have certain proliferative toxicity to the skin cell HaCaTs, while the thymidine derivative or the combination of thymidine derivative and uridine derivative has a significant relieving effect on the proliferative toxicity caused by the fluorinated drugs. The addition of the thymidine derivative or the combination of thymidine derivative and uridine derivative increases the cell survival rate, and has a certain advantage over the thymidine. The NSAID-containing thymidine derivative and uridine derivative still have the effect of relieving the cytotoxicity caused by 5-FU, and their relieving effects were better than those of thymidine derivative and uridine derivative not containing NSAID: at the same concentration, the NSAID-containing thymidine derivative and uridine derivative have higher relieving rates on cells, or higher relieving rates can be achieved at lower concentration.

### Examples 91-117: Thymidine derivative or a combination of thymidine derivative and uridine derivative can prevent/treat hand-foot syndrome caused by a chemotherapeutical drug in a rat model

The effects of thymidine derivative or a combination of thymidine derivative and uridine derivative on the chemotherapeutic drug-induced hand-foot syndrome in a rat model were tested according to the method in Examples 30-40. At the same time, paws of rats after treatment with the drug were taken and observed for their inflammation by hematoxylin-eosin staining (HE) and immunohistochemical staining (IHC).

The test thymidine derivative or the combination of thymidine derivative and uridine derivative, as well as the final concentration of the test compounds were shown in Table 8. Table 8 lists the animal experiment combination of a chemotherapeutical drug and the thymidine derivative gel (or the compound gel of thymidine derivative and uridine derivative) and the corresponding experimental results (wherein, the data in the column of control rate = the occurrence rate of hand-foot syndrome in Group of blank - the occurrence rate of hand-foot syndrome in Group of administration).

**Table 8 Experimental Conditions and Results of Examples 91-117**

| Exam ple No. | Chemotherapeu tical drug | Dose | Frequen cy | Applicati on | Concentrat ion wt% | Contro 1 | Da ys | Control Rate | |
|---|---|---|---|---|---|---|---|---|---|
| | | | | | | | | Forepa ws | Hind Paws |
| 91 | Capecitabine | 4000 mg/kg | Once daily | T25 | 0.10% | Betwe en groups | 40 | 22.22% | 33.33 % |
| | | | | | 0.50% | Betwe en groups | 40 | 22.22% | 44.44 % |
| | | | | | 1% | Betwe en groups | 40 | 37.50% | 50.00 % |
| | | | | | 3% | Betwe en groups | 40 | 62.50% | 75.00 % |
| | | | | | 5% | Betwe en groups | 40 | 57.14% | 71.43 % |
| 92 | | | | T27 | 0.10% | Betwe en groups | 40 | 22.22% | 44.44 % |
| | | | | | 0.50% | Betwe en groups | 40 | 33.33% | 33.33 % |
| | | | | | 1% | Betwe en groups | 40 | 25% | 50% |
| | | | | | 3% | Betwe en groups | 40 | 57.14% | 71.43 % |
| | | | | | 5% | Betwe en groups | 40 | 50% | 75.00 % |
| 93 | | | | T26 | 1% | Betwe en groups | 40 | 22.22% | 44.44 % |
| | | | | | 3% | Betwe en groups | 40 | 57.14% | 71.43 % |
| 94 | | | | T35 | 1% | Betwe en groups | 40 | 25% | 37.50 % |
| | | | | | 5% | Betwe en groups | 40 | 75.00% | 88% |
| 95 | | | | T50 | 3% | Betwe en groups | 40 | 71.43% | 57.14 % |
| 96 | | | | T36 | 1% | Betwe en groups | 40 | 28.57% | 42.86 % |
| | | | | | 3% | Betwe en groups | 40 | 44.44% | 44.44 % |
| 97 | | | | T32 | 3% | Betwe en groups | 40 | 37.50% | 62.50 % |
| 98 | | | | T37 | 3% | Betwe en groups | 40 | 28.57% | 42.86 % |
| 99 | | | | T40 | 3% | Betwe en groups | 40 | 44.44% | 66.67 % |
| 100 | | | | T41 | 3% | Betwe en groups | 40 | 50.00% | 62.50 % |
| | | | | | 5% | Betwe en groups | 40 | 50% | 75.00 % |
| 101 | | | | T45 | 1% | Betwe en groups | 40 | 25% | 37.50 % |
| | | | | | 5% | Betwe en groups | 40 | 55.56% | 77.78 % |
| 102 | | | | T25+U1 4 | 1%+3% | Betwe en groups | 40 | 57.14% | 42.86 % |
| 103 | | | | T25+U1 6 | 1%+3% | Betwe en groups | 40 | 33.33% | 33.33 % |
| | | | | | 3%+3% | Betwe en groups | 40 | 71.43% | 57.14 % |
| 104 | | | | T26+U1 8 | 1%+3% | Betwe en groups | 40 | 37.50% | 62.50 % |
| 105 | | | | T45+U2 0 | 1%+3% | Betwe en groups | 40 | 25% | 37.50 % |
| | | | | | 3%+5% | Betwe en groups | 40 | 44.44% | 66.67 % |
| 106 | | | | T1 | 3% | Betwe en groups | 40 | 57.14% | 71.43 % |
| 107 | | | | T4 | 3% | Betwe en groups | 40 | 28.57% | 42.86 % |
| 108 | Cytarabine | 120 mg/kg | Twice per day | T25 | 3% | Betwe en groups | 30 | 42.86% | 57.14 % |
| 109 | | | | T32 | 2% | Betwe en groups | 30 | 33.33% | 33.33 % |
| 110 | | | | T37 | 3% | Betwe en groups | 30 | 22.22% | 44.44 % |
| 111 | Doxorubicin | 7 mg/kg, tail intravenou s injection | Once every three days, for 20 days | T25 | 3% | Betwe en groups | 30 | 37.50% | 62.50 % |
| 112 | | | | T41 | 3% | Betwe en groups | 30 | 57.14% | 42.86 % |
| 113 | Acelarin (NUC-1031) | 130 mg/kg, intraperito neal injection | Twice a week | T25 | 3% | Betwe en groups | 30 | 57.14% | 42.86 % |
| 114 | | | | T32 | 3% | Betwe en groups | 30 | 50% | 62.50 % |
| 115 | | | | T37 | 3% | Betwe en groups | 30 | 42.86% | 71.43 % |
| 116 | | | | T40 | 3% | Betwe en groups | 30 | 50% | 62.50 % |
| 117 | | | | T26+U1 8 | 1%+3% | Betwe en groups | 30 | 37.50% | 62.50 % |

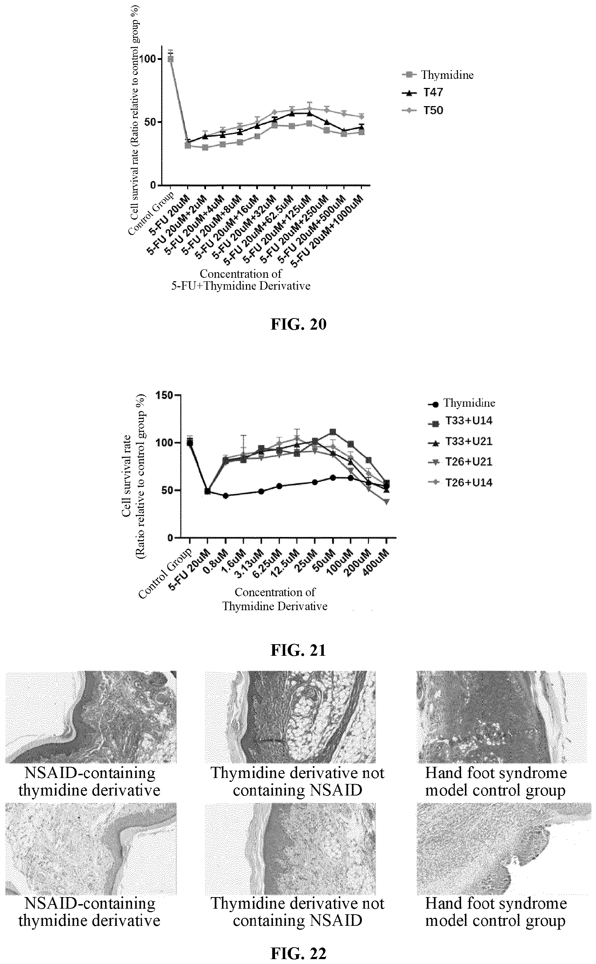

The results showed that (Table 8), different concentrations or different types of thymidine derivative and a combination of thymidine derivative and uridine derivative can all relieve the hand-foot syndrome to an extent, indicating that the NSAID-containing thymidine derivative and uridine derivative still have the effect of relieving the chemotherapeutic drug-induced hand-foot syndrome. FIG. 22 shows a representative staining diagram of a thymidine derivative containing NSAID and a thymidine derivative not containing NSAID, with the results showing that the effect of an NSAID-containing thymidine derivative (e.g., a compound of T25 to T50) or a combination of an NSAID-containing thymidine derivative and an NSAID-containing uridine derivative on alleviating the inflammation of rat paws was superior to those in the hand-foot syndrome model group of thymidine derivative not containing NSAID (e.g., a compound of T1 to T24) and the control hand-foot syndrome model group, indicating that the NSAID-containing thymidine derivative can produce the dual effects of NSAID and thymidine derivative, with more obvious advantage in terms of treating and preventing the hand-foot syndrome.

### Example 118: NSAID-containing thymidine derivative and/or uridine derivative alleviates pain of hand-foot syndrome caused by chemotherapeutical drug

According to the method of Examples 91-115, after administration of a thymidine derivative (at a concentration of 3%) and co-administration of a thymidine derivative (at a concentration of 1%) with a uridine derivative (at a concentration of 3%) to rat hand-foot syndrome models constructed from 4000 mg/kg of capecitabine for a period of time, pain analysis was performed on the rats, and the pain assessment model was mechanical sensitivity in rats (von Frey). The experimental steps were as follows: first, the rats were allowed to adapt in the room for 1 hour, then the rats were placed in an observation box with a metal mesh floor, and the mice were allowed to stay in the box for 20 minutes to adapt to the experimental platform. Next, a von Frey device was used to detect paw pain by stimulating the surface of the rat's palm with specially designed cilia to detect the mechanical sensitivity of the animal at strength of 0.4 g, 0.8 g, 1.5 g, 2.5 g, 4 g, 8 g, 10 g and 20 g (IITC Life Science, Woodland Hills, 2390 series). Rats were defined as responsive if they withdrew their paws or licked immediately after being applied a specific pressure, and as unresponsive if they did not withdraw their paws within 6 seconds. The rat movement response was considered to be a vague response, in which case the stimulation experiment was repeated.

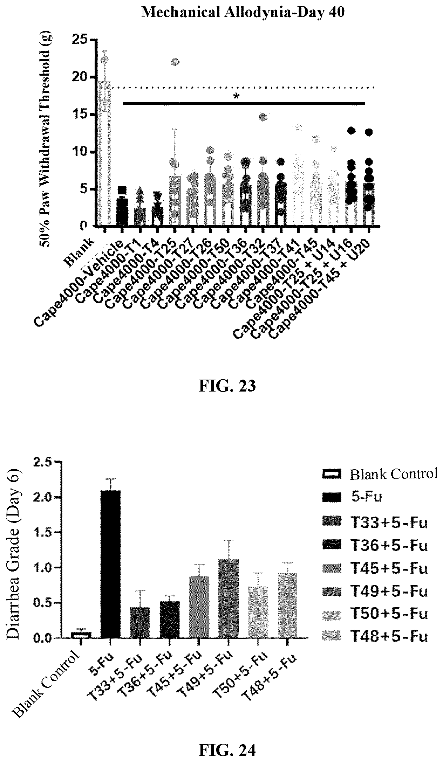

It can be seen from the results in FIG. 23 that the thymidine derivative not containing NSAID (T1 and T4, both at a concentration of 3%) did not significantly improve pain in rats; while the NSAID-containing thymidine derivative (T25, T26, T27, T50, T36, T32, T37, T41, T45, all at a concentration of 3%) and the combination of the NSAID-containing thymidine derivative and the NSAID-containing uridine derivative (T25+U14, T25+U16 and T45+U20, all at concentration of 1% + 3%) could significantly improve pain in rats. In FIG. 23, Cape4000 represents 4000 mg/kg of capecitabine, the concentration of thymidine derivative was 3% when used alone, and when thymidine derivative was used in combination with uridine derivative, the concentration of the thymidine derivative was 1%, and the concentration of the uridine derivative was 3%.

### Examples 119-123: Thymidine derivative or a combination of thymidine derivative and uridine derivative can prevent/treat hand-foot syndrome caused by a chemotherapeutic drug in a mouse model.

The effect of thymidine derivative or a combination of thymidine derivative and uridine derivative on a mouse model with chemotherapeutic drug (capecitabine)-induced hand-foot syndrome was tested according to the method in Examples 41-52. Wherein, the drugs were administered transdermally for Examples 119-120 and orally for Examples 121-123.

The test thymidine derivative or the combination of thymidine derivative and uridine derivative, as well as the final concentration of the test compounds were shown in Table 9. Table 9 lists the combinations of animal experiments of capecitabine and the thymidine derivative gel (or a compounded gel of thymidine derivative and uridine derivative) and the corresponding experimental results (wherein the data in the column of control rate = the occurrence rate of hand-foot syndrome in Group of blank - the occurrence rate of hand-foot syndrome in Group of administration).

**Table 9 Experimental Conditions and Results of Examples 119-123**

| Examp le No. | Chemotherapeuti cal Drug | Dose | Frequen cy | Applicati on | Concentrati on | Contro l | Day s | Control Rate | |
|---|---|---|---|---|---|---|---|---|---|
| | | | | | | | | Forepa ws | Hind Paws |
| 119 | Capecitabine | 1000 mg/k g | Once daily | T25 | 1% (wt%) | Betwee n groups | 15 | 22.22% | 44.44 % |
| | | | | | 3% (wt%) | Betwee n groups | 15 | 25% | 37.50 % |
| | | | | | 5% (wt%) | Betwee n groups | 15 | 44.44% | 66.67 % |
| 120 | | | | T27 | 1% (wt%) | Betwee n groups | 15 | 50% | 62.50 % |
| | | | | | 3% (wt%) | Betwee n groups | 15 | 42.86% | 71.43 % |
| 121 | | | | T32 | 300 mpk, twice per day | Betwee n groups | 8 | 71.43% | 57.14 % |
| 122 | | | | T40 | 200 mpk, twice per day | Betwee n groups | 8 | 75.00% | 88% |
| 123 | | | | T45 | 250 mpk, twice per day | Betwee n groups | 8 | 57.14% | 42.86 % |

It can be seen from the results in Table 9 that different concentrations or different types of the thymidine derivative or a combination of thymidine derivative and uridine derivative can both relieve the hand-foot syndrome to an extent.

### Examples 124-131: Effects of thymidine derivative or a combination of thymidine derivative and uridine derivative for relieving 5-FU-induced diarrhea in a mouse model

7-8-week-old male Balb/c mice were used to establish a 5-FU induced diarrhea model to test the effect of the thymidine derivative or the combination of thymidine derivative and uridine derivative on the relief of diarrhea.

Modeling dose of 5-Fu:175 mg/kg; single administration, intraperitoneal injection (ip.);
Administration mode of the compound to test (thymidine derivative or combination of thymidine derivative and uridine derivative): single administration, by gavage, advance administration of the compound to test at various time points prior to intraperitoneal injection of 5-FU. The compound to test was administered 1 h prior to the administration of 5-FU, a certain amount of 5-FU was injected intraperitoneally 1 h later, and the compound to test was administered once again 7 h after the injection of 5-FU.

The mice were fed and adapted for one week, and then divided into groups, with 10 mice in each group for administration test.

The mice in the Group of blank was injected with 0.9% NaCl, and evaluated the effect by observing the change of diarrhea grade, food uptake, and weight change. The severity of diarrhea was scored in accordance with the following standards: 0: normal feces, 1: soft feces, 2: slightly wet, soft feces, 3: wet, unformed feces, with moderate coloration in perianal region, 4: watery feces with heavy coloration of hair in perianal region. The severity of diarrhea was evaluated by occurrence scores of various grades of diarrhea (Scores 0 to 4) and average score of diarrhea. It was finally found that all the established diarrhea models were scored around 3. The models were stable, and satisfied the experimental requirements.

5-FU was dissolved in a 0.9% NaCl solution, the mice in the model group and the treatment group were all i.p. injected at a dose of 175 mg/kg/d, and the modeling was completed in three days. While modeling of injection administration of 5-FU, the thymidine derivative or the combination of thymidine derivative and uridine derivative was administered by gavage, and the compounds to test were dissolved in a PBS solution containing 5%DMSO+1%HPMC. The administration frequency and dose of the selected compounds are shown in Table 10. Body weight was weighed daily to count the weight change (Δ%BW, the weight changes recorded in the table were the values on Day 6), and the food was weighed daily to calculate the food uptake. The mice were daily observed for their clinical status, e.g., activity, diarrhea, hair, fighting, etc. After 6 days, the experiments were completed and statistically analyzed for the results.

**Table 10 Statistical table of diarrhea status, food uptake, survival rate, and weight change (Δ%BW) of mice after administration of thymidine derivative or combination of thymidine derivative and uridine derivative**

| Exampl es | Dru g | Dose | Compoun ds to test | Administrati on Dose | Experimen tal Time (days) | Averag e Diarrh ea Grade | Surviv al Rate | Δ %B W (on Day 6) |
|---|---|---|---|---|---|---|---|---|
| 124 | 5-FU | 175 mg/kg; single administrati on, intraperiton eal injection | - | - | 6 | 3 | 100% | 4.96 % ± 0.74 % |
| 125 | | | T33 | 50 mpk (gavage) | 6 | 1 | 100% | 4.18 % ± 0.56 % |
| 126 | | | T36 | 50 mpk (gavage) | 6 | 1.6 | 100% | 2.25 % ± 0.54 % |
| 127 | | | T45 | 15 mpk (gavage) | 6 | 1.4 | 100% | 0.34 % ± 0.36 % |
| 128 | | | T49 | 15 mpk (gavage) | 6 | 0.75 | 80% | 1.97 % ± 0.67 % |
| 129 | | | T48 | 15 mpk (gavage) | 6 | 1.3 | 100% | 1.97 % ± 0.67 % |
| 130 | | | T50 | 50 mpk (gavage) | 6 | 1.1 | 100% | 2.08 % ± 0.86 % |
| 131 | | | T33+U19 | 50 mpk+80 mpk (gavage) | 6 | 1.25 | 80% | 1.92 % ± 0.81 % |

The results were shown in Table 10 and FIG. 24, wherein the concentration of each group of compounds in FIG. 24 corresponds to the concentration shown in Table 10. It can be seen from the results that (Table 10 and FIG. 25), the thymidine derivative alone or the combination of thymidine derivative and uridine derivative can both effectively decrease the diarrhea grade caused by 5-FU drug, while the survival status, food uptake and weight change of the mice in the administration group were also improved dramatically compared with those in the model group in which thymidine derivative and/or thymidine derivative were/was not administered.

### Example 132: Preparation of compounds

This example lists the preparation of several representative compounds in T25 to T50 and U14 to U21. Other compounds not listed are prepared using the corresponding reactants and under the same conditions.

### (1) Synthesis of ((2R, 3S, 5R)-3-hydroxyl-5-(5-methyl-2,4-dioxy-3,4-dihydropyrimidin-1(2H)-yl)tetrahydrofuran-2-yl)2-(3-phenoxyphenyl) propionate methyl (T27)

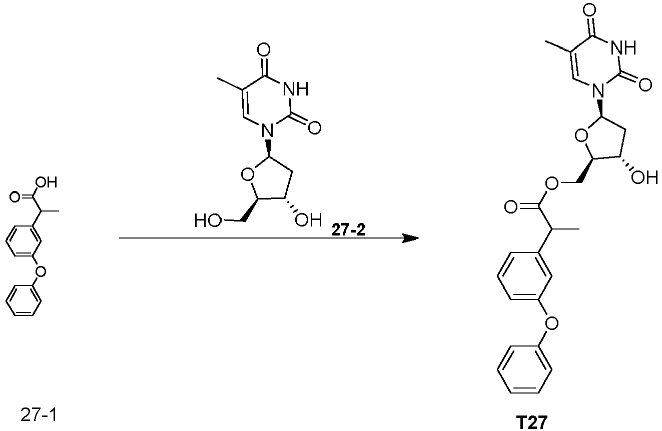

To a solution of 2-[3-(phenoxy) phenyl] propanoic acid (0.5 g, 2.1 mmol) in dichloromethane (10 mL) was added oxalyl chloride (310 mg, 2.5 mmol) dropwise and stirred at 25°C for 3 hours. The solvent was removed by distillation under reduced pressure to obtain a yellow solid. At 0°C, the yellow solid was added into a solution of β-thymidine (1.0 g, 4.2 mmol) in dichloromethane (10 mL) and pyridine (10 mL) and stirred for 1 hour, and further stirred at 25°C for 12 hours. The solvent was removed by rotary evaporation. Water (50 mL) was added, and the mixture was extracted with ethyl acetate (30 mL*3). The organic phase was collected, dried over anhydrous sodium sulfate, and filtered. The solvent was removed by rotary evaporation. Silica gel column chromatography (petroleum ether/ethyl acetate =5/1) was performed to obtain a crude product, which was further purified by preparative HPLC to obtain a compound ((2R, 3S, 5R)-3-hydroxyl-5-(5-methyl-2,4-dioxy-3,4-dihydropyrimidin-1(2H)-yl)tetrahydrofuran-2-yl)2-(3-phenoxyphenyl) propionate methyl (192.9 mg). LCMS: MS (ESI) m/z [M+H]⁺= 476.2. ¹H NMR (400 MHz, DMSO-*d₆*) *δ* 11.46 - 11.16 (m, 1H), 7.43 - 7.27 (m, 4H), 7.22-7.10 (m, 1H), 7.04 (t, *J* = 7.2 Hz, 1H), 7.05-6.90 (m, 3H), 6.96-6.80 (m, 1H), 6.24-6.09 (m, 1H), 4.28 - 4.13 (m, 3H), 4.08 - 4.01 (m, 1H), 3.94 - 3.80 (m, 3H), 2.13 - 1.99 (m, 1H), 1.75 (d, *J* = 12.0 Hz, 3H), 1.45-1.35 (m, 3H).

### (2) ((2R, 3S, 4R, 5R)-5-(2,4-dioxy-3,4-dihydropyrimidin-1(2H)-yl)-3,4-dihydroxyltetrahydrofuran-2-yl)methyl-2-(6-methoxynaphthalen-2-yl) propionate (U14)

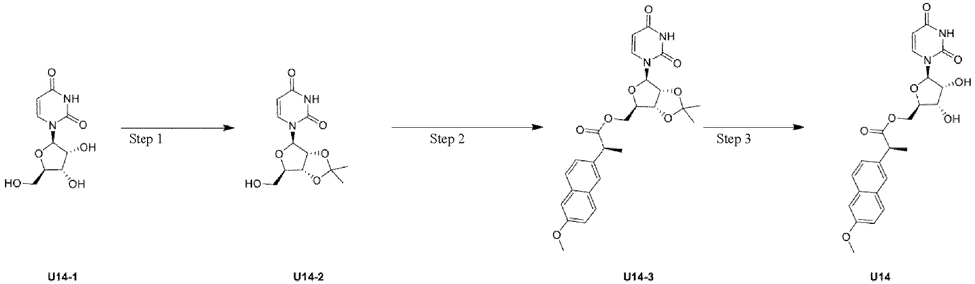

### Step 1. Synthesis of 1-((3aR, 4R, 6R, 6aR)-6-(hydroxymethyl)-2,2-dimethyl tetrahydrofuran[3,4-d][1,3]dioxol-4-yl) pyrimidine-2,4 (1H, 3H)-dione

At 0°C, to a solution of β-thymidine (750.0 g, 0.5 mol, 1.0 eq) and p-toluenesulfonic acid (52.9 g, 51.2 mmol, 0.1 eq) in acetone (18.0 L) was added 2,2-dimethoxy propane (352.0 g, 563.0 mmol, 68.9 mL, 1.1 eq). The mixture was stirred at 56°C for 1 hour. The reaction solution was cooled to room temperature, sodium bicarbonate (46.4 g, 92.1 mmol) was added, and stirred at 25°C for 0.5 hours. The mixture was concentrated under reduced pressure, and the crude product was purified by silica gel column chromatography (dichloromethane/methanol=10/1) to obtain 1-[(3aR, 4R, 6R, 6aR)-6-(hydroxymethyl)-2,2-dimethyl-3a,4,6,6a-tetrahydrofuran[3,4-d][1,3]dioxol-4-yl]pyrimidine-2,4-dione (800.0 g). LC-MS: (M+H)⁺, 284.9.

### Step 2. Synthesis of ((3aR,4R,6R,6aR)-6-(2,4-dioxy-3,4-dihydropyrimidin-1(2H)-yl)-2,2-dimethyl tetrahydrofuran[3,4-d] [1,3] dioxy-4-yl) methyl-2-(6-methoxynaphthalen-2-yl) propionate

At -30°C, to a solution of 1-[(3aR,4R,6R,6aR)-6-(hydroxymethyl)-2,2-dimethyl-3a,4,6,6a-tetrahydrofuran[3,4-d][1,3]dioxol-4-yl]pyrimidine-2,4-dione (188 g, 662 mmol, 1.0 eq) in pyridine (2.0 L) was added a solution of (2S)-2-(6-methoxy-2-naphthyl) propionyl chloride (168 g, 676 mmol, 1.02 eq) in dichloromethane (500 mL) dropwise. The reaction solution was stirred at -30°C for 4 hours, warmed to 25°C, quenched with water (10 mL), and concentrated under reduced pressure. The crude product was washed with ethyl acetate (400 mL) and aqueous hydrochloric acid solution (200 mL*3, 1 M), dried over anhydrous sodium sulfate, filtered, and concentrated under reduced pressure to obtain a crude product, which was recrystallized with isopropyl acetate (4.0 L) to obtain [(3aR,4R,6R,6aR)-4-(2,4-dioxopyrimidin-1-yl)-2,2-dimethyl-3a,4,6,6a-tetrahydrofuran [3,4-d] [1,3] dioxy-6-yl] methyl (2S)-2-(6-methoxy-2-naphthyl) propionate (160 g, 96.4% purity).

### Step 3. Synthesis of ((2R,3S,4R,5R)-5-(2,4-dioxy-3,4-dihydropyrimidin-1(2H)-yl)-3,4-dihydroxyltetrahydrofuran-2-yl) methyl-2-(6-methoxynaphthalen-2-yl) propionate

At 25°C, to a solution of [(3aR,4R,6R,6aR)-4-(2,4-dioxopyrimidin-1-yl)-2,2-dimethyl-3a,4,6,6a-tetrahydrofuran[3,4-d] [1,3]dioxy-6-yl]methyl (2S)-2-(6-methoxy-2-naphthyl) propionate (150 g, 302 mmol, 1.0 *eq*) in water (225 mL) was added trifluoroacetic acid (225 mL) and stirred at 25°C for 1 hour. The mixture was diluted with water (500 mL) and then filtered to obtain a solid crude product. The crude product was recrystallized with isopropyl acetate to obtain (2R,3S,4R,5R)-5-(2,4-dioxy-3,4-dihydropyrimidin-1(2H)-yl)-3,4-dihydroxyltetrahydrofuran-2-yl) methyl-2-(6-methoxynaphthalen-2-yl) propionate (95 g, 98.6% purity) as a white solid. LC-MS: (M+H)⁺, 457.1. ¹H NMR: (400 MHz, DMSO-d6) *δ* (ppm) 11.33 (d, *J* = 2.0 Hz, 1H), 7.77 (d, *J* = 8.0 Hz, 2H), 7.72 (s, 1H), 7.35-7.41 (m, 2H), 7.28 (d, *J* = 2.0 Hz, 1H), 7.20-7.10 (m, 1H), 5.80-5.58 (M, 1H), 5.62-5.47 (m, 1H), 4.41-4.20 (m, 2H), 3.94-4.00 (m, 2H), 3.84-3.89 (m, 4H), 3.79-3.82 (m, 1H), 1.48 (d, *J* = 8.0 Hz, 3H).

### (3) Synthesis of 2-ethylbutyl ((S)-(2R, 3S, 4R, 5R)-5-(2,4-dioxy-3,4-dihydropyrimidin-1(2H)-yl)-3,4-dihydroxytetrahydrofuran-2-yl)methoxy)(phenoxy)phosphoryl)-L-alaninate (U21)

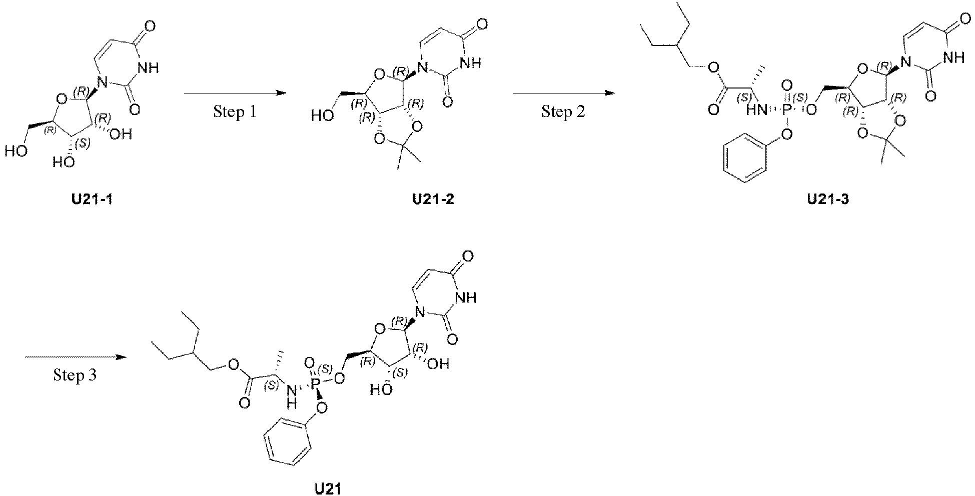

### Step 1. Synthesis of 1-((3aR,4R,6R,6aR)-6-(hydroxymethyl)-2,2-dimethyltetrahydrofuran[3,4-d] [1,3] dioxol-4-yl) pyrimidine-2,4(1H,3H)-dione

Into a solution of uracil-1-B-D-ribofuranoside (1.0 g, 4.1 mmol) in acetone (50 mL) was added sulfuric acid (0.5 mL) dropwise, and stirred at 25°C for 1 hour. The reaction solution was neutralized with triethylamine, and concentrated to obtain a crude product, which was purified by silica gel column chromatography (dichloromethane/methanol = 10/1) to obtain 1-((3aR,4R,6R,6aR)-6-(hydroxymethyl)-2,2-dimethyltetrahydrofuran[3,4-d][1,3]dioxol-4-yl)pyrimidine-2,4(1H,3H)-dione (1.2 g).

### Step 2. Synthesis of 2-ethylbutyl ((S)-(3aR,4R,6R,6aR)-6-(2,4-dioxy-3,4-dihydropyrimidin-1(2H)-yl)-2,2-dimethyltetrahydrofurfural[3,4-d][1,3]dioxy-4-yl)methoxy)(phenoxy)phosphoryl)-L-alaninate

At 25°C, to a solution of N-[(S)-(2,3,4,5,6-pentafluorophenoxy)phenoxyphosphoryl]-L-alaninate isopropyl (418 mg, 0.84 mmol, 1.2 eq) and 1-((3aR,4R,6R,6aR)-6-(hydroxymethyl)-2,2-dimethyltetrahydrofuran[3,4-d][1,3]dioxol-4-yl)pyrimidine-2,4(1H, 3H)-dione (200 mg, 0.70 mmol, 1.0 eq) in acetonitrile (20 mL) was added anhydrous magnesium chloride (67 mg, 0.70 mmol, 1.0 eq). The reaction solution was stirred at 50°C for ten minutes, into which was then dropwise added N,N-dimethylethylenediamine (227 mg, 1.76 mmol, 2.5 eq). The reaction solution was stirred at 50°C for 2 hours, quenched with water (50 mL), and extracted with ethyl acetate (30 mL*3). The organic phase was washed with saturated saline (20 mL), dried over anhydrous sodium sulfate, and filtered. The solvent was removed by rotary evaporation under reduced pressure. Silica gel column chromatography (dichloromethane/methanol=10/1) was performed for purification to obtain 2-ethylbutyl ((S)-(3aR,4R,6R,6aR)-6-(2,4-dioxy-3,4-dihydropyrimidin-1(2H)-yl)-2,2-dimethyltetrahydrofurfural[3,4-d] [1,3] dioxy-4-yl) methoxy) (phenoxy) phosphoryl)-L-alaninate (400 mg).

### Step 3. 2-ethylbutyl ((S)-(2R,3S,4R,SR)-5-(2,4-dioxy-3,4-dihydropyrimidin-1(2H)-yl)-3,4-dihydroxytetrahydrofuran-2-yl) methoxy)(phenoxy) phosphoryl)-L-alaninate

Into 2-ethylbutyl ((S)-(3aR,4R,6R,6aR)-6-(2,4-dioxy-3,4-dihydropyrimidin-1(2H)-yl)-2,2-dimethyltetrahydrofurfural[3,4-d] [1,3] dioxy-4-yl) methoxy)(phenoxy) phosphoryl)-L-alaninate (400 mg, 67.2 mmol, 1.0 eq) in water (1 mL) was added trifluoroacetic acid (4 mL). The reaction solution was stirred at 25°C for 2 hours, then concentrated under reduced pressure, and purified by preparative HPLC to obtain 2-ethylbutyl ((S)-(2R, 3S, 4R, 5R)-5-(2,4-dioxy-3,4-dihydropyrimidin-1(2H)-yl)-3,4-dihydroxytetrahydrofuran-2-yl) methoxy)(phenoxy) phosphoryl)-L-alaninate (99.8 mg). LCMS: MS (ESI) m/z [M+H]⁺= 556.1.¹H NMR (400 MHz, DMSO-*d₆*) *δ* 11.36 (d, *J* = 2.0 Hz, 1H), 7.58 (d, *J* = 8.0 Hz, 1H), 7.48 - 7.32 (m, 2H), 7.32-7.17 (m, 3H), 6.22-6.00 (m, 1H), 5.77 (d, *J* = 6.0 Hz, 1H), 5.59-5.48 (m, 1H), 5.47 (d, *J* = 6.0 Hz, 1H), 5.37-5.10 (m, 1H), 4.20 (m, 1H), 4.10 (m, 1H), 4.04 - 3.81 (m, 6H), 1.45 (m, 1H), 1.35 - 1.19 (m, 7H), 0.82 (t, *J* = 8.0 Hz, 6H).

### (4) Synthesis of 2-ethylbutyl ((S)-(2R,3S,5R)-3-hydroxyl-5-(5-methyl-2,4-dioxy-3,4-dihydropyrimidin-1(2H)-yl) tetrahydrofuran-2-yl)methoxy)(phenoxy) phosphoryl)-L-alaninate

### (T48) and 2-ethylbutyl ((S)-(2R,3S,5R)-2-(hydroxymethyl)-5-(5-methyl-2,4-dioxy-3,4-dihydropyrimidin-1(2H)-yl) tetrahydrofuran-3-yl)oxy)(phenoxy) phosphoryl)-L-alaninate (T49)

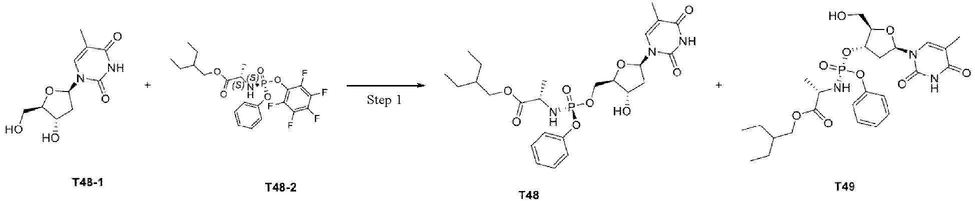

At 0°C, to a solution of β-thymidine (200 mg, 0.82 mmol, 1.0 eq) in DMF (10 mL) was added tert-butyl chlorinase (0.8 mL, 1 M) slowly. The reaction solution was stirred at 0°C for 0.5 hours, and stirred at room temperature for 30 minutes. After then, a solution of isopropyl N-[(S)-(2,3,4,5,6-pentafluorophenoxy)phenoxyphosphoryl]-L-alaninate (409 mg, 0.83 mmol, 1.0 eq) in DMF (1 mL) was added slowly, and the reaction solution was stirred at 25°C for 12 hours. The reaction solution was then quenched with saturated ammonium chloride aqueous solution (30 mL) and extracted with ethyl acetate (20 mL * 3). The organic phase was washed with saturated saline (10 mL), dried over anhydrous sodium sulfate, filtered, and concentrated under reduced pressure to obtain a crude product, which was purified by preparative HPLC and preparative SFC to obtain 2-ethylbutyl ((S)-(2R,3S,5R)-3-hydroxyl-5-(5-methyl-2,4-dioxy-3,4-dihydropyrimidin-1(2H)-yl) tetrahydrofuran-2-yl) methoxy)(phenoxy) phosphoryl)-L-alaninate (95.7 mg). LCMS:MS (ESI) m/z = 554.3 [M+H]⁺. ¹H NMR: (400 MHz, DMSO-*d₆*) *δ* 11.31 (s, 1H), 7.49 (t, *J* = 2.0 Hz, 1H), 7.41 - 7.31 (m, 2H), 7.30-7.17 (m, 3H), 6.18 (t,*J* = 8.0 Hz, 1H), 6.10-5.97 (M, 1H), 5.39 (d, *J* = 4.0 Hz, 1H), 4.30 - 4.14 (m, 2H), 4.13 - 4.03 (m, 1H), 4.00 - 3.77 (m, 4H), 2.14 - 1.98 (m, 2H), 1.78-1.70 (M, 3H), 1.44 (m, 1H), 1.34 - 1.18 (m, 7H), 0.82 (t, *J* = 8.0 Hz, 6H).

2-ethylbutyl ((S)-(2R,3S,5R)-2-(hydroxymethyl)-5-(5-methyl-2,4-dioxy-3,4-dihydropyrimidin-1(2H)-yl) tetrahydrofuran-3-yl)oxy)(phenoxy) phosphoryl)-L-alaninate (57.0 mg). LCMS: MS (ESI) m/z = 554.3 [M+H]⁺. ¹H NMR: (400 MHz, DMSO-*d₆*) *δ* 11.32 (s, 1H), 7.71 (dd, *J* = 5.2, 1.2 Hz, 1H), 7.38 (t, *J* = 8.0 Hz, 2H), 7.25 - 7.08 (m, 3H), 6.22 - 6.04 (m, 2H), 5.21 (t, *J* = 5.2 Hz, 1H), 4.98 (t, *J* = 6.0 Hz, 1H), 4.09 - 3.79 (m, 4H), 3.66 - 3.54 (m, 2H), 2.36 - 2.20 (m, 2H), 1.77 (d, *J=* 1.2 Hz, 3H), 1.46 (m, 1H), 1.36 - 1.19 (m, 7H), 0.89 - 0.77 (m, 6H).

The results of the structural assay of various compounds are shown in Table 11.

**Table 11 Results of structural assay of derivatives**

| Derivative Structure | Ms (M+H⁺) | Derivative Structure | Ms (M+H⁺) | Derivative Structure | Ms (M+H⁺) |
|---|---|---|---|---|---|
| T25 | 455.1 | T38 | 582.1 | U14 | 457.0 |
| T26 | 540.2 | T39 | 519.9 | U15 | 471.1 |
| T27 | 467.0 | T40 | 581.1 | U16 | 407.0 |
| T28 | 469.0 | T41 | 455.0 | U17 | 484.1 |
| T29 | 469.0 | T42 | 455.0 | U18 | 669.2 |
| T30 | 431.2 | T43 | 467.2 | U19 | 719.1 |
| T31 | 431.2 | T44 | 431.1 | U20 | 881.2 |
| T32 | 431.2 | T45 | 482.0 | U21 | 556.2 |
| T33 | 479.1 | T46 | 691.2 | | |
| T34 | 480.1 | T47 | 619.2 | | |
| T35 | 518.5 | T48 | 554.1 | | |
| T36 | 482.0 | T49 | 554.0 | | |
| T37 | 405.1 | T50 | 479.1 | | |

## Claims

1. Use of a thymidine derivative in preparation of a drug for preventing or treating a disease or disorder associated with administration of a chemotherapeutical drug in a subject, wherein at least one hydroxyl hydrogen of a deoxyribose in said thymidine derivative is substituted.

2. The use according to claim 1, wherein said thymidine derivative comprises a structure of Formula (I), or a pharmaceutically acceptable salt, solvent, hydrate, prodrug form and stereoisomer thereof: 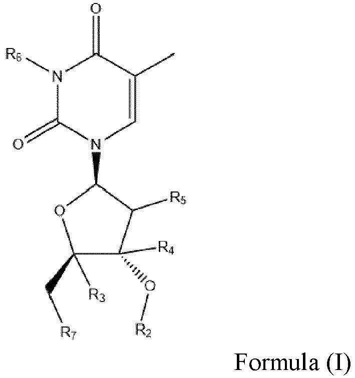 wherein R₂, R₃, R₄, R₅, R₆ and R₇ are not simultaneously hydrogen.

3. The use according to claim 2, wherein R₇ is

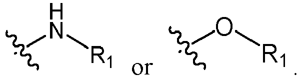

4. The use according to any one of claims 2-3, wherein R₆ is hydrogen.

5. The use according to any one of claims 2-3, wherein R₆ is 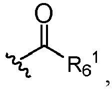 wherein said R₆¹ is C₁-C₆ alkyl.

6. The use according to any one of claims 2-5, wherein any one of R₁, R₂ and R₃ is independently 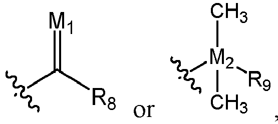 wherein M₁ is oxygen or sulfur, and R₈ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; M₂ is silicon or carbon, and R₉ comprises a group selected from the group consisting of hydrogen, substituted or unsubstituted hydroxyl, substituted or unsubstituted sulfhydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl,
R₉ is selected from the group consisting of C₁-C₅ alkyl, C₁-C₅ cycloalkyl, phenyl or benzyl.

7. The use according to any one of claims 2-6, wherein R₁, R₂ or R₃ is each independently selected from the group consisting of -(C=O)-R₈¹, -(C=O)-OR₈², -(C=O)-NR₈³R₈⁴, -(C=S)-R₈⁵, -(C=S)-OR₈⁶, and -(C=S)-NR₈⁷R₈⁸, wherein any one of R₈¹, R₈², R₈³, R₈⁴, R₈⁵, R₈⁶, R₈⁷ and R₈⁸ is independently selected from the group consisting of hydrogen, substituted or unsubstituted alkyl, substituted or unsubstituted alkynyl, substituted or unsubstituted cycloalkyl, substituted or unsubstituted heterocycloalkyl, substituted or unsubstituted aryl and substituted or unsubstituted heteroaryl.

8. The use according to claim 7, wherein any one of R₈¹, R₈² , R₈³, R₈⁴, R₈⁵ , R₈⁶, R₈⁷ and R₈⁸ is independently selected from the group consisting of hydrogen, C₁-C₅ alkyl, C₁-C₅ cycloalkyl, C₁-C₅ heterocycloalkyl, phenyl, and benzyl.

9. The use according to any one of claims 7-8, wherein R₈¹ is selected from the group consisting of methyl, ethyl, propyl, butyl, cyclopentyl, cyclopropyl, and benzyl.

10. The use according to any one of claims 7-9, wherein R₈² is selected from the group consisting of cyclopropyl, propyl, butyl, pentyl, phenyl, and benzyl.

11. The use according to any one of claims 7-10, wherein R₈³ is hydrogen.

12. The use according to any one of claims 7-11, wherein R₈⁴ is selected from the group consisting of cyclopropyl, cyclopentyl, and phenyl.

13. The use according to any one of claims 6-12, wherein R₉ is butyl.

14. The use according to any one of claims 2-13, wherein R₃ is hydrogen.

15. The use according to any one of claims 2-14, wherein R₄ is hydrogen.

16. The use according to any one of claims 2-15, wherein R₅ is hydrogen.

17. The use according to any one of claims 2-16, wherein R₁ and/or R₂ is 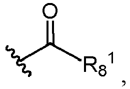 wherein R₈¹ comprises one or more groups selected from the group consisting of C₁-C₆ alkyl, C₁-C₆ silyl, C₁-C₆ alkoxy, C₁-C₆ alkyl nitro, C₃-C₇ cycloalkyl, C₃-C₇ cycloalkylnitro, C₄-C₇ aryl, C₄-C₇ alkoxy aryl and/or C₄-C₇ heteroaryl.

18. The use according to any one of claims 1-17, wherein said thymidine derivative is one or more selected from the group consisting of

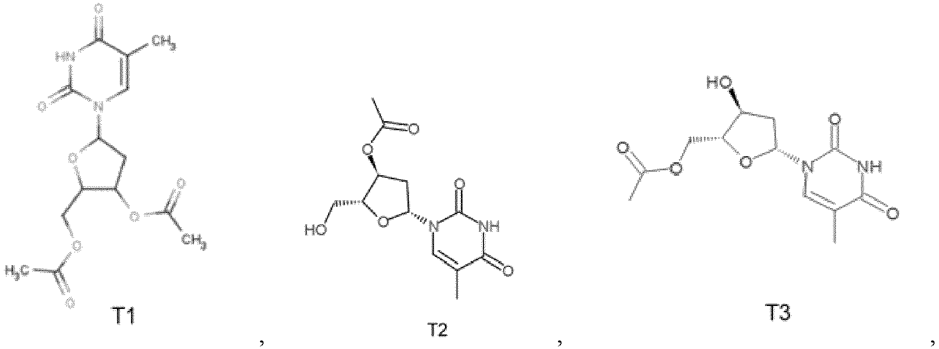

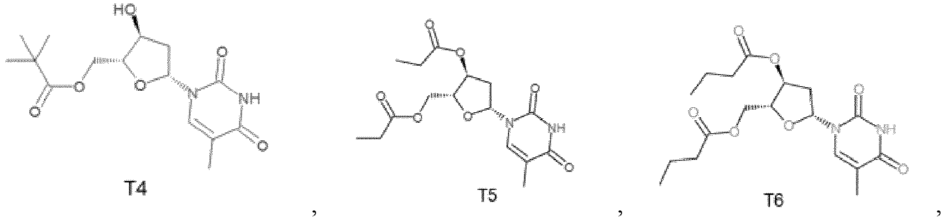

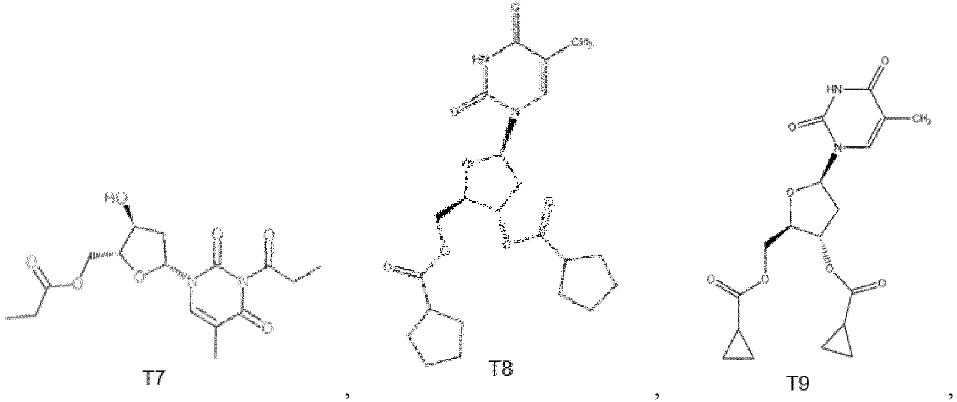

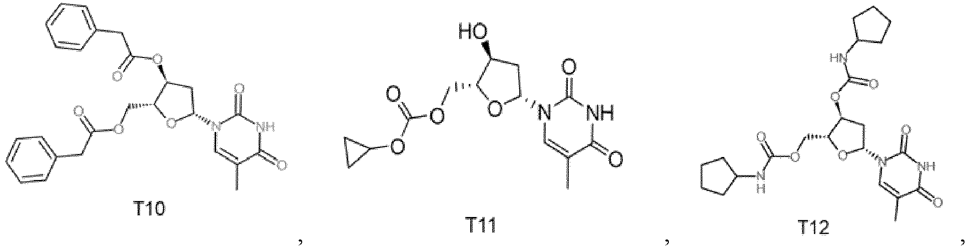

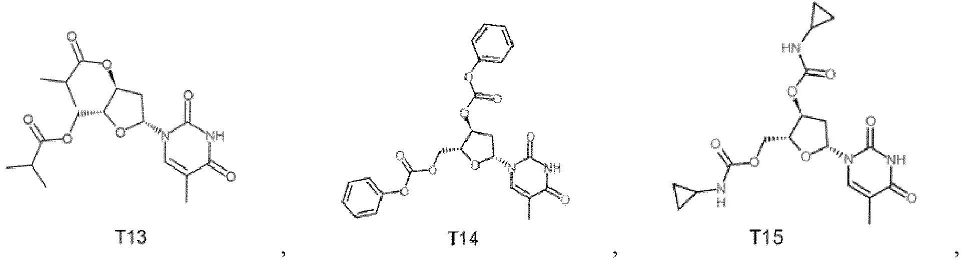

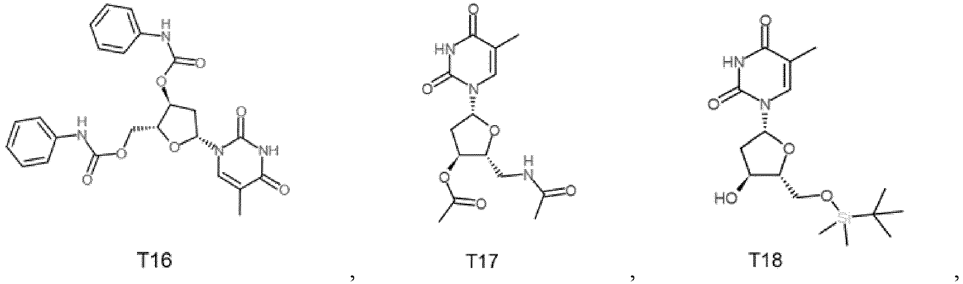

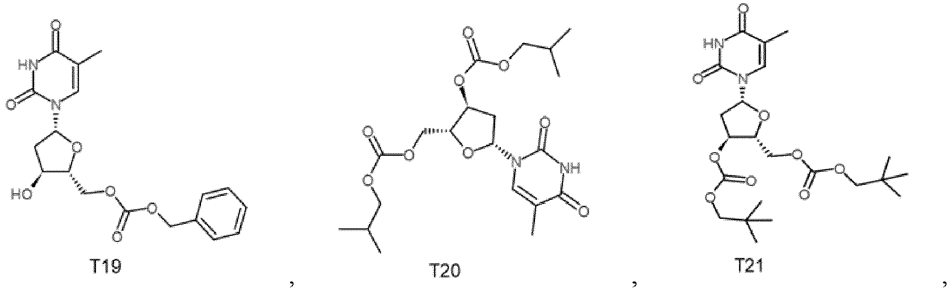

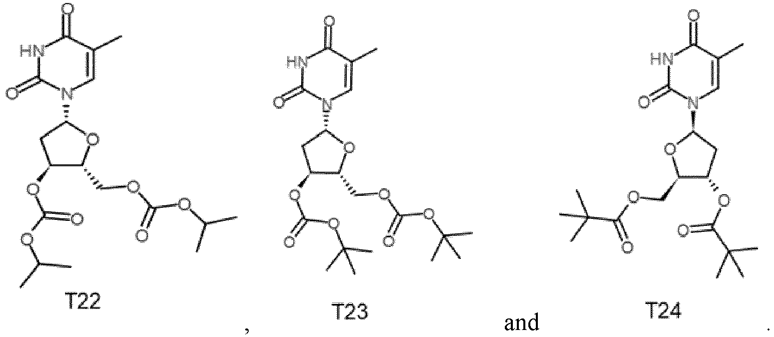

19. The use according to any one of claims 1-18, wherein said thymidine derivative comprises a structure of Formula (II): 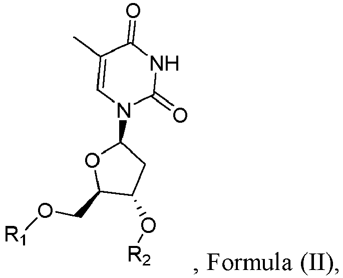 wherein at least one of R₁ and R₂ comprises a non-steroidal anti-inflammatory drug (NSAID) moiety.

20. The use according to claim 19, wherein said NSAID moiety comprises salicylic acid or a derivative thereof, aryl acetic acid or a derivative thereof, heteroaryl acetic acid or a derivative thereof, indoleacetic acid or a derivative thereof, indene acetic acid or a derivative thereof, anthranilic acid or a derivative thereof and/or enolic acid or a derivative thereof.

21. The use according to any one of claims 19-20, wherein R₁ or R₂ is hydrogen.

22. The use according to any one of claims 19-21, wherein R₁ is any one group selected from the group consisting of: 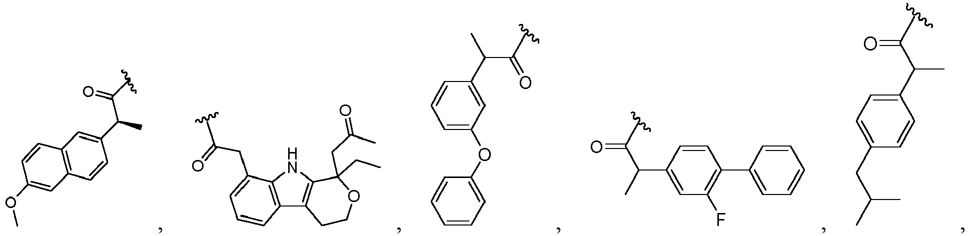 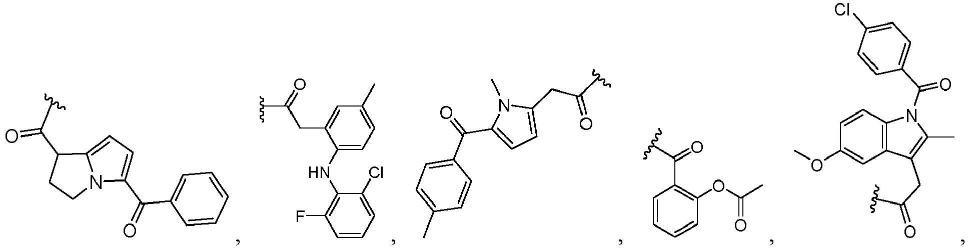 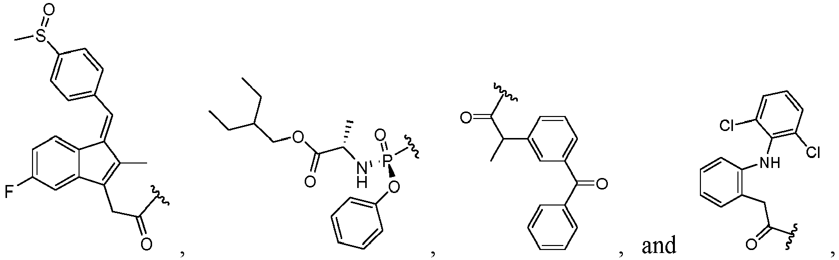 and R₂ is hydrogen.

23. The use according to any one of claims 19-22, wherein R₂ is any one group selected from the group consisting of: 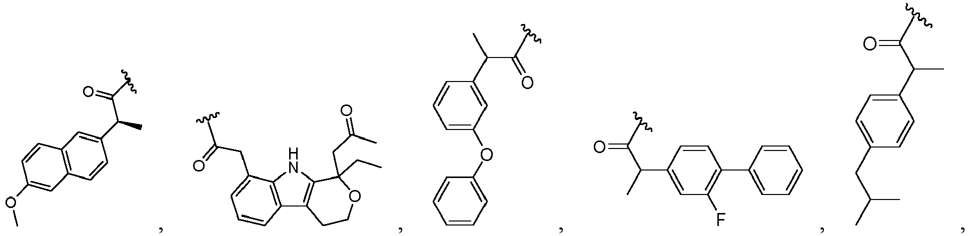 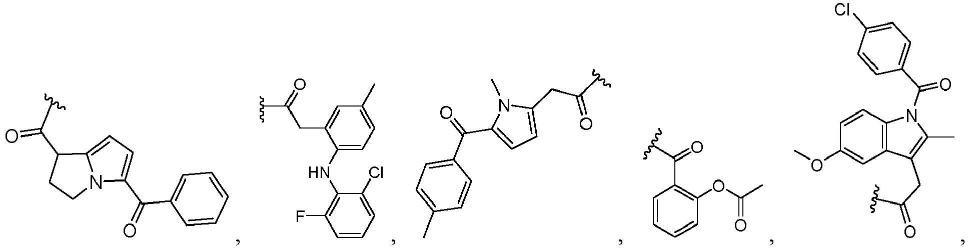 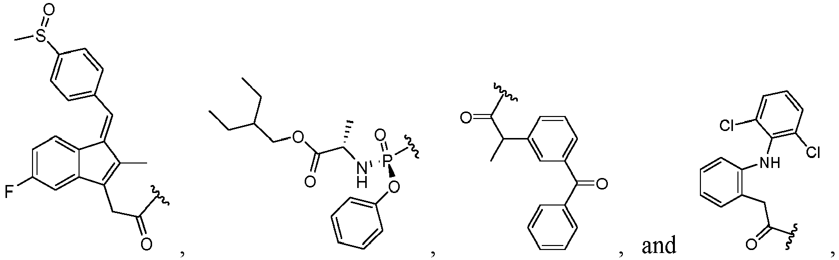 and R₁ is hydrogen.

24. The use according to any one of claims 19-23, wherein R₁ and R₂ are each independently any one group selected from the group consisting of

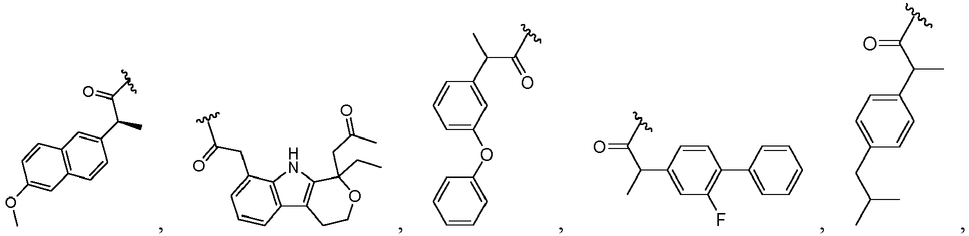

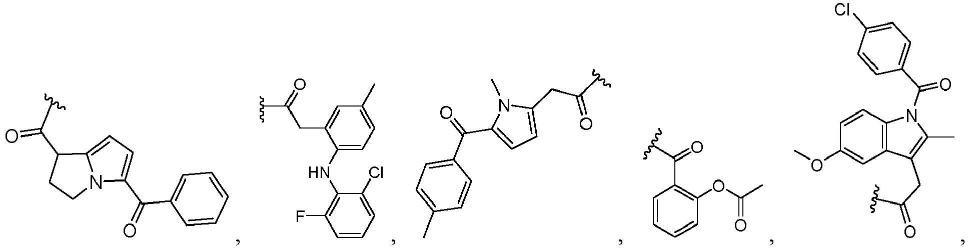

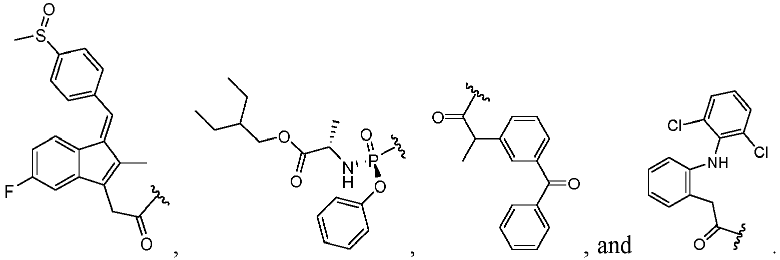

25. The use according to any one of claims 1-24, wherein said thymidine derivative is one or more selected from the group consisting of:

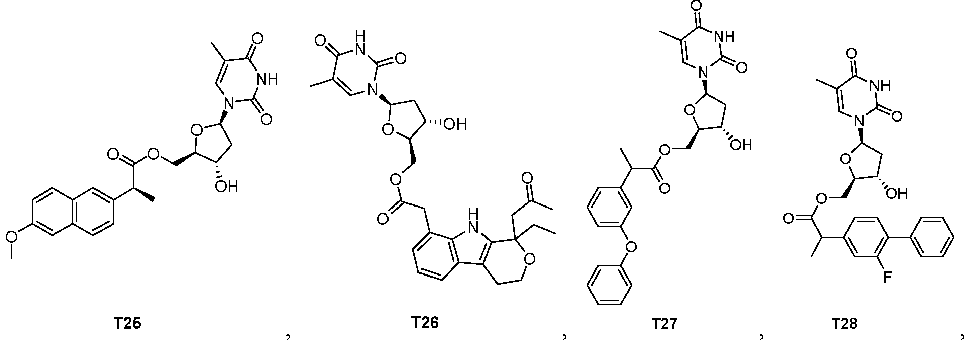

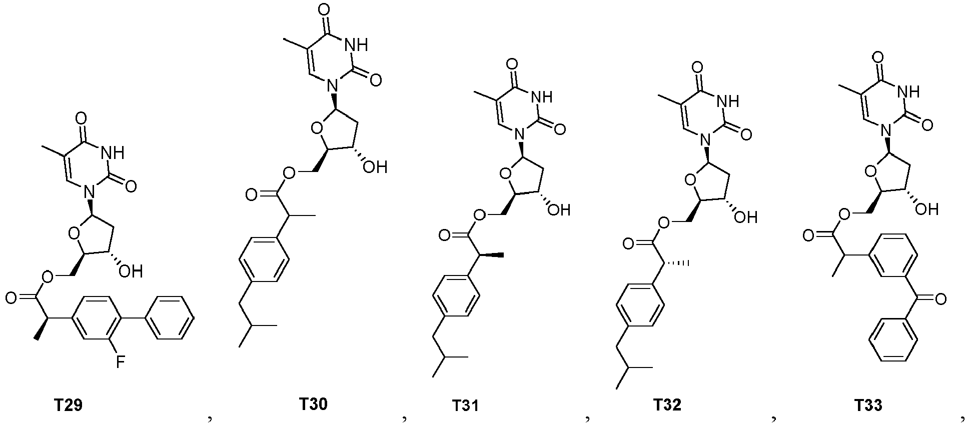

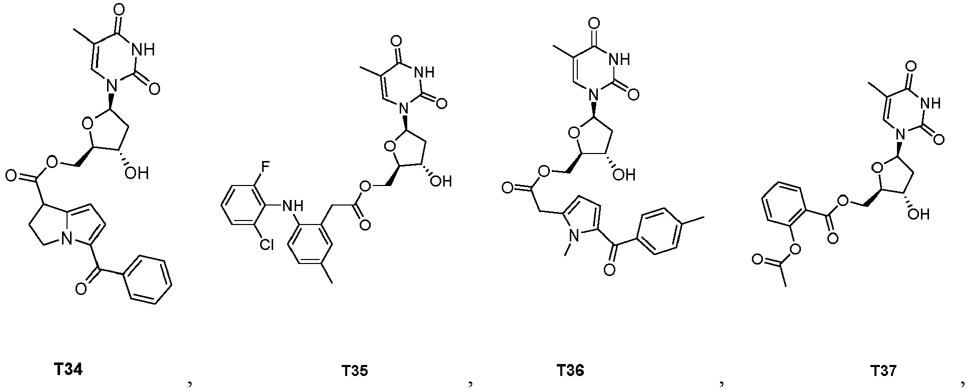

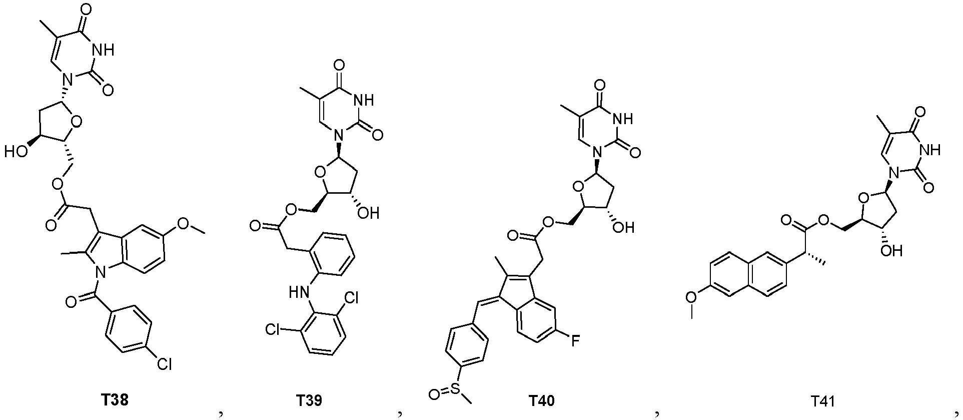

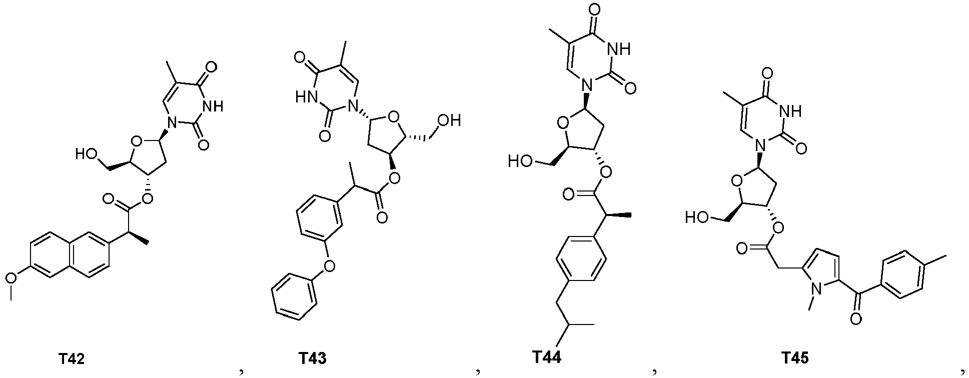

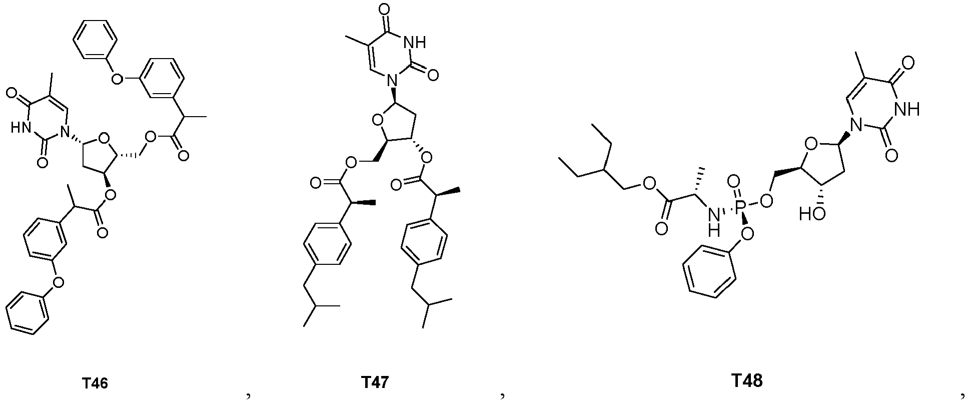

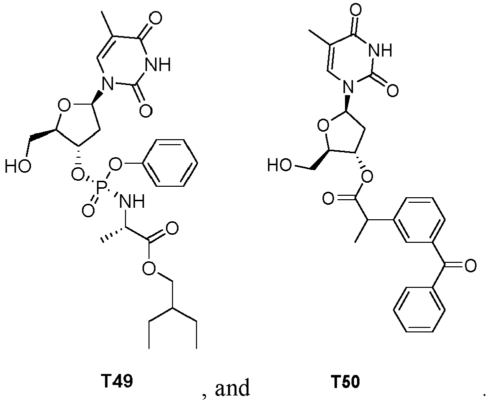

26. The use according to any one of claims 1-25, wherein said chemotherapeutical drug is used for treating cancers.

27. The use according to any one of claims 1-26, wherein said chemotherapeutical drug comprises a pyrimidine nucleoside analog or a prodrug thereof.

28. The use according to any one of claims 1-27, wherein said chemotherapeutical drug comprises one or more selected from the group consisting of capecitabine, cytarabine, docetaxel, adriamycin, fluorouracil (5-FU), floxuridine, tegafur, idarubicin, paclitaxel, epirubicin, acelarin (NUC-1031), doxorubicin, folinic acid, cis-platinum, cyclophosphamide, vincristine, and 5-FU pro-drugs.

29. The use according to claim 28, wherein said 5-FU pro-drugs comprise tegafur, 5'-deoxyfloxuridine, floxuridine, 2'-deoxyfloxuridine, a pro-drug derivative of floxuridine or a pro-drug derivative of 2'-deoxyfloxuridine, trifluoro-methyl-2'-deoxyuridine, 6-azauridine, and/or 3-deazauridine.

30. The use according to any one of claims 1-29, wherein said disease or disorder is caused by administration of the chemotherapeutical drug.

31. The use according to any one of claims 1-30, wherein said disease or disorder associated with administration of the chemotherapeutical drug comprises skin tissue disease or disorder associated with administration of the chemotherapeutical drug, hemopoietic tissue disease or disorder associated with administration of the chemotherapeutical drug, limb disease or disorder associated with administration of the chemotherapeutical drug, cardiac disease or disorder associated with administration of the chemotherapeutical drug, nervous system disease or disorder associated with administration of the chemotherapeutical drug, facial feature disease or disorder associated with administration of the chemotherapeutical drug and/or gastrointestinal disease or disorder associated with administration of the chemotherapeutical drug.

32. The use according to claim 31, wherein said skin tissue disease or disorder associated with administration of the chemotherapeutical drug comprises epithelial tissue disease or disorder associated with administration of the chemotherapeutical drug in skin, limbs, facial features and/or gastrointestinal tract.

33. The use according to claim 32, wherein said epithelial tissue disease or disorder associated with administration of the chemotherapeutical drug in the skin, limbs, facial features and/or gastrointestinal tract comprises disease or disorder associated with endothelial cell lesion, and/or disease or disorder associated with epithelial cell lesion, and wherein said endothelial cell lesions and/or epithelial cell lesions are associated with administration of the chemotherapeutic drug.

34. The use according to claim 33, wherein said epithelial cell comprises skin epithelial cell, oral epithelial cell, nasal epithelial cell, gastric epithelial cell and/or intestinal epithelial cell.

35. The use according to any one of claims 1-34, wherein said disease or disorder associated with administration of the chemotherapeutical drug comprises hand-foot syndrome associated with administration of the chemotherapeutical drug and/or diarrhea associated with administration of the chemotherapeutical drug.

36. The use according to any one of claims 1-35, wherein the severity of said disease or disorder associated with administration of the chemotherapeutical drug is grade 1 or above, grade 2 or above, garde 3 or above, grade 4 or above, and/or grade 5 in accordance with NCI-CTCAE V5.0.

37. The use according to any one of claims 1-36, wherein said drug is prepared to be applicable for topical or oral administration.

38. The use according to any one of claims 1-37, wherein the concentration of said thymidine derivative in said drug is about 0.0001% (w/w) to about 50% (w/w).

39. The use according to any one of claims 1-38, wherein the administration concentration of said thymidine derivative is about 0.5 µM to about 1000 µM.

40. The use according to any one of claims 1-38, wherein the administration dose of said thymidine derivative is about 15 mpk to about 300 mpk.

41. The use according to any one of claims 1-40, wherein said drug further comprises one or more active ingredients.

42. The use according to any one of claims 1-41, wherein said drug does not substantially affect the therapeutical effect of said chemotherapeutical drug.

43. The use according to any one of claims 1-42, wherein said subject comprises a cancer patient.

44. The use according to any one of claims 1-43, wherein said subject has been, is being and/or will be administered with the chemotherapeutical drug.

45. The use according to any one of claims 1-44, wherein said subject has or is susceptible to the disease or disorder associated with the administration of chemotherapeutical drug.

46. The use according to any one of claims 1-45, wherein the severity of said disease or disorder increases after administration of said chemotherapeutical drug.

47. The use according to any one of claims 1-46, wherein before administration of said chemotherapeutical drug, said subject did not have said disease or disorder.

48. A pharmaceutical combination or a kit, comprising: 1) said chemotherapeutical drug of any one of claims 1-47; and 2) said thymidine derivative of any one of claims 1-47.

49. The pharmaceutical combination or the kit according to claim 48, wherein said chemotherapeutical drug and said thymidine derivative are not mixed with each other.

50. The pharmaceutical combination or the kit according to any one of claims 48-49, wherein said chemotherapeutical drug and said thymidine derivative is each independently present in separate containers.

51. The pharmaceutical combination or the kit according to any one of claims 48-50, wherein said thymidine derivative in 2) can prevent or treat a disease or disorder associated with administration of said chemotherapeutical drug in 1).

52. The pharmaceutical combination or the kit according to any one of claims 48-51, wherein said thymidine derivative in 2) does not substantially affect the therapeutical effect of said chemotherapeutical drug in 1).

53. The pharmaceutical combination or the kit according to any one of claims 48-52, wherein said thymidine derivative in 2) is administered before, simultaneously with, or after the administration of said chemotherapeutical drug in 1).

54. Use of the combination of thymidine derivative and uridine derivative in preparation of a drug for preventing or treating a disease or disorder associated with administration of a chemotherapeutical drug in a subject, wherein said thymidine derivative comprises a structure of Formula (I): 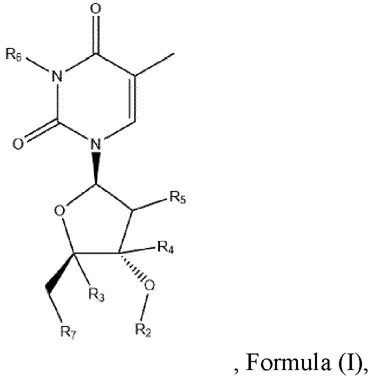 wherein R₂, R₃, R₄, R₅, R₆ and R₇ are not simultaneously hydrogen.

55. The use according to claim 54, wherein R₇ is

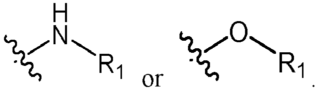

56. The use according to any one of claims 54-55, wherein R₆ is hydrogen.

57. The use according to any one of claims 54-56, wherein R₆ is 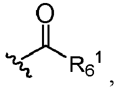 wherein said R₆¹ is C₁-C₆ alkyl.

58. The use according to any one of claims 54-57, wherein any one of R₁, R₂ and R₃ is independently 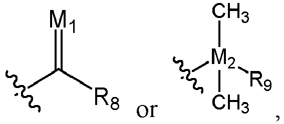 wherein M₁ is oxygen or sulfur, and R₈ comprises one or more groups selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl; M₂ is silicon or carbon, and R₉ is selected from the group consisting of hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted cycloalkyl, substituted or unsubstituted heterocycloalkyl, substituted or unsubstituted aryl, substituted or unsubstituted heteroaryl, and substituted or unsubstituted aralkyl,
R₉ is selected from the group consisting of C₁-C₅ alkyl, C₁-C₅ cycloalkyl, phenyl or benzyl.

59. The use according to any one of claims 54-58, wherein R₁, R₂ or R₃ are each independently selected from the group consisting of -(C=O)-R₈¹, -(C=O)-OR₈², -(C=O)-NR₈³R₈⁴, -(C=S)-R₈⁵ , -(C=S)-OR₈⁶ and -(C=S)-NR₈⁷R₈⁸, wherein any one of R₈¹, R₈² , R₈³, R₈⁴, R₈⁵ , R₈⁶, R₈⁷ and R₈⁸ independently comprises one or more groups selected from the group consisting of hydrogen, substituted or unsubstituted alkyl, substituted or unsubstituted alkynyl, substituted or unsubstituted cycloalkyl, substituted or unsubstituted heterocycloalkyl, substituted or unsubstituted aryl and substituted or unsubstituted heteroaryl.

60. The use according to claim 59, wherein any one of R₈¹, R₈² , R₈³, R₈⁴, R₈⁵ , R₈⁶, R₈⁷ and R₈⁸ is independently selected from the group consisting of hydrogen, C₁-C₅ alkyl, C₁-C₅ cycloalkyl, C₁-C₅ heterocycloalkyl, phenyl, and benzyl.

61. The use according to any one of claims 59-60, wherein R₈¹ comprises one or more groups selected from the group consisting of methyl, ethyl, propyl, butyl, cyclopentyl, cyclopropyl, and benzyl.

62. The use according to any one of claims 59-61, wherein R₈² comprises one or more groups selected from the group consisting of cyclopropyl, propyl, butyl, pentyl, phenyl, and benzyl.

63. The use according to any one of claims 59-62, wherein R₈³ is hydrogen.

64. The use according to any one of claims 59-63, wherein R₈⁴ is selected from the group consisting of cyclopropyl, cyclopentyl, and phenyl.

65. The use according to any one of claims 59-64, wherein R₉ is butyl.

66. The use according to any one of claims 59-65, wherein R₃ is hydrogen.

67. The use according to any one of claims 59-66, wherein R₄ is hydrogen.

68. The use according to any one of claims 59-67, wherein R₅ is hydrogen.

69. The use according to any one of claims 59-68, wherein R₁ and/or R₂ are/is 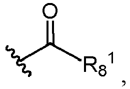 wherein R₈¹ comprises one or more groups selected from the group consisting of C₁-C₆ alkyl, C₁-C₆ silyl, C₁-C₆ alkoxy, C₁-C₆ alkyl nitro, C₃-C₇ cycloalkyl, C₃-C₇ cycloalkyl nitro, C₄-C₇ aryl, C₄-C₇ alkoxy aryl and/or C₄-C₇ heteroaryl.

70. The use according to any one of claims 54-69, wherein said thymidine derivative is one or more selected from the group consisting of

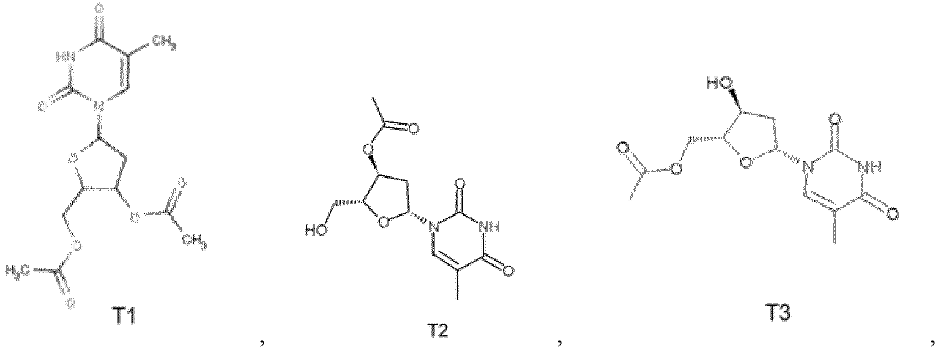

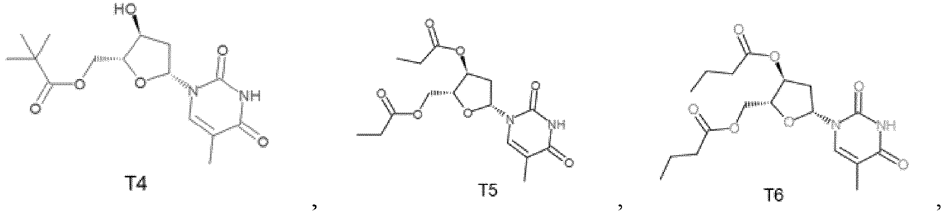

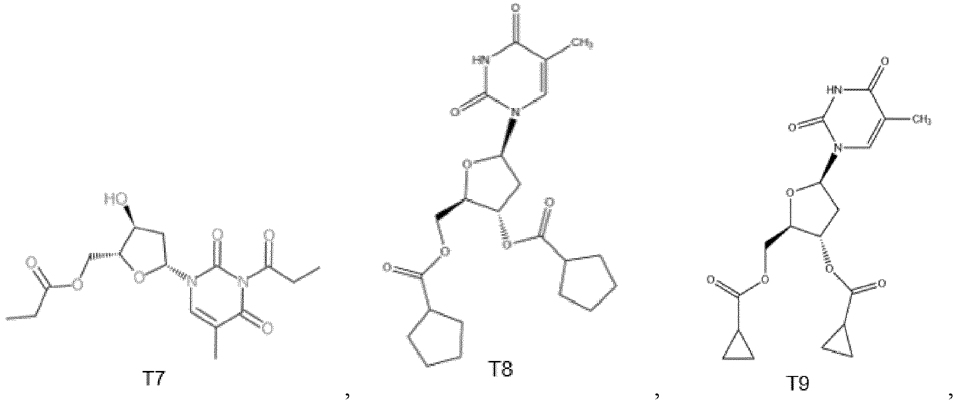

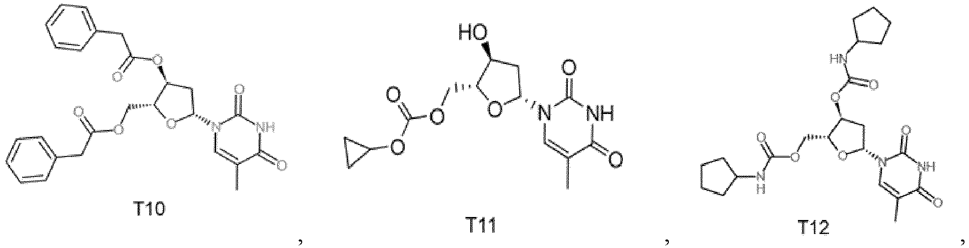

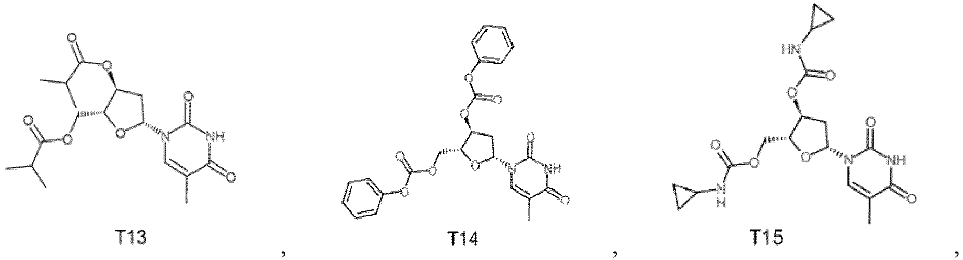

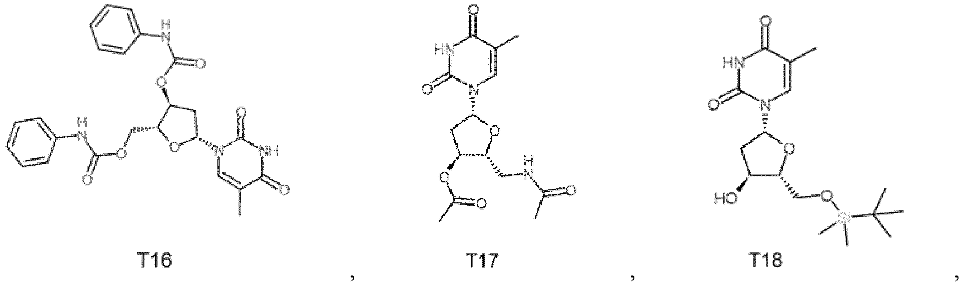

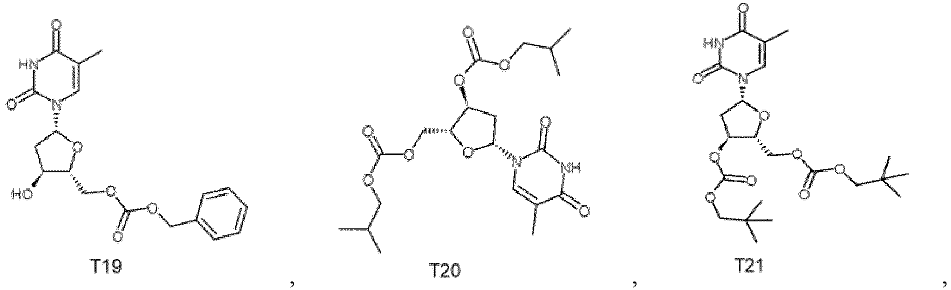

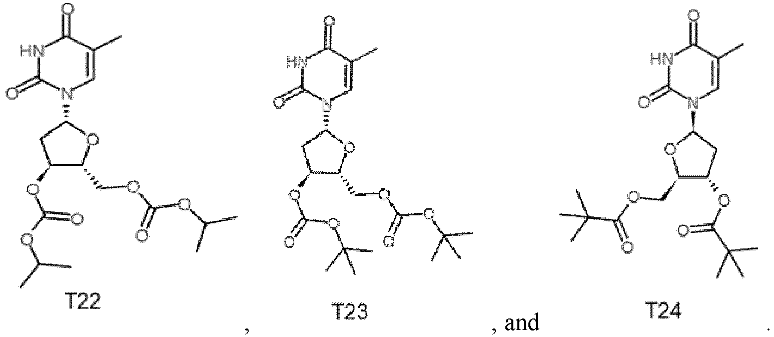

71. The use according to any one of claims 54-63, wherein said thymidine derivative comprises a structure of Formula (II) 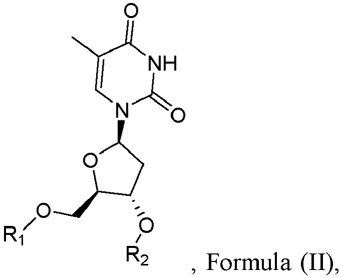 wherein at least one of R₁ and R₂ comprises a non-steroidal anti-inflammatory drug (NSAID) moiety.

72. The use according to claim 71, wherein said NSAID comprises salicylic acid or a derivative thereof, aryl acetic acid or a derivative thereof, heteroaryl acetic acid or a derivative thereof, indoleacetic acid or a derivative thereof, indene acetic acid or a derivative thereof, anthranilic acid or a derivative thereof and/or enolic acid or a derivative thereof.

73. The use according to any one of claims 71-72, wherein R₁ or R₂ is hydrogen.

74. The use according to any one of claims 71-73, wherein R₁ and R₂ are not simultaneously hydrogen.

75. The use according to any one of claims 71-74, wherein R₁ is any one group selected from the group consisting of: 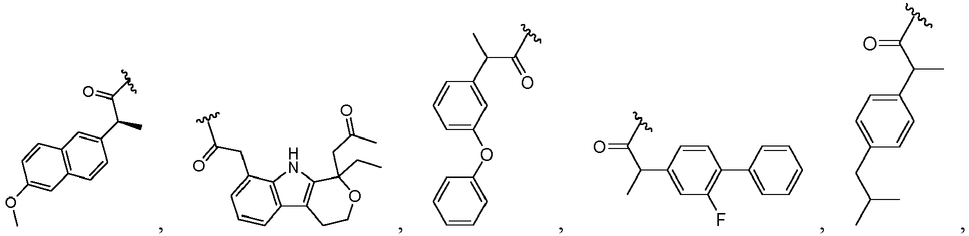 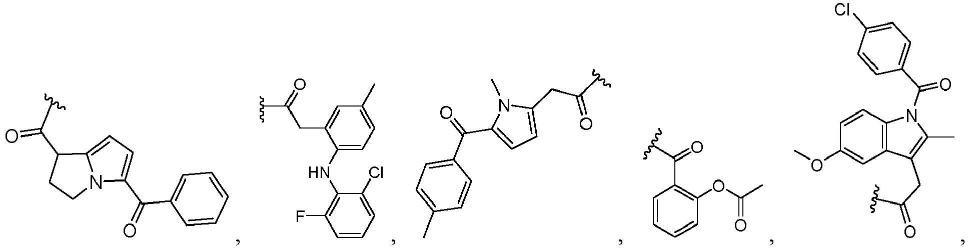 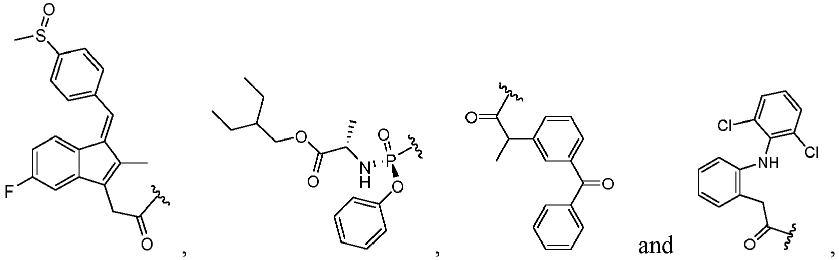 and R₂ is hydrogen.

76. The use according to claims 71-74, wherein R₂ is any one group selected from the group consisting of: 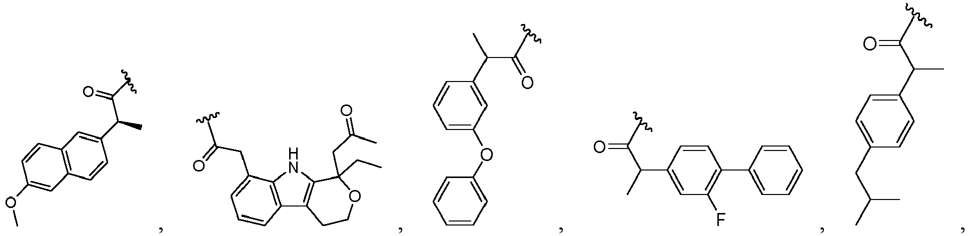 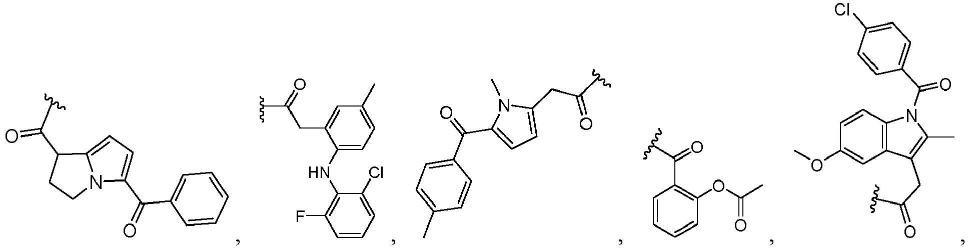 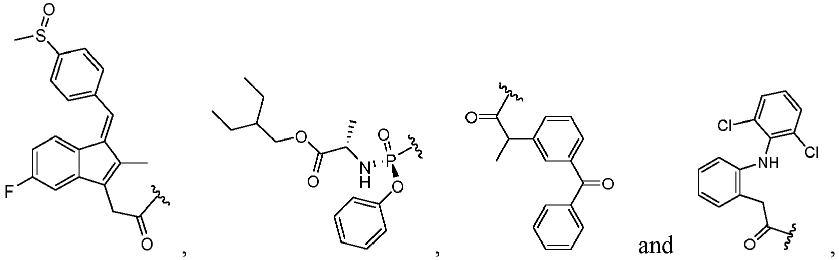 and R₁ is hydrogen.

77. The use according to any one of claims 71-74, wherein R₁ and R₂ are each independently any one group selected from the group consisting of

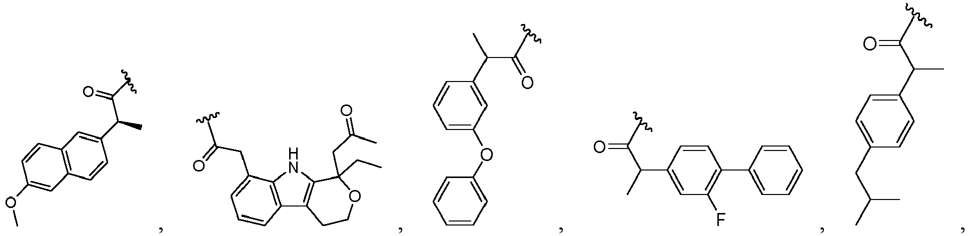

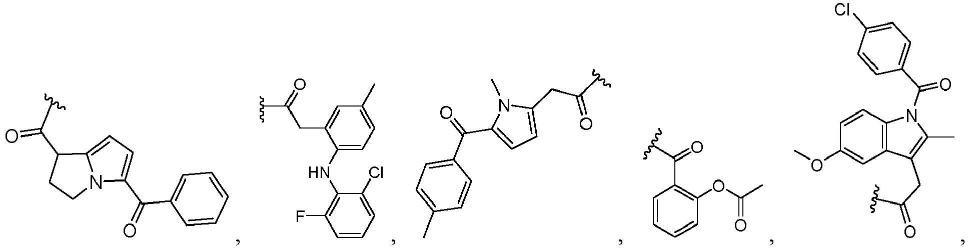

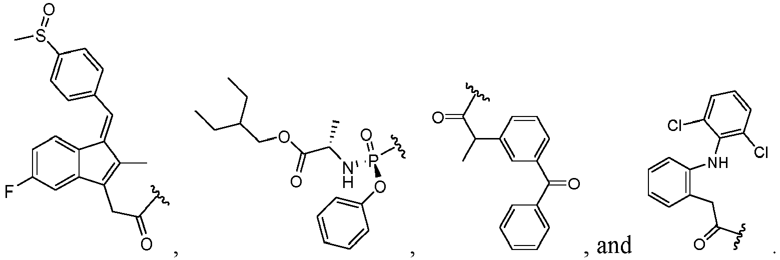

78. The use according to any one of claims 54-77, wherein said thymidine derivative is one or more selected from the group consisting of: 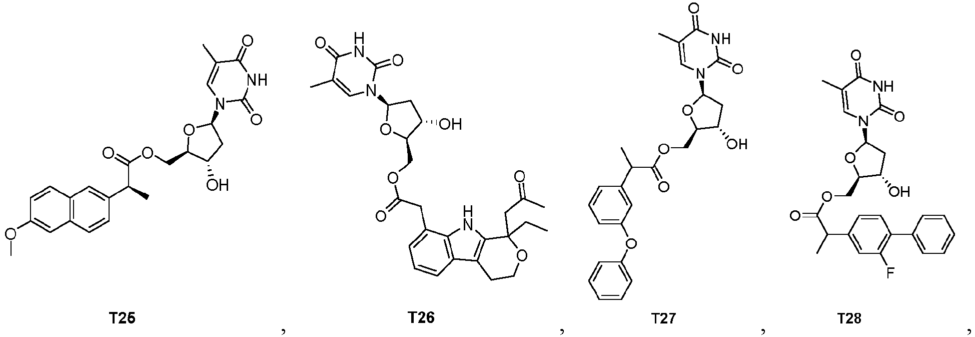 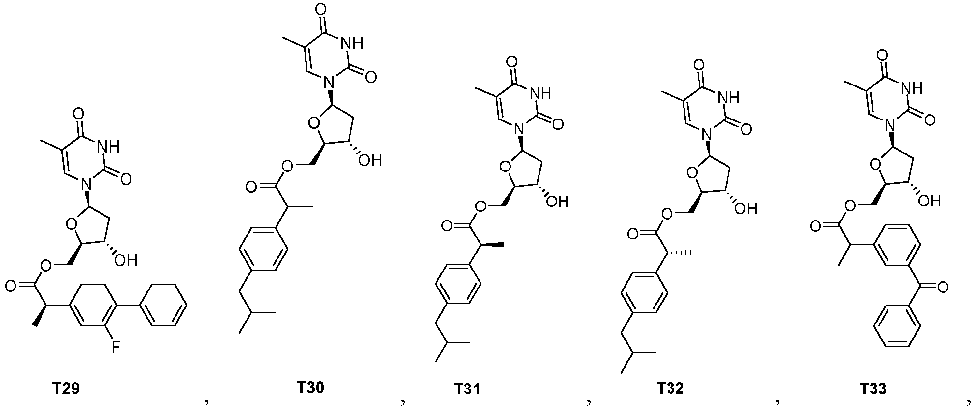 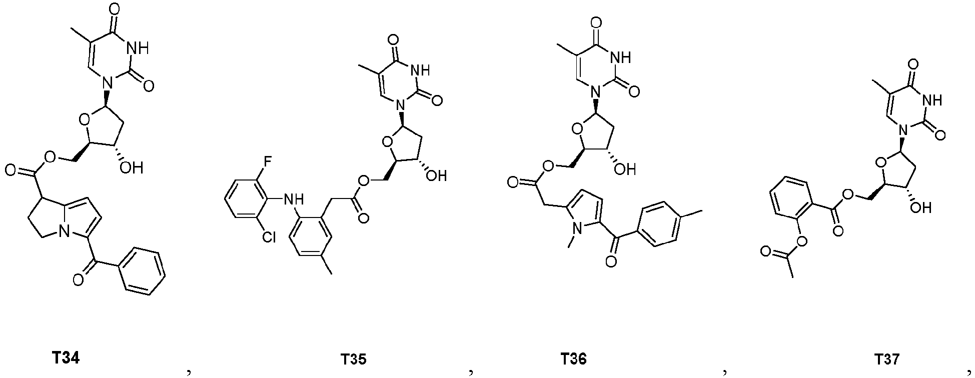 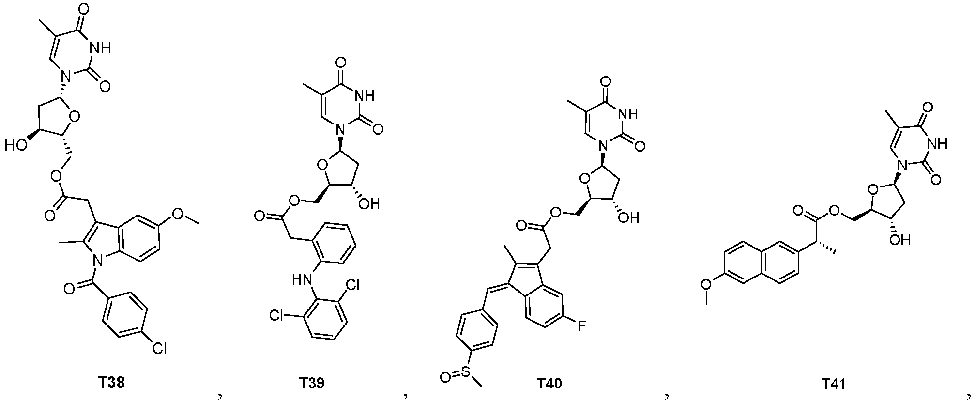 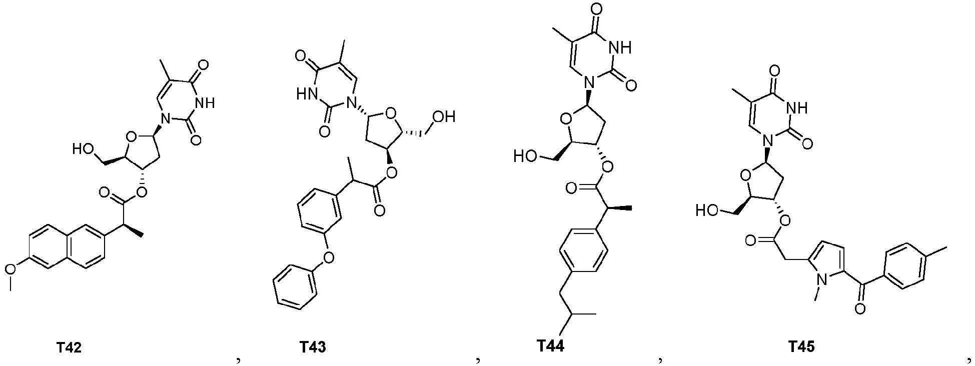 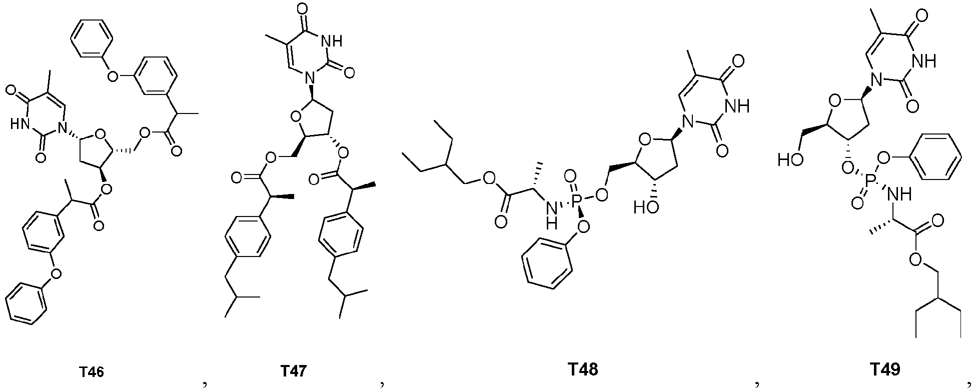 and

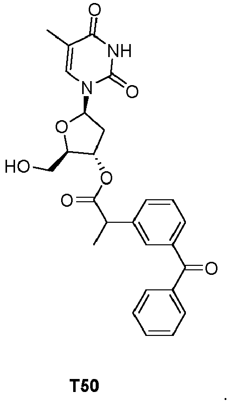

79. The use according to any one of claims 54-78, wherein said uridine derivative comprises a structure of Formula (III): 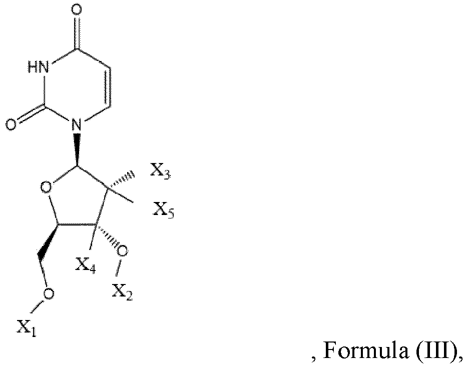 wherein, when X₄ and X₅ are both hydrogens, X₁, X₂ and X₃ are not simultaneously hydrogen or are not simultaneously CH₃CO-.

80. The use according to claim 79, wherein X₁ is hydrogen or 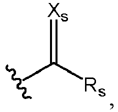 wherein said Xₛ is oxygen or sulfur, Rₛ is selected from hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted C₁-C₅ cycloalkyl, substituted or unsubstituted heterocycloalkyl, substituted or unsubstituted aryl, and substituted or unsubstituted aralkyl.

81. The use according to claim 80, wherein Xₛ is oxygen.

82. The use according to any one of claims 79-81, wherein X₂ is hydrogen or 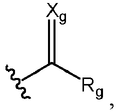 wherein said X_{g} is oxygen or sulfur, R_{g} is selected from hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted C₁-C₅ cycloalkyl, substituted or unsubstituted heterocycloalkyl, substituted or unsubstituted aryl, and substituted or unsubstituted aralkyl.

83. The use according to claim 82, wherein X_{g} is oxygen.

84. The use according to any one of claims 79-83, wherein X₃ is 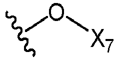 or hydrogen, wherein said X₇ is hydrogen or 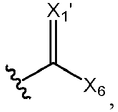 wherein said X₁' is oxygen or sulfur, X₆ is selected from hydrogen, substituted or unsubstituted hydroxyl, substituted or unsubstituted sulfydryl, substituted or unsubstituted amino, substituted or unsubstituted C₁-C₅ alkyl, substituted or unsubstituted C₁-C₅ alkynyl, substituted or unsubstituted C₁-C₅ cycloalkyl, substituted or unsubstituted heterocycloalkyl, substituted or unsubstituted aryl, and substituted or unsubstituted aralkyl.

85. The use according to any one of claims 79-84, wherein X₃ is 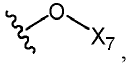 said C₇ is 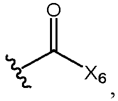 wherein X₆ is selected from hydrogen, C₁-C₆ alkyl, C₁-C₆ alkoxy, C₃-C₁₀ cycloalkyl, C₃-C₁₀ cycloalkoxy, C₄-C₁₀ aralkyl, C₄-C₁₀ aralkoxy or C₄-C₁₀ aryl.

86. The use according to any one of claims 79-85, wherein said X₃ is hydrogen.

87. The use according to any one of claims 79-86, wherein said X₁ is 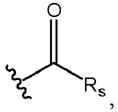 wherein Rₛ is selected from hydrogen, C₁-C₆ alkyl, C₁-C₆ alkoxy, C₃-C₁₀ cycloalkyl, C₃-C₁₀ cycloalkoxy, C₄-C₁₀ aralkyl, C₄-C₁₀ aralkoxy or C₄-C₁₀ aryl.

88. The use according to any one of claims 79-87, wherein said X₂ is 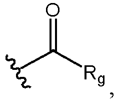 wherein R_{g} is selected from hydrogen, C₁-C₆ alkyl, C₁-C₆ alkoxy, C₃-C₁₀ cycloalkyl, C₃-C₁₀ cycloalkoxy, C₄-C₁₀ aralkyl, C₄-C₁₀ aralkoxy or C₄-C₁₀ aryl.

89. The use according to any one of claims 79-88, wherein X₄ is hydrogen.

90. The use according to any one of claims 79-89, wherein X₅ is hydrogen.

91. The use according to any one of claims 79-90, wherein said uridine derivative is one or more selected from the group consisting of: 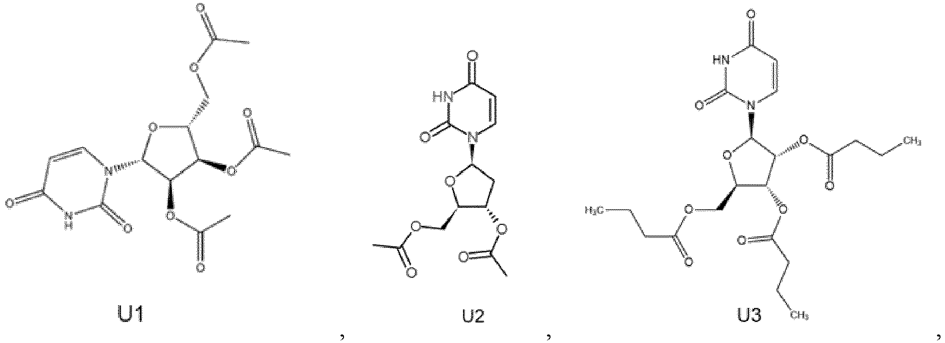 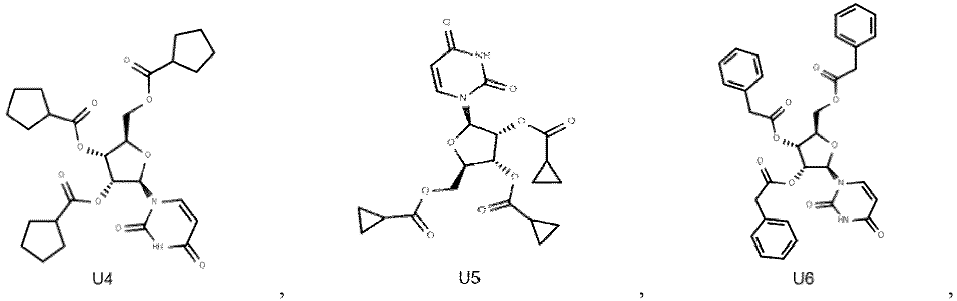 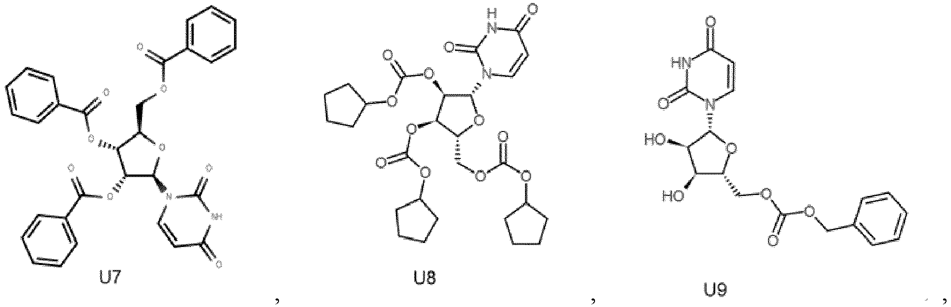 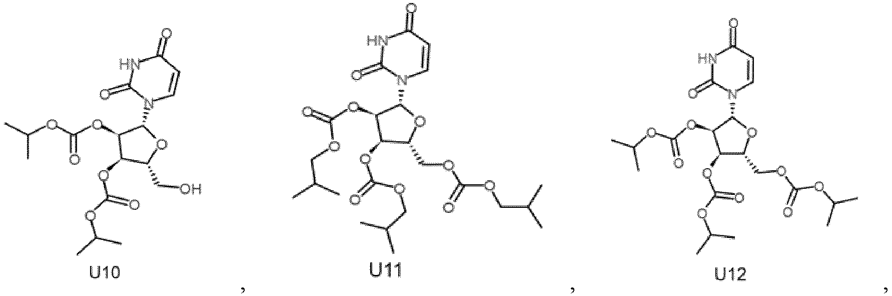 and

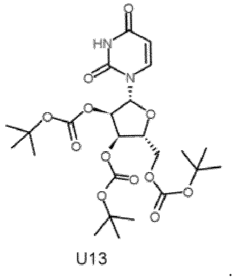

92. The use according to any one of claims 54-91, wherein said uridine derivative comprises a compound of Formula (IV): 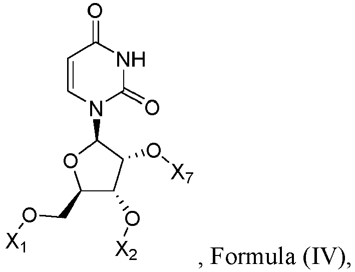 wherein, at least one of said X₁, X₂ and X₇ comprises a non-steroidal anti-inflammatory drug (NSAID) moiety.

93. The use according to claim 92, wherein said NSAID moiety comprises salicylic acid or a derivative thereof, aryl acetic acid or a derivative thereof, heteroaryl acetic acid or a derivative thereof, indole acetic acid or a derivative thereof, indene acetic acid or a derivative thereof, anthranilic acid or a derivative thereof and/or enolic acid or a derivative thereof.

94. The use according to any one of claims 92-93, wherein X₁, X₂ or X₇ is hydrogen.

95. The use according to any one of claims 92-94, wherein X₁, X₂ and X₇ are not simultaneously hydrogen.

96. The use according to any one of claims 92-95, wherein any one of X₁, X₂ and X₇ is independently selected from the group consisting of

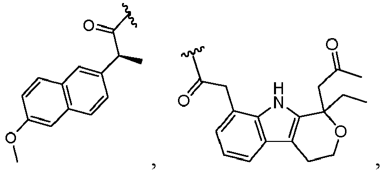

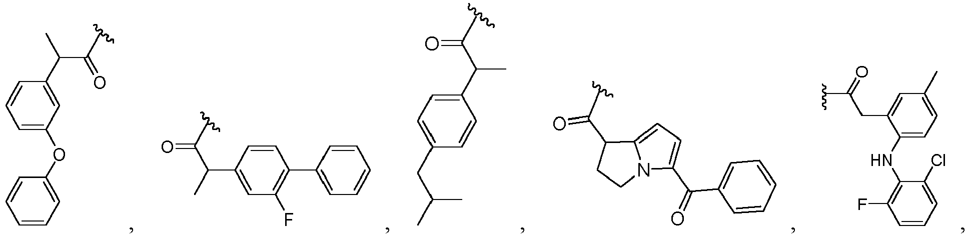

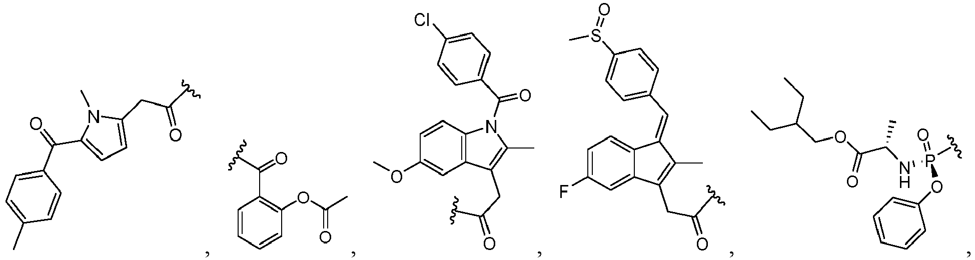

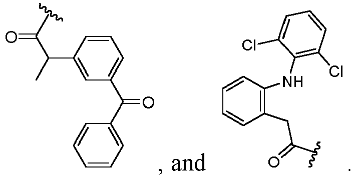

97. The use according to any one of claims 54-96, wherein said uridine derivative is one or more selected from the group consisting of:

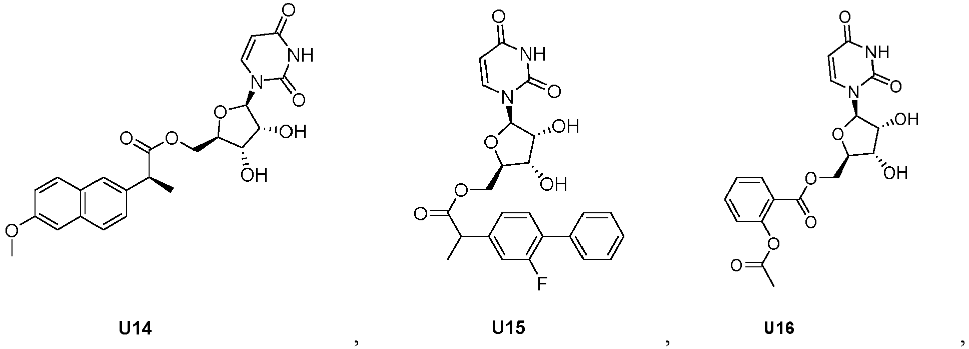

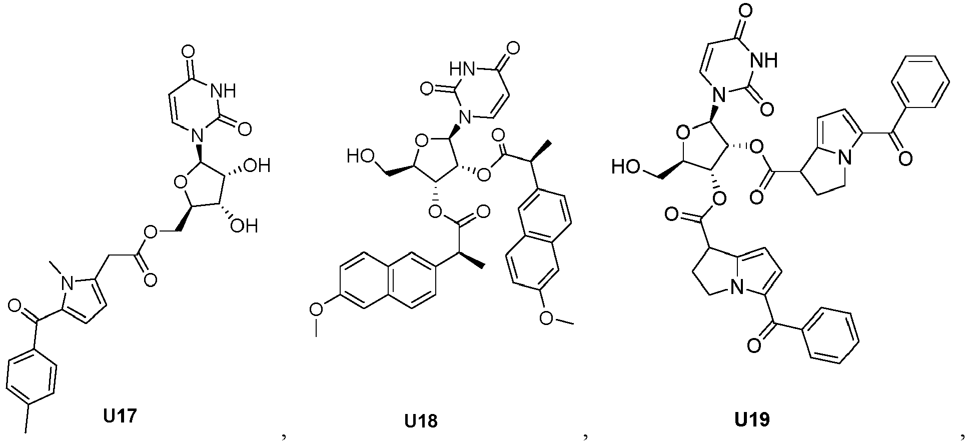

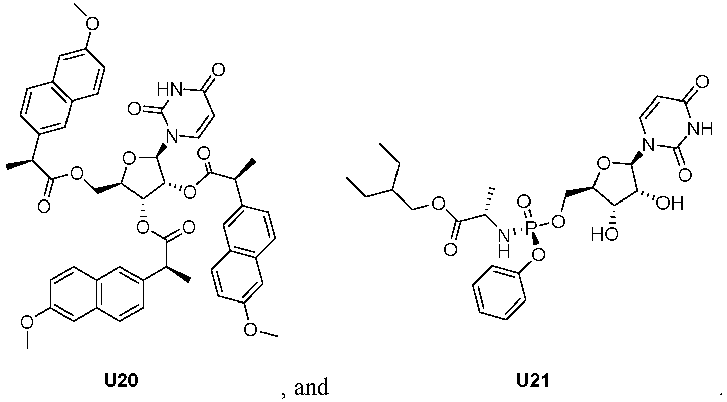

98. The use according to any one of claims 54-97, wherein said chemotherapeutical drug is used for treating cancers.

99. The use according to any one of claims 54-98, wherein said chemotherapeutical drug comprises a pyrimidine nucleoside analog or a prodrug thereof.

100. The use according to any one of claims 54-99, wherein said chemotherapeutical drug comprises one or more selected from the group consisting of capecitabine, cytarabine, docetaxel, adriamycin, fluorouracil (5-FU), floxuridine, tegafur, idarubicin, paclitaxel, epirubicin, acelarin (NUC-1031), doxorubicin, folinic acid, cis-platinum, cyclophosphamide, vincristine, and 5-FU pro-drugs.

101. The use according to claim 100, wherein said 5-FU pro-drugs comprise tegafur, 5'-deoxyfloxuridine, floxuridine, 2'-deoxyfloxuridine, a pro-drug derivative of floxuridine or a pro-drug derivative of 2'-deoxyfloxuridine, trifluoro-methyl-2'-deoxyuridine, 6-azauridine, and 3-deazauridine.

102. The use according to any one of claims 54-101, wherein said disease or disorder is caused by administration of the chemotherapeutical drug.

103. The use according to any one of claims 54-102, wherein said disease or disorder associated with administration of the chemotherapeutical drug comprises skin tissue disease or disorder associated with administration of the chemotherapeutical drug, hemopoietic tissue disease or disorder associated with administration of the chemotherapeutical drug, limb disease or disorder associated with administration of the chemotherapeutical drug, cardiac disease or disorder associated with administration of the chemotherapeutical drug, nervous system disease or disorder associated with administration of the chemotherapeutical drug, facial feature disease or disorder associated with administration of the chemotherapeutical drug and/or gastrointestinal disease or disorder associated with administration of the chemotherapeutical drug.

104. The use according to claims 54-103, wherein said hemopoietic tissue disease or disorder associated with administration of the chemotherapeutical drug comprises marrow disease or disorder associated with administration of the chemotherapeutical drug and blood disease or disorder associated with administration of the chemotherapeutical drug.

105. The use according to claims 54-104, wherein said disease or disorder associated with administration of the chemotherapeutical drug comprises epithelial tissue disease or disorder associated with administration of the chemotherapeutical drug.

106. The use according to claim 105, wherein said epithelial tissue disease or disorder associated with administration of the chemotherapeutical drug comprises disease or disorder associated with endothelial cell lesion, and/or disease or disorder associated with epithelial cell lesion, and wherein said endothelial cell lesion and/or epithelial cell lesion are/is associated with administration of the chemotherapeutic drug.

107. The use according to claim 106, wherein said endothelial cell comprises vascular endothelial cell.

108. The use according to any one of claims 106-107, wherein said epithelial cell comprises skin epithelial cell, oral epithelial cell, nasal epithelial cell, gastric epithelial cell and/or intestinal epithelial cell.

109. The use according to any one of claims 54-108, wherein said disease or disorder associated with administration of the chemotherapeutical drug comprises hand-foot syndrome associated with administration of the chemotherapeutical drug and/or diarrhea associated with administration of the chemotherapeutical drug.

110. The use according to any one of claims 54-109, wherein the severity of said disease or disorder associated with administration of the chemotherapeutical drug is grade 1 or above, grade 2 or above, grade 3 or above, grade 4 or above, and/or grade 5 in accordance with NCI-CTCAE V5.0.

111. The use according to any one of claims 54-110, wherein said drug is prepared to be applicable for topical or oral administration.

112. The use according to any one of claims 54-111, wherein the concentration of said thymidine derivative and uridine derivative in said drug is about 0.0001% (w/w) to about 50% (w/w).

113. The use according to any one of claims 54-112, wherein the administration dose of said thymidine derivative and uridine derivative is about 0.5 µM to about 1000 µM.

114. The use according to any one of claims 54-113, wherein the administration dose of said thymidine derivative and uridine derivative is about 10 mpk to about 1000 mpk.

115. The use according to any one of claims 54-114, wherein said drug further comprises one or more active ingredients.

116. The use according to any one of claims 54-115, wherein said drug does not substantially affect the therapeutical effect of said chemotherapeutical drug.

117. The use according to any one of claims 54-116, wherein said subject comprises a cancer patient.

118. The use according to any one of claims 54-117, wherein said subject has been, is being and/or will be administered with the chemotherapeutical drug.

119. The use according to any one of claims 54-118, wherein said subject has or is susceptible to have said disease or disorder associated with administration of the chemotherapeutical drug.

120. The use according to any one of claims 54-119, wherein the severity of said disease or disorder increases after administration of said chemotherapeutical drug.

121. The use according to any one of claims 54-120, wherein before administration of said chemotherapeutical drug, said subject did not have said disease or disorder.

122. A pharmaceutical combination or a kit, comprising: 1) said chemotherapeutical drug of any one of claims 54-121; and 2) the combination of said thymidine derivative and uridine derivative of any one of claims 54-121.

123. The pharmaceutical combination or the kit according to claim 122, wherein said chemotherapeutical drug and the combination of thymidine derivative and uridine derivative are not mixed with each other.

124. The pharmaceutical combination or the kit according to any one of claims 122-123, wherein said chemotherapeutical drug and the combination of thymidine derivative and uridine derivative are each independently present in separate containers.

125. The pharmaceutical combination or the kit according to any one of claims 122-124, wherein said combination of thymidine derivative and uridine derivative in 2) can prevent or treat a disease or disorder associated with administration of said chemotherapeutical drug in 1).

126. The pharmaceutical combination or the kit according to any one of claims 122-125, wherein said combination of thymidine derivative and uridine derivative in 2) does not substantially affect the therapeutical effect of said chemotherapeutical drug in 1).

127. The pharmaceutical combination or the kit according to any one of claims 122-126, wherein said combination of thymidine derivative and uridine derivative in 2) is administered before, simultaneously with, or after the administration of said chemotherapeutical drug in 1).
